# EUROPÄISCHE PATENTSCHRIFT

(11) **EP 4 146 338 B1**
(45) Veröffentlichungstag und Bekanntmachung des Hinweises auf die Patenterteilung: **24.07.2024**
(21) Anmeldenummer: 20772005.3
(22) Anmeldetag: 07.09.2020
(51) Int. Cl.: A61P 3/00, C07C 67/03, C07C 69/67, C07C 67/08, C07C 69/70, C12P 7/62

(54) **VERFAHREN ZUR HERSTELLUNG VON CARBONSÄUREESTERN VON HYDROXYBUTANOATEN**
PROCESS FOR PREPARING CARBOXYLIC ACID ESTERS OF HYDROXYBUTANOATES
PROCÉDÉ DE PRÉPARATION D'ESTERS D'ACIDE CARBOXYLIQUE D'HYDROXYBUTANOATES

(30) Priorität: 13.07.2020 WO PCT/EP2020/069707
(43) Veröffentlichungstag der Anmeldung: 15.03.2023
(73) Patentinhaber: KetoLipix Therapeutics GmbH, 20459 Hamburg (DE)
(72) Erfinder: LOCHMANN, Dirk, 58453 Witten (DE); REYER, Sebastian, 58453 Witten (DE); STEHR, Michael, 58453 Witten (DE)
(74) Vertreter: Von Rohr Patentanwälte Partnerschaft mbB
(86) Internationale Anmeldenummer: PCT/EP2020/074890
(87) Internationale Veröffentlichungsnummer: WO 2022/012765

(56) Entgegenhaltungen:
- EP-A1- 1 746 110
- WO-A1-2016/012657
- WO-A1-2017/213999
- WO-A1-2019/147503
- WO-A1-2020/147981
- WO-A1-2020/249197
- WO-A1-2020/249198
- JP-A- 2016 193 849
- JP-A- 2018 154 559
- US-A1- 2019 177 673
- US-A1- 2019 211 144
- FUJIWARA TOSHIO ET AL: "Application of a novel chromophoric reagent, 2,2'-binaphthyl-3,3'-dicarbonyl cyanide, to the absolute configuration determination of chiral secondary alcohols", TETRAHEDRON LETTERS, vol. 61, no. 24, 1 June 2020 (2020-06-01), Amsterdam , NL, pages 151984, XP055807867, ISSN: 0040-4039, DOI: 10.1016/j.tetlet.2020.151984
- RAGHAVAN SADAGOPAN ET AL: "Synthesis of an advanced intermediate enroute to thiomarinol antibiotics", TETRAHEDRON, vol. 73, no. 19, 2017 - 2017, pages 2814 - 2823, XP029975323, ISSN: 0040-4020, DOI: 10.1016/J.TET.2017.03.089
- MORLEY K L ET AL: "Parallel synthesis of an ester library for substrate mapping of esterases and lipases", TETRAHEDRON ASYMMETRY, PERGAMON PRESS LTD, OXFORD, GB, vol. 15, no. 18, 20 September 2004 (2004-09-20), pages 3005 - 3009, XP027414599, ISSN: 0957-4166, [retrieved on 20040922]
- RU RU H ET AL: "-HYDROXYTETRAPHENYLCYCLOPENTADIENYL(TETRAPHENYL-2,4-CYCLOPENTADIEN-1-ONE) 1-Hydroxytetraphenylcyclopentadienyl- (tetraphenyl-2,4-cyclopentadien-1-one)- -hydrotetracarbonyldiruthenium(II)", 1 January 2009 (2009-01-01), pages 1 - 7, XP055807919, Retrieved from the Internet <URL:https://onlinelibrary.wiley.com/doi/pdfdirect/10.1002/047084289X.rn01063> [retrieved on 20210526]
- HÄCKER CHRISTINE ET AL: "General Stereodivergent Enantioselective Total Synthetic Approach toward Macrosphelides A-G and M", J. ORG. CHEM., vol. 80, no. 16, 21 August 2015 (2015-08-21), pages 8055 - 8064, XP055807921, ISSN: 0022-3263, Retrieved from the Internet <URL:https://pubs.acs.org/doi/pdf/10.1021/acs.joc.5b01171> DOI: 10.1021/acs.joc.5b01171
- BEAT?M. BACHMANN ET AL: "Synthesis and Structure of Linear and Cyclic Oligomers of 3-Hydroxybutanoic Acid with Specific Sequences of (R)- and (S)-Configurations", HELVETICA CHIMICA ACTA, vol. 81, no. 12, 16 December 1998 (1998-12-16), pages 2430 - 2461, XP055213551, ISSN: 0018-019X, DOI: 10.1002/(SICI)1522-2675(19981216)81:12<2430::AID-HLCA2430>3.0.CO;2-W
- LI JUN ET AL: "Conformational Analysis of Oligomers of (R)-3-Hydroxybutanoic Acid in Solutions by HNMR Spectroscopy", BULLETIN OF THE CHEMICAL SOCIETY OF JAPAN,, vol. 71, no. 7, 1 January 1998 (1998-01-01), pages 1683 - 1689, XP008177515, ISSN: 0009-2673, DOI: 10.1246/BCSJ.71.1683

## Beschreibung

Die vorliegende Erfindung betrifft das Gebiet der Ketokörper und des damit zusammenhängenden Stoffwechsels sowie die Therapie von damit im Zusammenhang stehenden Erkrankungen.

Insbesondere betrifft die vorliegende Erfindung ein Verfahren zur Herstellung von Carbonsäureestern von 3-Hydroxybutanoat sowie die auf diese Weise erhältlichen bzw. hergestellten Reaktionsprodukte (d. h. Carbonsäureester von 3-Hydroxybutanoat) und deren Verwendung, insbesondere in pharmazeutischen Zusammensetzungen, wie Arzneimitteln oder Medikamenten, oder in Nahrungsmittel- und/oder Lebensmittelerzeugnissen, sowie deren weitere Anwendungen bzw. Verwendungen.

Des Weiteren betrifft die vorliegende Erfindung pharmazeutische Zusammensetzungen, insbesondere Arzneimittel oder Medikamente, welche die nach dem erfindungsgemäßen Herstellungsverfahren erhältlichen bzw. hergestellten Reaktionsprodukte (d. h. Carbonsäureester von 3-Hydroxybutanoat) umfassen, sowie deren Anwendungen bzw. Verwendungen.

Schließlich betrifft die vorliegende Erfindung Nahrungsmittel- und/oder Lebensmittelerzeugnisse, insbesondere Nahrungsergänzungsmittel, funktionelle Lebensmittel *(Functional Food), Novel Food,* Lebensmittelzusatzstoffe, Nahrungszusätze, diätetische Lebensmittel, Power-Snacks, Appetitzügler und Kraft- und/oder Ausdauersport-Supplements, welche die nach dem erfindungsgemäßen Herstellungsverfahren erhältlichen bzw. hergestellten Reaktionsprodukte (d. h. Carbonsäureester von 3-Hydroxybutanoat) umfassen, sowie deren Anwendungen bzw. Verwendungen.

Im menschlichen Energiestoffwechsel ist Glucose der kurzfristig zur Verfügung stehende Energieträger, welcher in den Mitochondrien unter Freisetzung von Wasser und Kohlendioxid zu Energie verstoffwechselt wird. Bereits durch die Schlafperiode während der Nacht sind aber die Glycogenspeicher der Leber geleert. Jedoch benötigen vor allem das menschliche zentrale Nervensystem (ZNS) und das Herz eine permanente Energieversorgung.

Die physiologische Alternative zu Glucose, welche vor allem dem zentralen Nervensystem zur Verfügung steht, sind die sogenannten Ketokörper (synonym auch als Ketonkörper bezeichnet oder Englisch auch als *"Keton Bodies"* bezeichnet).

Der Begriff der Ketokörper ist insbesondere eine Sammelbezeichnung für drei Verbindungen, welche vor allem in katabolen Stoffwechsellagen (wie z. B. bei Hunger, Reduktionsdiät oder kohlenhydratarmer Ernährung) gebildet werden und unter Umständen zu einer Ketose führen. Unter den Begriff der Ketokörper fasst man insbesondere die drei Verbindungen Acetoacetat (synonym auch Acetacetat genannt) und Aceton sowie 3-Hydroxybuttersäure (nachfolgend synonym auch als beta-Hydroxybuttersäure oder BHB oder 3-BHB bezeichnet) bzw. deren Salz (d. h. 3-Hydroxybutyrat oder beta-Hydroxybutyrat) zusammen, wobei letztere Verbindung die bedeutendste der drei vorgenannten Verbindungen ist. 3-Hydroxybuttersäure bzw. deren Salz kommt physiologisch als (R)-Enantiomer vor, d. h. als (R)-3-Hydroxybuttersäure (synonym auch (3R)-3-Hydroxybuttersäure genannt, um das Chiralitätszentrum in 3-Position hervorzuheben) bzw. deren Salz.

Diese Ketokörper werden auch in großer Zahl beim Fasten oder Hungern physiologisch aus im Körper eingelagerten Lipiden durch Lipolyse bereitgestellt und ersetzen den Energieträger Glucose fast vollständig.

Die Ketokörper werden in der Leber aus Acetyl-Coenzym A (= Acetyl-CoA) gebildet, welches aus der beta-Oxidation stammt; sie stellen eine transportable Form des Acetyl-Coenzyms A im menschlichen Körper dar. Zur Verwertung der Ketokörper müssen sich Gehirn und Muskeln aber zunächst umstellen, indem sie Enzyme exprimieren, welche zur Rückwandlung von Ketokörpern in Acetyl-Coenzym A benötigt werden. Insbesondere in Hungerzeiten tragen die Ketokörper einen beträchtlichen Anteil zur Energiegewinnung bei. So ist es beispielsweise dem Gehirn nach einiger Zeit möglich, mit nur einem Drittel der Tagesmenge an Glucose auszukommen.

Physiologisch erfolgt die Synthese der Ketokörper aus zwei Molekülen aktivierter Essigsäure in Form von Acetyl-Coenzym A, dem normalen Zwischenprodukt des Fettsäureabbaus, wobei zunächst mit Hilfe der Acetyl-Coenzym A-Acetyltransferase das Acetoacetyl-Coenzym A gebildet wird, welches unter Verwendung einer weiteren Acetyl-Coenzym A-Einheit und des Enzyms HMG-CoA-Synthase zum Zwischenprodukt 3-Hydroxy-3-methyl-glutaryl-CoA (HMG-CoA) verlängert wird, wobei schließlich die HMG-CoA-Lyase das Acetoacetat abspaltet. Diese drei Schritte finden ausschließlich in den Mitochondrien der Leber statt (Lynenzyklus), wobei 3-Hydroxybutyrat schließlich im Zytosol durch die D-beta-Hydroxybutyrat-Dehydrogenase entsteht. HMG-CoA ist außerdem ein Endprodukt beim Abbau der Aminosäure Leucin, während Acetoacetat beim Abbau der Aminosäuren Phenylalanin und Tyrosin entsteht.

Durch spontane Decarboxylierung entsteht aus Acetoacetat Aceton; es ist gelegentlich im Atem von Diabetikern und Diäthaltenden wahrzunehmen. Es kann vom Körper nicht weiterverwendet werden. Der Anteil von Aceton an den Ketokörpern ist allerdings gering.

Acetoacetat wird also reduktiv in die physiologisch relevante Form der 3-Hydroxybuttersäure bzw. des 3-Hydroxybutyrats überführt, kann aber auch unter Kohlenstoffdioxidfreisetzung in das physiologisch unbrauchbare Aceton zerfallen, was bei einer schweren Ketose, einer Ketoacidose (z. B. bei Diabetes mellitus Typ 1-Patienten ohne Insulinsubstitution), im Urin und in der Ausatemluft nachweisbar und olfaktorisch wahrnehmbar ist.

3-Hydroxybuttersäure wird derzeit im Bereich des Kraftsports als Natrium-, Magnesium- oder Calcium-Salz eingesetzt und in den Handel gebracht.

Jedoch ist 3-Hydroxybuttersäure für den Menschen evolutionär nicht oder in nur sehr geringer Menge bekannt, da Pflanzen keine 3-Hydroxybuttersäure produzieren und 3-Hydroxybuttersäure im tierischen Organismus nur bei toten ausgezehrten Tieren in der Ketose vorkommt, so dass 3-Hydroxybuttersäure bei peroraler Verabreichung Brechreiz auslöst. 3-Hydroxybuttersäure in Form der freien Säure sowie deren Salzen schmeckt zudem stark bitter und kann schweres Erbrechen und Übelkeit hervorrufen.

Zudem können Patienten, vor allem Neugeborene, aber auch Erwachsene größere Mengen an Salzen der 3-Hydroxybuttersäure nicht permanent verkraften, da diese Verbindungen nierenschädigend wirken können.

Außerdem ist die Plasmahalbwertszeit von 3-Hydroxybuttersäure und deren Salzen derart gering, dass selbst bei Einnahme von mehreren Gramm die Ketose nur für ca. drei bis vier Stunden vorhält, d. h. Patienten können insbesondere während der Nacht daher nicht von einer Therapie mit 3-Hydroxybuttersäure oder deren Salzen kontinuierlich profitieren. Bei Stoffwechselerkrankungen kann dies zu lebensbedrohlichen Situationen führen.

Daher werden im Fall der Therapie derartiger Stoffwechselerkrankungen heute sogenannte mittelkettige Triglyceride, sogenannte MCTs, für die ketogene Therapie eingesetzt, d. h. es wird die metabolische Umwandlung von Capron-, Capryl- und Caprinsäure (d. h. von gesättigten linearen C₆-, C₈- und C₁₀-Fettsäuren) aus den korrespondierenden Triglyceriden beabsichtigt.

Grundsätzlich stellt aber aus pharmazeutischer und klinischer Hinsicht 3-Hydroxybuttersäure demgegenüber ein wirksameres pharmazeutisch-pharmakologisches Zielmolekül, welches nach den Erkenntnissen des Standes der Technik prinzipiell für die Therapie einer Vielzahl von Erkrankungen zum Einsatz kommen könnte, aber aufgrund seiner mangelnden physiologischen Kompatibilität dort nicht zum Einsatz kommen kann (z. B. bei Erkrankungen im Zusammenhang mit einer Störung des Energiestoffwechsels, insbesondere Ketokörperstoffwechsels, oder neurodegenerativen Erkrankungen wie Demenz, Morbus Alzheimer, Morbus Parkinson etc., Fettstoffwechselerkrankungen usw.).

Die nachfolgende Tabelle veranschaulicht rein beispielhaft, aber keinesfalls beschränkend potentielle Therapiemöglichkeiten bzw. mögliche Indikationen für den Wirkstoff 3-Hydroxybuttersäure.

| **Indikation** | **Therapeutischer Effekt** |
|---|---|
| Schädel-Hirn-Trauma | Unter BHB sinkt die Apoptose und Nekrose-Rate von Nervenzellen. |
| Schlaganfall | Unter BHB sinkt die Apoptose und Nekrose-Rate von Nervenzellen. |
| Refeeding-Syndrom | Bei Anorexie, Absetzen enteraler oder parenteraler Ernährung und nach langen Hungerperioden kann der Konsum von Stärke oder Glucose zum Tod führen (siehe auch WHO-Schema Erdnusspaste). BHB kann hier als Therapeutikum zum schnelleren Erlangen einer normalen Nahrungsaufnahme eingesetzt werden. |
| Appetitzügler | BHB unterdrückt im Zentralnervensystem (ZNS) das Hungergefühl. |
| Epilepsie | Herkömmliche ketogene Diät zur signifikanten Reduzierung der Häufigkeit von Krampfanfällen hat extrem schlechte Patienten-Verträglichkeit. BHB bietet hier eine unmittelbar wirksame Alternative. |
| Morbus Alzheimer, Demenz | Unter BHB zeigen Patienten eine bessere kognitive Leistung. BHB ist auch für die Prävention von neurodegenerativen Erkrankungen wirksam. |
| Störungen der Fettsäureoxidation (z. B. Electron-Transfer-Protein Defekt) | Ausgleich eines Nährstoffmangels bei Defekt im Energiestoffwechsel. |

Daher ist es aus pharmazeutischer und klinischer Hinsicht wünschenswert, wirksame Präkursoren oder Metabolite auffinden zu können, welche physiologisch einen direkten oder indirekten Zugang zu 3-Hydroxybuttersäure oder deren Salzen ermöglichen, insbesondere im physiologischen Stoffwechsel des menschlichen oder tierischen Körpers.

Folglich hat es im Stand der Technik nicht an Versuchen gefehlt, physiologisch geeignete Präkursoren oder Metaboliten für 3-Hydroxybuttersäure bzw. deren Salze aufzufinden. Bislang wurden im Stand der Technik jedoch keine effizienten diesbezüglichen Verbindungen aufgefunden. Auch ist ein diesbezüglicher Zugang zu solchen Verbindungen nach dem Stand der Technik bislang nicht bzw. nicht ohne Weiteres möglich.

Die US 2019/177673 A1 betrifft ein Getränk, welches D-Ethyl-3-Hydroxybutyrat, D-1,3-Butandiol, D-β-Hydroxybutyratsalze, D-β-Hydroxybutyrat/D-1,3-Butandiol-monoester, D-Ethylhydroxybutyrat, Hydroxybutyrat und/oder D-Hydroxybuttersäure mit mindestens 0,5 Vol.-% enthält, wobei der Anteil an D-Ethyl-3-Hydroxybutyrat, D-1,3-Butandiol, D-β-Hydroxybutyratsalze, D-β-Hydroxybutyrat/D-1,3-Butandiolmonoester, D-Ethylhydroxybutyrat, Hydroxybutyrat und/oder D-Hydroxybuttersäure zwischen 5 und 19 % oder höher als 19 % liegt, wobei das Getränk durch die Einarbeitung von Geschmacksstoffen aus Hopfen und/oder anderen Zutaten verbessert werden kann.

Darüber hinaus betrifft die WO 2017/213999 A1 Fettsäure-β-Hydroxyester-Verbindungen, Fettsäureester von Butandiol und pharmazeutisch verträgliche Salze davon sowie pharmazeutische Zusammensetzungen mit einer oder mehreren Fettsäure-β-Hydroxyester-Verbindungen und/oder einem oder mehreren Fettsäureestern von Butandiol. Weiterhin betrifft Dokument die WO 2017/213999 A1 ein Verfahren zur Behandlung eines Patienten durch Verabreichung eines oder mehrerer Ester an die Patienten sowie Kits, welche ein oder mehrere der betreffenden Ester enthalten.

Die WO 2020/249198 A1 betrifft ein Verfahren zur Herstellung von Polyolestern, insbesondere Polyglycerinestern, der acylverkappten bzw. acylblockierten 3-Hydroxybuttersäure sowie die auf diese Weise erhältlichen Produkte und deren funktionalisierte (z. B. veresterte) Derivate sowie die verschiedenen Verwendungen und Anwendungen dieser Produkte.

Weiterhin betrifft die WO 2020/249197 A1 ein Verfahren zur Herstellung von gegebenenfalls funktionalisierten acylverkappten (acylblockierten) 3-Hydroxybuttersäuren und deren Salzen und Estern sowie die auf diese Weise erhältlichen Produkte und deren Verwendung.

Darüber hinaus betrifft die WO 2020/147981 A1 ein Verfahren zur Herstellung von verkappten (blockierten) 3-Hydroxybuttersäuren und deren Salzen und Estern sowie die auf diese Weise erhältlichen Produkte und deren Verwendung.

Weiterhin betrifft die WO 2019/147503 A1 ein Verfahren zur Herstellung von Hydroxybutyryl, 3-Hydroxybutyrat und ähnliche Verbindungen sowie Verwendungsverfahren davon.

Darüber hinaus betrifft Fujiwara Toshio et al.: "Application of a novel chromophoric reagent, 2,2'-binaphthyl-3,3'-dicarbonyl cyanide, to the absolute configuration determination of chiral secondary alcohols", Tetrahedron Letters, Bd. 61, Nr. 24, 1. Juni 2020, Seite 151984 die Synthese von 2,2'-Binaphthyl-3,3'-Dicarbonylcyanid mit zwei Reaktionsstellen, wobei das 2,2'-Binaphthyl-3,3'-Dicarbonylcyanid eine neue chromophore Reagenz für den exciton-gekoppelten Circulardichroismus (ECCD).

Die JP 2018-154559 A betrifft ein Verfahren zur Isolierung des R-Isomers von 3-Hydroxybuttersäureester zur Verbesserung der optischen Reinheit dieses R-Isomers, wobei das Verfahren ein Acylierungsschritt der Hydroxylgruppe an der 3-Position des 3-Hydroxybuttersäureesters beinhaltet, wobei das R-Isomer anschließend durch Lipase deacyliert wird und durch eine darauf folgende Destillierung separiert wird.

Weiterhin betrifft Raghavan Sadagopan et al.: "Synthesis of an advanced intermediate enroute to thiomarinol antibiotics", Tetrahedron, Bd. 73, Nr. 19, Seiten 2814-2823 die stereoselektive Synthese von C₁-C₁₄-Fragmenten von Thiomarinol, wobei das Verfahren die Schritte der stereoselektiven Herstellung eines allylischen Sulfids via eines Chlorosulfids durch 1,2-asymmetrische Induktion, Ringschluss-Metathese-Reaktion, Kirmse-Doyle-Reaktion zur Herstellung eines γ,δ-ungesättigten Esters, Nozaki-Hiyama-Kishi-Kupplung und Julia-Kocienski Olefinierungsreaktion umfasst.

Die JP 2016-193849 A betrifft eine Dicarbonsäure oder deren Ester, welche in der Lage ist, ein Polymer mit hohem Molekulargewicht zu bilden, selbst wenn viel Gerüst enthalten ist, das von 3-Hydroxybuttersäure mit sekundären Hydroxylgruppen abgeleitet ist, und ein Harz, welches diese Dicarbonsäure oder deren Ester als Polymerisationskomponenten enthält sowie ein entsprechendes Verfahren zur Herstellung der Dicarbonsäure oder deren Ester.

Darüber hinaus betrifft die US 2019/211144 A1 biologisch abbaubare Polymere sowie ein Verfahren zur Herstellung solcher Polymere, wobei gemäß einer Ausführungsform ein chemischer Syntheseweg zur Herstellung von biologisch abbaubarem Poly(3-hydroxybutyrat) mit hoher Isotaktizität und hohem Molekulargewicht offenbart wird, wobei diese Route racemisches achtgliedriges cyklisches Diolyt (racDL), meso-DL oder eine Mischung aus rac-DL und meso-DL, abgeleitet von biologisch gewonnenem Dimethylsuccinat, und enantiomerem (R,R)-DL und (S,S)-DL, optisch aufgelöst durch metallbasierte Katalysatoren herstellt.

Des Weiteren betrifft die WO 2016/012657 A1 Medikamente, welche auf oligomeren Formen von 3-Hydroxybutyrat, insbesondere 3-Hydroxybutyratmethylester-Dimeren und Trimeren, insbesondere zur Verwendung in der Behandlung, Verhinderung und/oder Hemmung der Entwicklung einer Störung oder eines Zustands im Zusammenhang mit oxidativem Stress, basieren. Weiterhin betrifft WO 2016/012657 A1 die Verwendung dieser Moleküle als Antioxidanten und ein Verfahren zur Erhöhung der Proliferation und Lebensfähigkeit von Pflanzenzellen.

Morley K L et al.: "Parallel synthesis of an ester library for substrate mapping of esterases and lipases", Tetrahedron Asymmetry, Pergamon Press LTD, Oxford, GB, Bd. 15, Nr. 18, 20. September 2004, Seiten 3005-3009 betrifft die Verwendung von feststoffgeschützten Reagenzien, welche die routinemäßige Acylierung von primärem und sekundärem Alkohol vereinfachen, da die herkömmliche Reinigung entfällt, wobei die Verwendung eines parallelen Synthesizers acht primäre und sekundäre Alkohole mit Säurechlorid in Gegenwart von Poly(4-vinylpyridin), das als Base und Acylierungskatalysator fungiert, reagiert, wobei anschließend eine Filtration und Zugabe von aminofunktionalisiertem Kieselgel zur Entfernung von überschüssigem Säurechlorid und eine zweite Filtration, die die entsprechenden Ester liefert, erfolgt.

Ru Ru H et al.: " 1-Hydroxytetraphenylcyclopentadienyl-(tetraphenyl-2,4-cyclopentadien-1-one)-hydrotetracarbonyldiruthenium(II)", 1.1.2009, Seiten 1-7, gefunden: URL:https//onlinelibrary.wiley.com/doi/pdfdirect/10.1002/0470 84289X. rn01063 (gefunden am 26.5.2021) betrifft 1-Hydroxytetraphenylcyclopentadienyl-(tetraphenyl-2,4-cyclopentadien-1-one)-µ-hydroxytetracarbonyldiruthenium(II) und dessen Verwendung als Katalysator bzw. als Prekatalysator in verschiedenen Reaktionen.

Darüber hinaus betrifft Häcker Christine et al.: "General Stereodivergent Enantioselective Total Synthetic Approach toward Macrsphelides A-G and M", J. Org. Chem., Bd. 80, Nr. 16, 21. August 2015, Seiten 8055-8064, gefunden: URL:https:/pubs.acs.org/doi/pdf/10.1021/acs.joc.5b01 171 einen allgemeinen stereodivergenten enantioselektiven total synthetischen Ansatz für Makrosphelide A-G und M, wobei neun bis elf Schritte notwendig sind und von tert-Butylsäurebad ausgegangen wird, wobei eine Hochdruckveresterung die selektive Ringvergrößerung des 15-gliedrigen Makrosphelids in ein 16-gliedriges Gegenstück ermöglicht.

Weiterhin betrifft die EP 1 746 110 A1 eine Katalysatorkomponente zur Polymerisation von Olefin CH₂=CHR, wobei R Wasserstoff oder C₁-C₁₂ Alkyl oder Aryl darstellt, umfassend Magnesium, Titan, ein Halogen und eine Elektronendonorverbindung (a), welche mindestens ausgewählt aus der Gruppe bestehend aus dibasischen Esterverbindungen der Formel (I) ist, und wobei die Katalysatorkomponente gegebenenfalls ferner eine Elektronendonorverbindung (b) ausgewählt aus der Gruppe bestehend aus aliphatischen Dicarbonsäureestern und aromatischen Dicarbonsäureestern, und/oder eine Elektronendonorverbindung (c) ausgewählt aus der Gruppe bestehend aus 1,3-Dietherverbindungen der Formel (IV), und einen Katalysator, umfassend die Katalysatorkomponente.

Beat M. Bachmann et al.: "Synthesis and Structure of Linear and Cyclic Oligomers of 3-Hydroxybutanoic Acid with Specific Sequences of (R)- and (S)-Configurations", Helvetica Chimica Acta, Bd. 81, Nr. 12, 16. Dezember 1998, Seiten 2430-2461 betrifft die Synthese und Struktur von linearen und cyklischen Oligomeren von 3-Hydroxybuttersäure mit spezifischen Sequenzen der (R)- und (S)-Konfigurationen.

Schließlich betrifft Li Jun et al.: "Conformational Analysis of Oligomers of (R)-3-Hydroxybutanoic Acid in Solutions by HNMR Spectroscopy", Bulletin of the Chemical Society of Japan, Bd. 71, Nr. 7, 1. Januar 1998, Seiten 1683-1689 Oligomere von (R)-3-Hydroxybuttersäure(3-HB) mit verschiedenen Endgruppen und Kettenlängen, welche als Modellkomponenten von bakteriellen [(R)-3-Hydroxybutanoat] [P(3-HB)] hergestellt sind, wobei deren konformatives Verhalten durch Rotation entlang der CH₂-CH der 3-HB Einheit in Lösung untersucht wird.

Das der vorliegenden Erfindung zugrundeliegende Problem liegt also in der Bereitstellung eines effizienten Herstellungsverfahrens von physiologisch geeigneten bzw. physiologisch kompatiblen Präkursoren und/oder Metaboliten von 3-Hydroxybuttersäure (d. h. beta-Hydroxybuttersäure bzw. BHB bzw. 3-BHB) oder deren Salzen.

Ein solches Verfahren soll insbesondere die betreffenden BHB-Präkursoren und/oder BHB-Metaboliten in effizienter Weise zugänglich machen, insbesondere auch in größeren Mengen und ohne nennenswerte Mengen an toxischen Nebenprodukten.

In vollkommen überraschender Weise hat die Anmelderin nunmehr herausgefunden, dass Carbonsäureester von 3-Hydroxybutanoaten (3-Hyroxybuttersäureestern) einen effizienten und physiologisch wirksamen bzw. physiologisch kompatiblen Präkursor und/oder Metaboliten für den Ketokörper 3-Hydroxybuttersäure bzw. deren Salze darstellen, und hat in diesem Zusammenhang ein effizientes Herstellungsverfahren für diese Verbindungen auffinden bzw. entwickeln können, welches einen direkten und wirksamen, insbesondere ökonomischen wie auch großtechnisch umsetzbaren Zugang zu diesen Verbindungen ermöglicht.

Zur Lösung des zuvor geschilderten Problems schlägt die vorliegende Erfindung daher - gemäß einem **ersten** Aspekt der vorliegenden Erfindung - ein Verfahren zur Herstellung von Carbonsäureestern von 3-Hydroxybutanoat gemäß Anspruch 1 vor; weitere, insbesondere besondere und/oder vorteilhafte Ausgestaltungen des erfindungsgemäßen Verfahrens sind Gegenstand der diesbezüglichen Verfahrensunteransprüche.

Weiterhin betrifft die vorliegende Erfindung - gemäß einem **zweiten** Aspekt der vorliegenden Erfindung - einen Carbonsäureester von 3-Hydroxybutanoat gemäß den diesbezüglichen Ansprüchen (Ansprüche 6 und 7) bzw. ein diesbezüglich erhältliches Gemisch von mindestens zwei Carbonsäureestern von 3-Hydroxybutanoat, gemäß dem diesbezüglichen Anspruch (Anspruch 8); weitere, insbesondere besondere und/oder vorteilhafte Ausgestaltungen dieses Erfindungsaspekts sind jeweils Gegenstand der diesbezüglichen Unteransprüche.

Gleichermaßen betrifft die vorliegende Erfindung - gemäß einem **dritten** Aspekt der vorliegenden Erfindung - eine pharmazeutische Zusammensetzung, insbesondere Arzneimittel oder Medikament, gemäß dem diesbezüglichen unabhängigen Anspruch (Anspruch 9); weitere, insbesondere besondere und/oder vorteilhafte Ausgestaltungen dieses Erfindungsaspekts sind Gegenstand des diesbezüglichen Unteranspruchs.

Darüber hinaus betrifft die vorliegende Erfindung - gemäß einem **vierten** Aspekt der vorliegenden Erfindung - ein erfindungsgemäßes Reaktionsprodukt bzw. einen erfindungsgemäßen Carbonsäureester von 3-Hydroxybutanoat bzw. ein erfindungsgemäßes Gemisch von mindestens zwei Carbonsäureestern von 3-Hydroxybutanoat zur prophylaktischen und/oder therapeutischen Behandlung bzw. zur Verwendung bei der prophylaktischen und/oder therapeutischen Behandlung von Erkrankungen des menschlichen oder tierischen Körpers gemäß dem diesbezüglichen unabhängigen Anspruch (Anspruch 11).

Des Weiteren betrifft die vorliegende Erfindung - gemäß einem **fünften** Aspekt der vorliegenden Erfindung - die Verwendung eines erfindungsgemäßen Reaktionsprodukts bzw. eines erfindungsgemäßen Carbonsäureesters von 3-Hydroxybutanoat bzw. eines erfindungsgemäßen Gemischs von mindestens zwei Carbonsäureestern von 3-Hydroxybutanoat zur prophylaktischen und/oder therapeutischen Behandlung bzw. zur Herstellung eines Arzneimittels zur prophylaktischen und/oder therapeutischen Behandlung von Erkrankungen des menschlichen oder tierischen Körpers gemäß dem diesbezüglichen unabhängigen Anspruch (Anspruch 12).

Darüber hinaus betrifft die vorliegende Erfindung - gemäß einem **sechsten** Aspekt der vorliegenden Erfindung - die Verwendung eines erfindungsgemäßen Reaktionsprodukts bzw. eines erfindungsgemäßen Carbonsäureesters von 3-Hydroxybutanoat bzw. eines erfindungsgemäßen Gemischs von mindestens zwei Carbonsäureestern von 3-Hydroxybutanoat gemäß dem diesbezüglichen unabhängigen Anspruch (Anspruch 13).

Des Weiteren betrifft die vorliegende Erfindung - gemäß einem **siebten** Aspekt der vorliegenden Erfindung - ein Nahrungsmittel und/oder Lebensmittelerzeugnis gemäß dem diesbezüglichen unabhängigen Anspruch (Anspruch 14); weitere, insbesondere besondere und/oder vorteilhafte Ausgestaltungen des erfindungsgemäßen Nahrungsmittel- und/oder Lebensmittelerzeugnisses sind Gegenstand des diesbezüglichen Unteranspruchs.

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Des Weiteren versteht es sich von selbst, dass einzelne Aspekte und Ausführungsformen der vorliegenden Erfindung auch in beliebiger Kombination mit anderen Aspekten und Ausführungsformen der vorliegenden Erfindung als offenbart gelten und insbesondere auch eine beliebige Kombination von Merkmalen und Ausführungsformen, wie sie sich aus den Rückbezügen aller Patentansprüche ergibt, umfangreich als offenbart gilt, und zwar im Hinblick auf alle sich ergebenden Kombinationsmöglichkeiten.

Bei allen nachstehend genannten relativen bzw. prozentualen gewichtsbezogenen Angaben, insbesondere relativen Mengen- oder Gewichtsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder aber einzelfallbedingt - von den nachfolgend angeführten Bereichsangaben erforderlichenfalls abweichen kann, ohne dass er den Rahmen der vorliegenden Erfindung verlässt. Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit ein Verfahren zur Herstellung von Carbonsäureestern von 3-Hydroxybutanoat,
wobei mindestens ein 3-Hydroxybutanoat der allgemeinen Formel (I)

CH₃-CH(OH)-CH₂-C(O)OR¹ (I)

wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
mit mindestens einer Carbonsäure (II), ausgewählt aus der Gruppe von Bernsteinsäure, Weinsäure, Milchsäure, Citronensäure, Äpfelsäure, Adipinsäure, Fumarsäure und Maleinsäure und deren Anhydriden sowie deren Kombinationen oder Mischungen, in einer Veresterungsreaktion und/oder unter Veresterungsbedingungen umgesetzt wird,
wobei die Umsetzung in Abwesenheit von Lösemitteln und/oder ohne jedwedes Lösemittel durchgeführt wird; und
wobei die Umsetzung in Abwesenheit eines Katalysators und/oder ohne jedweden Katalysator durchgeführt wird oder aber die Umsetzung in Gegenwart eines Enzyms als Katalysator durchgeführt wird,
so dass als Reaktionsprodukt (III) ein oder mehrere Carbonsäureester von 3-Hydroxybutanoat erhalten werden.

Gemäß der vorliegenden Erfindung wird somit ein Herstellungsverfahren für Carbonsäureester von 3-Hydroxybutanot bereitgestellt. 3-Hydroxybutanoat kann synonym auch als 3-Hydroxybuttersäureester oder aber auch als ein 4-Oxo-2-butanol bezeichnet werden.

Genau genommen wird als 3-Hydroxybutanoat der allgemeinen Formel (I) entweder ein (C₁-C₅-Alkyl)-3-hydroxybutanoat (= 3-Hydroxybuttersäure-(C₁-C₅-alkyl)-ester), d. h. ein C₁-C₅-Alkyester der 3-Hydroxybuttersäure, welcher synonym auch als ein 4-Oxo-4-(C₁-C₅-alkoxy)-2-butanol bezeichnet werden kann, oder aber ein (Hydroxy-C₃-C₅-alkyl)-3-hydroxybutanoat (= 3-Hydroxybuttersäure-(hydroxy-C₃-C₅-alkyl)-ester), d. h. ein Hydroxy-C₃-C₅-alkyester der 3-Hydroxybuttersäure, welcher synonym auch als ein 4-Oxo-4-(hydroxy-C₃-C₅-alkoxy)-2-butanol bezeichnet werden kann, eingesetzt.

In diesem Zusammenhang können die 3-Hydroxybutanoate durch Veresterung der freien 3-Hydroxybuttersäure mit dem entsprechenden Alkohol (z.B. Monoalkohol oder Diol) hergestellt werden. Im Folgenden ist die Synthese von Hydroxybutyl-3-hydroxybutanoat (d. h. 3-Hydroxybutanoat der allgemeinen Formel (I) mit R¹ = Hydroxybutyl) schematisch dargestellt:

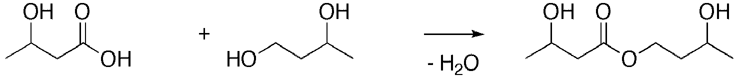

Weiterhin ist im Folgenden die Synthese von Hydroxypentyl-3-hydroxybutanoat (d. h. 3-Hydroxybutanoat der allgemeinen Formel (I) mit R¹ = Hydroxypentyl) schematisch dargestellt:

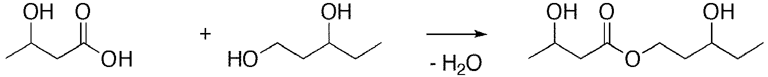

Die Herstellung bzw. Synthese weiterer 3-Hydroxybutanoate verläuft analog.

Im Rahmen des erfindungsgemäßen Verfahrens fungiert also das Edukt bzw. die Ausgangsverbindung 3-Hydroxybutanoat der allgemeinen Formel (I) über die Hydroxylgruppe in 3-Position als Veresterungs-Alkohol und reagiert hierüber mit der Carboxylgruppe der Carbonsäure (II), sodass als Reaktionsprodukt (III) ein entsprechender Carbonsäureester von 3-Hydroxybutanoat, d. h. ein entsprechender Carbonsäureester von 4-Oxo-4-(C₁-C₅-alkoxy)-2-butanol bzw. 4-Oxo-4-(hydroxy-C₃-C₅-alkoxy)-2-butanol, gebildet wird.

Im Rahmen der vorliegenden Erfindung ist die eingesetzte Carbonsäure (II) eine organische Carbonsäure. D. h. die Carbonsäure (II) ist eine organische Verbindung mit einer oder mehreren Carboxylgruppen (-COOH), welche einen aciden Charakter aufweist.

Überraschenderweise hat die Anmelderin eine Möglichkeit gefunden, 3-Hydroxybuttersäure bzw. deren Derivat in einer organoleptisch kompatiblen Form bereitzustellen, wobei die 3-Hydroxybuttersäure dennoch gut freigesetzt werden kann, insbesondere vom tierischen oder menschlichen Körper.

Darüber hinaus ist es der Anmelderin gelungen, die organoleptisch kompatible Form der 3-Hydroxybuttersäure derart bereitzustellen, dass ein Retardierungseffekt vorliegt; d. h. die 3-Hydroxybuttersäure wird über einen längeren Zeitraum kontinuierlich freigesetzt, insbesondere vom menschlichen oder tierischen Körper.

Außerdem sind auch die weiteren Abspaltprodukte (d. h. die Abspaltprodukte, welche neben bzw. zusätzlich zu der 3-Hydroxybuttersäure freigesetzt werden) vom Körper verwertbar, zumindest aber vom Körper verarbeitbar. Insbesondere werden Abspaltprodukte freigesetzt, welche Edukte, Produkte oder Zwischenprodukte des Citratzyklus sind oder aber Derivate bzw. Salze hiervon sind, welche durch Oxidation eines Edukts, Produkts oder Zwischenprodukts des Citratzyklus gebildet werden. Somit können auch die weiteren Abspaltprodukte, welche bei der Freisetzung von 3-Hydroxybuttersäure gebildet werden, als Energiequelle vom tierischen oder menschlichen Körper genutzt werden. Bei diesen Abspaltprodukten handelt es sich typischerweise um die Carbonsäure (II) bzw. deren Salze oder Derivate.

Bei der Verwendung von Carbonsäuren mit mindestens zwei freien Carboxylgruppen, ist es möglich, dass mehrere 3-Hydroxybutanoate mit einer Carbonsäure reagieren (d. h. mehrere 3-Hydroxybutanoate werden an eine Carbonsäure addiert bzw. einer Carbonsäure wird mit mehreren 3-Hydroxybutanoaten verestert), sodass eine hohe Dichte an 3-Hydroxybutanoaten innerhalb eines Moleküls und somit eine hohe Wirkstoffdichte vorliegt.

Wie zuvor ausgeführt, hat die Anmelderin nämlich vollkommen überraschend herausgefunden, dass die auf diese Weise hergestellten Carbonsäureester von 3-Hydroxybutanoat, insbesondere Carbonsäureester von 4-Oxo-4-(C₁-C₅-alkoxy)-2-butanol oder von 4-Oxo-4-(hydroxy-C₃-C₅-alkoxy)-2-butanol, effiziente, da physiologisch verträgliche Präkursoren und/oder Metabolite der 3-Hydroxybuttersäure bzw. deren Salzen darstellen, welche pharmazeutisch bzw. klinisch auch in größeren Mengen zum Einsatz kommen können, da sie physiologisch kompatibel sind.

Die vorgenannten Carbonsäureester von 3-Hydroxybutanoat, insbesondere von 4-Oxo-4-(C₁-C₅-alkoxy)-2-butanol oder von 4-Oxo-4-(hydroxy-C₃-C₅-alkoxy)-2-butanol, welche durch das erfindungsgemäße Herstellungsverfahren erstmals in effizienter Weise zugänglich sind, stellen eine physiologisch und pharmakologisch relevante Alternative zu der freien 3-Hydroxybuttersäure bzw. deren Salzen dar.

Die Herstellung von Carbonsäureestern von 3-Hydroxybutanoat, insbesondere Carbonsäureestern von 4-Oxo-4-(C₁-C₅-alkoxy)-2-butanol oder von 4-Oxo-4-(hydroxy-C₃-C₅-alkoxy)-2-butanol, mittels herkömmlicher organischer Synthese ist komplex und aufwendig, da 3-Hydroxybuttersäure sowie deren Salze und Ester verstärkt zur Polymerisation und anderen unerwünschten Nebenreaktionen (z. B. Wasserabspaltung, Zersetzung etc.) neigen. Im Rahmen der vorliegenden Erfindung konnte erstmals ein effizient arbeitendes Herstellungsverfahren bereitgestellt werden, mit welchem sich Carbonsäureester von 3-Hydroxybutanoat, insbesondere Carbonsäureester von 4-Oxo-4-(C₁-C₅-alkoxy)-2-butanol oder von 4-Oxo-4-(hydroxy-C₃-C₅-alkoxy)-2-butanol, zumindest im Wesentlichen ohne unerwünschte Nebenreaktionen herstellen lassen, insbesondere einstufig.

Das erfindungsgemäße Verfahren ermöglicht somit erstmalig die Bereitstellung untoxischer Carbonsäureester von 3-Hydroxybutanoat, insbesondere Carbonsäureester von 4-Oxo-4-(C₁-C₅-alkoxy)-2-butanol oder von 4-Oxo-4-(hydroxy-C₃-C₅-alkoxy)-2-butanol, aus an sich bekannten, kommerziell verfügbaren und vor allem physiologisch unbedenklichen Komponenten bzw. Edukten (Ausgangsverbindungen). Die resultierenden Carbonsäureester von 3-Hydroxybutanoat, insbesondere Carbonsäureester von 4-Oxo-4-(C₁-C₅-alkoxy)-2-butanol oder von 4-Oxo-4-(hydroxy-C₃-C₅-alkoxy)-2-butanol, können physiologisch, insbesondere im Magen und/oder im Darm, aufgespalten werden und das Zielmolekül "3-Hydroxybuttersäure" bzw. deren Salze oder Ester als Wirkstoff bzw. Wirkkomponente freisetzen bzw. generieren.

Darüber hinaus weisen die vorgenannten Carbonsäureester von 3-Hydroxybutanoat, insbesondere Carbonsäureester von 4-Oxo-4-(C₁-C₅-alkoxy)-2-butanol oder von 4-Oxo-4-(hydroxy-C₃-C₅-alkoxy)-2-butanol, auch einen akzeptablen Geschmack auf, um eine Kompatibilität auch bei oraler Verabreichung größerer Mengen über einen längeren Zeitraum zu gewährleisten (z. B. Verabreichung von 50 g Tagesdosis oder mehr).

Gleichermaßen ermöglicht es das erfindungsgemäße Herstellungsverfahren, die Carbonsäureester von 3-Hydroxybutanoat, insbesondere Carbonsäureester von 4-Oxo-4-(C₁-C₅-alkoxy)-2-butanol oder von 4-Oxo-4-(hydroxy-C₃-C₅-alkoxy)-2-butanol, frei von toxischen Verunreinigungen bereitzustellen.

Darüber hinaus kann bei entsprechenden Ausgangsmaterialien die Herstellung auch enantioselektiv durchgeführt werden. So ermöglicht es beispielsweise das erfindungsgemäße Herstellungsverfahren, die biologisch relevante Form, d. h. das (R)-Enantiomer anzureichern, beispielsweise durch Enzymkatalyse oder die gezielte Auswahl der eingesetzten Ausgangsverbindungen (Edukte), um bei oraler Verabreichung das renale System von Patienten nicht zu belasten (d. h. Elimination über die Nieren). Grundsätzlich ist es aber auch möglich und kann es unter bestimmten Voraussetzungen zweckdienlich sein, das (S)-Enantiomer anzureichern.

Darüber hinaus ist das erfindungsgemäße Herstellungsverfahren, einschließlich optionaler Weiterverarbeitungs- bzw. Aufreinigungsverfahrensschritte, wirtschaftlich bzw. ökonomisch betreibbar und auch großtechnisch umsetzbar.

Insbesondere verwendet das erfindungsgemäße Herstellungsverfahren kommerziell verfügbare Edukte oder Edukte, welche durch einfache und großtechnisch durchführbare Verfahren synthetisiert werden können, und ermöglicht darüber hinaus insgesamt eine relativ einfache Verfahrensführung auch bei großtechnischer Umsetzung.

Im Gegensatz zu herkömmlichen Herstellungsverfahren des Standes der Technik kommt das erfindungsgemäße Herstellungsverfahren ohne komplexe Edukte und ohne die Verwendung von Schutzgruppen aus und verläuft nur einstufig. Dennoch werden im Rahmen des erfindungsgemäßen Herstellungsverfahrens exzellente Ausbeuten erzielt, wobei in gleicher Weise die Bildung von Nebenprodukten minimiert bzw. vermieden wird.

Darüber hinaus ist das erfindungsgemäße Verfahren einfach und wirtschaftlich. Insbesondere wird das erfindungsgemäße Verfahren in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*); folglich sind die erhaltenen Reaktionsprodukte nicht mit Lösemittel verunreinigt und es muss kein Lösemittel nach Durchführung des Verfahrens bzw. der Reaktion aufwendig und energieträchtig entfernt und entsorgt bzw. rezykliert werden. Es werden zudem auch keine toxischen Nebenprodukte gebildet.

Das erfindungsgemäße Herstellungsverfahren führt üblicherweise zu einem Gemisch verschiedener Carbonsäureester von 3-Hydroxybutanoat, insbesondere verschiedener Carbonsäureester von 4-Oxo-4-(C₁-C₅-alkoxy)-2-butanol oder von 4-Oxo-4-(hydroxy-C₃-C₅-alkoxy)-2-butanol, d. h. zu einem Gemisch von mindestens zwei voneinander verschiedenen Carbonsäureestern von 3-Hydroxybutanoat, insbesondere Carbonsäureestern von 4-Oxo-4-(C₁-C₅-alkoxy)-2-butanol oder von 4-Oxo-4-(hydroxy-C₃-C₅-alkoxy)-2-butanol. Das resultierende Rohreaktionsprodukt bzw. Rohgemisch kann mit an sich bekannten Methoden aufgereinigt werden, insbesondere von gegebenenfalls noch vorhandenen Edukten und/oder gegebenenfalls vorhandenen Nebenprodukten befreit werden, und darüber hinaus - sofern gewünscht - mit ebenfalls an sich bekannten Methoden aufgespalten werden, insbesondere destillativ und/oder chromatographisch (z. B. Fraktionierung in die einzelnen Carbonsäureester von 3-Hydroxybutanoat, d. h. z. B. Trennung des entsprechenden Monoesters, Diesters etc., oder aber Fraktionierung in Fraktionen mit angereicherten und abgereicherten Anteilen einzelner etc.).

Wie zuvor ausgeführt, betrifft die vorliegende Erfindung gemäß dem ersten Aspekt ein Verfahren zur Herstellung von Carbonsäureestern von 3-Hydroxybutanoat,
wobei mindestens ein 3-Hydroxybutanoat der allgemeinen Formel (I)

CH₃-CH(OH)-CH₂-C(O)OR¹ (I)

wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
mit mindestens einer Carbonsäure (II), ausgewählt aus der Gruppe von Bernsteinsäure, Weinsäure, Milchsäure, Citronensäure, Äpfelsäure, Adipinsäure, Fumarsäure und Maleinsäure und deren Anhydriden sowie deren Kombinationen oder Mischungen, in einer Veresterungsreaktion und/oder unter Veresterungsbedingungen umgesetzt wird,
wobei die Umsetzung in Abwesenheit von Lösemitteln und/oder ohne jedwedes Lösemittel durchgeführt wird; und
wobei die Umsetzung in Abwesenheit eines Katalysators und/oder ohne jedweden Katalysator durchgeführt wird oder aber die Umsetzung in Gegenwart eines Enzyms als Katalysator durchgeführt wird,
so dass als Reaktionsprodukt (III) ein oder mehrere Carbonsäureester von 3-Hydroxybutanoat erhalten werden.

Gemäß einer besonderen Ausführungsform der vorliegenden Erfindung kann die Verbindung der allgemeinen Formel (I) in racemischer Form oder in Form des (R)-Enantiomers eingesetzt werden. Die (R)-Konfiguration bezieht sich auf das chirale Kohlenstoffatom in 3-Position der Verbindung der allgemeinen Formel (I).

Erfindungsgemäß bevorzugt ist es, wenn in der allgemeinen Formel (I) der Rest R¹ Ethyl darstellt.

Mit anderen Worten ist es erfindungsgemäß bevorzugt, dass als Verbindung der allgemeinen Formel (I) Ethyl-3-hydroxybutyrat (synonym auch als 3-Hydroxybuttersäureethylester bzw. 4-Ethoxy-4-oxo-2-butanol bezeichnet) der Formel CH₃-CH(OH)-CH₂-C(O)OC₂H₅ eingesetzt wird.

Dies ermöglicht eine besonders effiziente Verfahrensführung und hohe Ausbeuten mit minimierter bzw. unterdrückter Nebenproduktbildung. Zudem ist der 3-Hydroxybuttersäureethylester bzw. 4-Ethoxy-4-oxo-2-butanol auch in großen Mengen kommerziell verfügbar und kann insbesondere großtechnisch z. B. durch Claisen-Kondensation von Ethylacetat gewonnen werden.

Gemäß einer besonderen Ausführungsform der vorliegenden Erfindung kann die Carbonsäure (II) in Form der freien Carbonsäure, in Form eines Salzes der Carbonsäure, in Form des Carbonsäureanhydrids oder in Form eines Esters der Carbonsäure, insbesondere in Form der freien Carbonsäure oder in Form des Carbonsäureanhydrids, eingesetzt werden.

Insbesondere durch die Verwendung einer zuvor definierten Carbonsäure mit mindestens einer, vorzugsweise zwei endständigen bzw. primären Carboxylgruppen kann die Veresterungsreaktion zwischen der Carbonsäure (II) und dem 3-Hydroxybutanoat besonders effektiv und mit minimierter Nebenproduktbildung ablaufen, ohne dass extreme Reaktionsbedingungen (z. B. sehr hohe Temperatur, sehr hoher Druck etc.) notwendig sind. Darüber hinaus kann durch die Anzahl der vorhandenen Carboxylgruppen die Wirkstoffdichte (d. h. insbesondere 3-Hydroxybutanoat-Dichte) beeinflusst werden.

Gemäß dem erfindungsgemäßen Verfahren ist die Carbonsäure (II) ausgewählt aus der Gruppe von Bernsteinsäure, Weinsäure, Milchsäure, Citronensäure, Äpfelsäure, Adipinsäure, Fumarsäure und Maleinsäure und deren Anhydriden sowie deren Kombinationen oder Mischungen, insbesondere ausgewählt ist aus der Gruppe von Bernsteinsäure, Weinsäure, Milchsäure, Citronensäure, Äpfelsäure, Adipinsäure und Fumarsäure und deren Anhydriden sowie deren Kombinationen oder Mischungen.

Die zuvor genannten Carbonsäuren eignen sich besonders für die Umsetzung mit 3-Hydroxybutanoaten und sind zudem kommerziell erhältlich.

Insbesondere ist es im Rahmen des erfindungsgemäßen Verfahrens bevorzugt, wenn die Carbonsäure (II) eine natürlich vorkommende Carbonsäure oder deren Anhydrid oder Derivat, insbesondere Umsetzungsprodukt, ist, insbesondere eine im menschlichen und/oder tierischen Stoffwechsel vorkommende Carbonsäure oder deren Anhydrid oder Derivat, insbesondere Umsetzungsprodukt.

Insbesondere ist es in diesem Zusammenhang vorteilhaft, wenn Carbonsäuren oder deren Anhydride oder Derivate verwendet werden, welche im Citratzyklus vorkommen, aus dem Citratzyklus entstehen oder mit dem Citratzyklus im Zusammenhang stehen. Dabei können Derivate beispielsweise Salze darstellen, welche durch Oxidation eines Stoffwechselprodukts (beispielsweise aus dem Citratzyklus) erhältlich sind. Durch die Verwendung von Carbonsäuren oder deren Anhydriden oder Derivaten, welche Teil des menschlichen und/oder tierischen Stoffwechsels bzw. Edukts bzw. Produkts bzw. Zwischenprodukts eines menschlichen und/oder tierischen Stoffwechsels darstellen, kann bei der Verwendung des erfindungsgemäßen Reaktionsprodukts dem menschlichen und/oder tierischen Körper eine weitere Energiequelle (zusätzlich zu dem Ketokörper 3-Hydroxybuttersäure bzw. 3-Hydroxybutanoat) bereitgestellt werden und die Reaktionsprodukte sind besonders geeignet für die Verwendung in oder als Arzneimittel, Medikament oder Nahrungsmittel- und Lebensmittelerzeugnis.

Weiterhin kann es im Rahmen des erfindungsgemäßen Verfahrens auch bevorzugt sein, wenn die Carbonsäure (II) ein lebensmittelrechtlich zugelassener Inhaltsstoff, insbesondere Zusatzstoff, ist.

Lebensmittelrechtlich zugelassene Inhaltsstoffe bzw. Zusatzstoffe sind solche, welche zur Verwendung in Lebensmitteln in bestimmten Mengen zugelassen sind und keine Gesundheitsrisiken aufweisen. EU-weit wird eine Liste für Lebensmittelzusatzstoffe geführt, worin jeder Lebensmittelzusatzstoff eine eigene Kennzeichnung (sogenannte E-Nummer) erhält. Beispielsweise werden die folgenden Carbonsäuren in der Lebensmittelzusatzstoffliste geführt: Bernsteinsäure (E363), Weinsäure (E334), Milchsäure (E270), Citronensäure (E330), Äpfelsäure (E296), Adipinsäure (E355) und Fumarsäure (E297). Diese Säuren sind alle Teil des Citratzyklus oder durch Oxidation eines Stoffwechselprodukts des Citratzyklus erhältlich. Der Citratzyklus ist ein Kreislauf biochemischer Reaktionen, der eine wichtige Rolle im Stoffwechsel (Metabolismus) aerober Zellen von Lebewesen spielt und hauptsächlich dem oxidativen Abbau organischer Stoffe zum Zweck der Energiegewinnung und der Bereitstellung von Zwischenprodukten für Biosynthesen dient. Somit können die Säuren, welche bei Verwendung des aus dem erfindungsgemäßen Verfahren erhältlichen Reaktionsprodukts (III) durch Abbau gebildet werden, als eine weitere alternative Energiequelle vom Körper verwertet werden.

Die vorliegende Erfindung betrifft gemäß diesem Erfindungsaspekt somit ein Verfahren zur Herstellung von Carbonsäureestern von 3-Hydroxybutanoat, insbesondere Carbonsäureestern von 4-Oxo-4-(C₁-C₅-alkoxy)-2-butanol oder von 4-Oxo-4-(hydroxy-C₃-C₅-alkoxy)-2-butanol,
wobei mindestens ein 3-Hydroxybutanoat, insbesondere ein 4-Oxo-4-(C₁-C₅-alkoxy)-2-butanol oder ein 4-Oxo-4-(hydroxy-C₃-C₅-alkoxy)-2-butanol, der allgemeinen Formel (I)

   CH₃-CH(OH)-CH₂-C(O)OR¹ (I)
wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
mit mindestens einer Carbonsäure (II), ausgewählt aus der Gruppe von Bernsteinsäure, Weinsäure, Milchsäure, Citronensäure, Äpfelsäure, Adipinsäure, Fumarsäure und Maleinsäure und deren Anhydriden sowie deren Kombinationen oder Mischungen, insbesondere ausgewählt ist aus der Gruppe von Bernsteinsäure, Weinsäure, Milchsäure, Citronensäure, Äpfelsäure, Adipinsäure und Fumarsäure und deren Anhydriden sowie deren Kombinationen oder Mischungen, in einer Veresterungsreaktion und/oder unter Veresterungsbedingungen umgesetzt wird,
wobei die Umsetzung in Abwesenheit von Lösemitteln und/oder ohne jedwedes Lösemittel durchgeführt wird; und
wobei die Umsetzung in Abwesenheit eines Katalysators und/oder ohne jedweden Katalysator durchgeführt wird oder aber die Umsetzung in Gegenwart eines Enzyms als Katalysator durchgeführt wird,
so dass als Reaktionsprodukt (III) ein oder mehrere Carbonsäureester von 3-Hydroxybutanoat, insbesondere ein oder mehrere Carbonsäureester von 4-Oxo-4-(C₁-C₅-alkoxy)-2-butanol oder von 4-Oxo-4-(hydroxy-C₃-C₅-alkoxy)-2-butanol, erhalten werden.

Gemäß einer weiteren bevorzugten Ausführungsform betrifft die vorliegende Erfindung gemäß diesem Erfindungsaspekt auch ein Verfahren zur Herstellung von Carbonsäureestern von 3-Hydroxybutanoat, insbesondere Carbonsäureestern von 4-Oxo-4-(C₁-C₅-alkoxy)-2-butanol oder von 4-Oxo-4-(hydroxy-C₃-C₅-alkoxy)-2-butanol, insbesondere ein wie zuvor beschriebenes Verfahren,
wobei mindestens ein 3-Hydroxybutanoat, insbesondere ein 4-Oxo-4-(C₁-C₅-alkoxy)-2-butanol oder ein 4-Oxo-4-(hydroxy-C₃-C₅-alkoxy)-2-butanol, der allgemeinen Formel (I)

   CH₃-CH(OH)-CH₂-C(O)OR¹ (I)
wobei in der allgemeinen Formel (I) der Rest R¹ Ethyl darstellt,
mit mindestens einer Carbonsäure (II), ausgewählt aus der Gruppe von Bernsteinsäure, Weinsäure, Milchsäure, Citronensäure, Äpfelsäure, Adipinsäure, Fumarsäure und Maleinsäure und deren Anhydriden sowie deren Kombinationen oder Mischungen, insbesondere ausgewählt ist aus der Gruppe von Bernsteinsäure, Weinsäure, Milchsäure, Citronensäure, Äpfelsäure, Adipinsäure und Fumarsäure und deren Anhydriden sowie deren Kombinationen oder Mischungen, in einer Veresterungsreaktion und/oder unter Veresterungsbedingungen umgesetzt wird,
wobei die Umsetzung in Abwesenheit von Lösemitteln und/oder ohne jedwedes Lösemittel durchgeführt wird; und
wobei die Umsetzung in Abwesenheit eines Katalysators und/oder ohne jedweden Katalysator durchgeführt wird oder aber die Umsetzung in Gegenwart eines Enzyms als Katalysator durchgeführt wird,
so dass als Reaktionsprodukt (III) ein oder mehrere Carbonsäureester von 3-Hydroxybutanoat, insbesondere ein oder mehrere Carbonsäureester von 4-Oxo-4-(C₁-C₅-alkoxy)-2-butanol oder von 4-Oxo-4-(hydroxy-C₃-C₅-alkoxy)-2-butanol, erhalten werden.

Gemäß der vorliegenden Erfindung wird die Umsetzung in Abwesenheit von Lösemitteln und/oder ohne jedwedes Lösemittel durchgeführt werden. D. h. die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Gemäß der vorliegenden Erfindung wird die Umsetzung in Abwesenheit eines Katalysators und/oder ohne jedweden Katalysator durchgeführt werden, oder aber die Umsetzung wird alternativ in Gegenwart eines Enzyms als Katalysator durchgeführt. Insbesondere kann der Katalysator nach Umsetzung rezykliert werden.

Im Rahmen des erfindungsgemäßen Verfahrens ist es insbesondere bevorzugt, wenn die Umsetzung in Abwesenheit von Lösemitteln und/oder ohne jedwedes Lösemittel durchgeführt wird und wenn die Umsetzung in Abwesenheit eines Katalysators und/oder ohne jedweden Katalysator durchgeführt wird.

Durch die Abwesenheit von Lösemitteln und die Abwesenheit eines Katalysators, können die Reaktionsprodukte nicht durch unvollständig entferntes Lösemittel oder unvollständig entferntem Katalysator verunreinigt werden. Darüber hinaus fallen die energieträchtigen und aufwendigen Entfernungs- bzw. Abtrennungsschritte weg. Überaschenderweise ist das erfindungsgemäße Verfahren gemäß dieser bevorzugten Ausführungsform trotzdem wirtschaftlich und resultiert in hohen Umsätzen ohne signifikante Nebenproduktbildung.

Wie zuvor ausführt, kann gemäß einer Ausführungsform des erfindungsgemäßen Herstellungsverfahrens die Umsetzung in Abwesenheit eines Katalysators und/oder ohne jedweden Katalysator durchgeführt werden.

Sofern die Umsetzung in Abwesenheit eines Katalysators und/oder ohne jedweden Katalysator durchgeführt wird, ist es bevorzugt, wenn die Umsetzung bei Temperaturen im Bereich von 20 °C bis 160°C, insbesondere im Bereich von 50 °C bis 150°C, vorzugsweise im Bereich von 70 °C bis 140°C, besonders bevorzugt im Bereich von 80 °C bis 135 °C, ganz besonders bevorzugt im Bereich von 100 °C bis 130 °C, durchgeführt wird.

Im Fall der Umsetzung in Abwesenheit eines Katalysators, kann auch der angewendete Druckbereich in weiten Bereichen variieren. Insbesondere kann die Umsetzung in Abwesenheit eines Katalysators und/oder ohne jedweden Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Bei der Umsetzung in Abwesenheit eines Katalysators, ist es bevorzugt, wenn die Umsetzung in Gegenwart eines Inertgases, insbesondere in Gegenwart von Helium, Argon oder Stickstoff, bevorzugt in Gegenwart von Stickstoff, durchgeführt wird. Insbesondere können hierdurch unerwünschte Nebenreaktionen, insbesondere aufgrund von Oxidation oder Hydrolyse, verhindert werden.

Alternativ zu dieser Ausführungsform ist es aber auch möglich, die Umsetzung z. B. in Gegenwart eines Enzyms als Katalysator durchzuführen.

Dabei kann das Enzym insbesondere ausgewählt sein aus Synthetasen (Ligasen), Katalasen, Esterasen, Lipasen und deren Kombinationen. Erfindungsgemäß werden als Synthetasen (synonym Ligasen) insbesondere Enzyme aus der Klasse der Ligasen bezeichnet; Ligasen sind Enzyme, welche das Verknüpfen zweier oder mehrerer Moleküle durch eine kovalente Bindung katalysieren. Katalasen im Sinne der vorliegenden Erfindung sind insbesondere Enzyme, welche Wasserstoffperoxid zu Sauerstoff und Wasser umzusetzen imstande sind. Der Begriff der Esterasen bezeichnet insbesondere Enzyme, welche imstande sind, Ester hydrolytisch in Alkohol und Säure aufzuspalten (Verseifung); es handelt sich somit insbesondere um Hydrolasen, wobei fettspaltende Esterasen auch als Lipasen bezeichnet werden. Lipasen im Sinne der vorliegenden Erfindung sind insbesondere Enzyme, welche von Lipiden, wie Glyceriden, freie Fettsäuren abzuspalten imstand sind (Lipolyse).

In diesem Zusammenhang kann sich das als Katalysator eingesetzte Enzym insbesondere ableiten von *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei (Rhizomucor miehei)* und *Thermomyces lanuginosus.*

Gemäß einer besonderen Ausführungsform kann das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt werden.

Im Rahmen der vorliegenden Erfindung ist es bevorzugt, wenn im Fall der Verwendung eines Enzyms als Katalysator das Enzym nach der Umsetzung rezykliert wird.

Sofern die Umsetzung im Rahmen des erfindungsgemäßen Herstellungsverfahrens in Gegenwart eines Enzyms als Katalysator durchgeführt wird, ist es bevorzugt, wenn die Umsetzung bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird.

Im Fall der Verwendung eines Enzyms als Katalysator kann die Menge des eingesetzten Enzyms in weiten Bereichen variieren. Insbesondere kann das Enzym in Mengen, bezogen auf die Gesamtmenge der Ausgangsverbindungen (I) und (II), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden. Dennoch kann es einzelfallbedingt oder anwendungsbezogen erforderlich sein, von den vorgenannten Mengen abzuweichen, ohne dass der Rahmen der vorliegenden Erfindung verlassen ist.

Wenn gemäß einer besonderen Ausführungsform der vorliegenden Erfindung die Umsetzung in Gegenwart eines Enzyms als Katalysator durchgeführt wird, kann auch der angewendete Druckbereich in weiten Bereichen variieren. Typischerweise kann die Umsetzung in Gegenwart eines Enzyms als Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 0,5 bar, durchgeführt werden.

Gemäß der besonderen Ausführungsform der vorliegenden Erfindung, wonach die Umsetzung in Gegenwart eines Enzyms als Katalysator durchgeführt wird, ist es bevorzugt, wenn die Umsetzung in Gegenwart eines Inertgases, insbesondere in Gegenwart von Helium, Argon oder Stickstoff, bevorzugt in Gegenwart von Stickstoff, durchgeführt wird. Wie zuvor bereits im Zusammenhang mit der Umsetzung in Abwesenheit eines Katalysators ausgeführt, können durch die Umsetzung in Gegenwart eines Inertgases unerwünschte Nebenreaktionen, insbesondere aufgrund von Oxidation oder Hydrolyse, verhindert werden.

Was die Menge an Edukten bzw. Ausgangsverbindungen insgesamt anbelangt, so kann diese in weiten Bereichen variiert werden.

Unter Berücksichtigung von Verfahrensökonomie und Optimierung des Verfahrensablaufs, insbesondere im Hinblick auf die Minimierung von Nebenprodukten, ist es vorteilhaft, wenn das 3-Hydroxybutanoat der allgemeinen Formel (I), bezogen auf die Carboxylgruppen der Carbonsäure (II) in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird.

Gleichermaßen unter Berücksichtigung von Verfahrensökonomie und Optimierung des Verfahrensablaufs, insbesondere im Hinblick auf die Minimierung von Nebenprodukten, ist es vorteilhaft, wenn das 3-Hydroxybutanoat der allgemeinen Formel (I) und die Carbonsäure (II) in einem Molverhältnis von 3-Hydroxybutanoat der allgemeinen Formel (I) / Carbonsäure (II) in einem Bereich von 1 :1 bis 10 : 1, insbesondere in einem Bereich von 2 : 1 bis 8 : 1, vorzugsweise in einem Bereich von 3 : 1 bis 6 : 1, eingesetzt werden.

Typischerweise wird im Rahmen des erfindungsgemäßen Verfahrens bei der Umsetzung von 3-Hydroxybutanoat der allgemeinen Formel (I) mit einer Carbonsäure (II) in Form der freien Säure gleichzeitig Wasser gebildet. Insbesondere ist es bevorzugt, wenn das Wasser der Umsetzung entzogen wird, insbesondere mittels vorzugsweise kontinuierlicher, insbesondere destillativer oder adsorptiver Entfernung.

Üblicherweise wird im Rahmen des erfindungsgemäßen Verfahrens bei der Umsetzung von 3-Hydroxybutanoat der allgemeinen Formel (I) mit einer Carbonsäure (II) in Form des Anhydrids pro Mol des eingesetzten Anhydrids ein Mol der entsprechenden freien Carbonsäure (II) gebildet. Insbesondere wird die entstehende freie Carbonsäure (II) mit 3-Hydroxybutanoat der allgemeinen Formel (I) umgesetzt oder nach erfolgter Reaktion entfernt und gegebenenfalls rezykliert, insbesondere in Abhängigkeit von den Mengen und/oder Mengenverhältnissen der eingesetzten Ausgangsverbindungen (I) und (II).

Bei der Verwendung von inneren bzw. cyclischen Anhydriden (wie beispielsweise Bernsteinsäureanhydrid oder Maleinsäureanhydrid) jedoch wird der Ring geöffnet und kein Abspaltprodukt gebildet, so dass das Reaktionsprodukt eine endständige freie Säure aufweist. Diese endständige freie Säure kann dann gegebenenfalls wieder mit einem 3-Hydroxybutanoat der allgemeinen Formel (I) reagieren. Dieser Verlauf ist im Folgenden am Beispiel der Reaktion von Maleinsäureanhydrid mit 3-Hydroxybuttersäureethylester dargestellt:

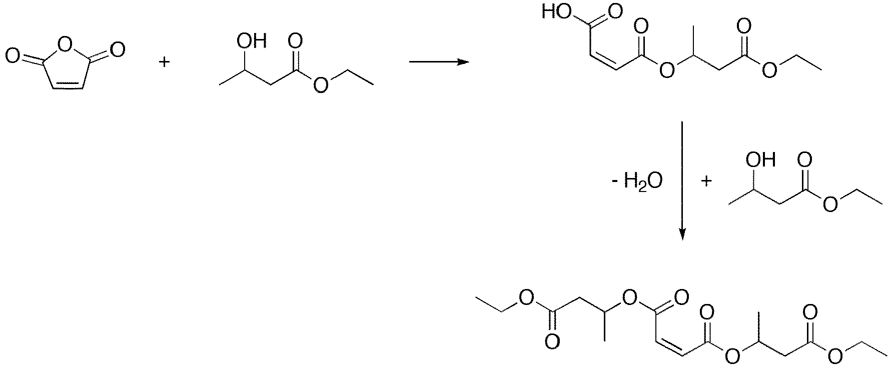

Im Rahmen des erfindungsgemäßen Herstellungsverfahren kann die Zusammensetzung des Reaktionsprodukts, insbesondere das Vorhandensein verschiedener Carbonsäureester von 3-Hydroxybutanoat (III) und deren Anteil im Fall eines Gemischs, mittels der Umsetzungsbedingungen kontrolliert und/oder gesteuert werden, insbesondere durch Auswahl der Umsetzungstemperatur (Reaktionstemperatur) und/oder Auswahl des Umsetzungsdrucks (Reaktionsdrucks) und/oder Abwesenheit oder Vorsehen eines Katalysators und dessen Auswahl in Bezug auf Art und/oder Menge und/oder Auswahl der Mengen der Ausgangsverbindungen (Edukte) und/oder Vorsehen der Entfernung der gegebenenfalls gebildeten Nebenprodukte, insbesondere Wasser.

Somit ist es möglich die Zusammensetzung des Produkts bzw. Produktgemischs je nach Anwendung maßzuschneidern. Insbesondere kann beispielsweise die Anzahl der substituierten 3-Hydroxybuttersäure-Resten (3-Hydroxybutanoaten) eingestellt werden, so dass die Dichte an Ketokörpern in Form von 3-Hydroxybutanoaten pro Molekül zielgerichtet eingestellt werden kann.

Im Anschluss an die Umsetzung kann das erhaltene Reaktionsprodukt weiteren üblichen bzw. an sich bekannten Aufreinigungs- bzw. Aufarbeitungsschritten unterzogen werden.

In diesem Zusammenhang kann das erhaltene Reaktionsprodukt nach erfolgter Umsetzung fraktioniert werden, insbesondere destillativ fraktioniert werden.

Auch können nichtumgesetzte Ausgangsverbindungen (I) und/oder (II) aus dem Reaktionsprodukt abgetrennt und anschließend rezykliert werden.

Gemäß einer besonderen Ausführungsform des erfindungsgemäßen Herstellungsverfahrens kann insbesondere derart vorgegangen werden, dass im Reaktionsprodukt nach erfolgter Umsetzung noch vorhandene Hydroxylgruppen und/oder Carboxylgruppen zumindest teilweise, vorzugsweise vollständig, funktionalisiert, insbesondere verestert werden.

Mit anderen Worten kann sich der Umsetzung eine teilweise, insbesondere vollständige Funktionalisierung, insbesondere Veresterung, noch vorhandener Hydroxylgruppen und/oder Carboxylgruppen anschließen.

Bei dieser besonderen Ausführungsform des erfindungsgemäßen Verfahrens kann die Funktionalisierung, insbesondere die Veresterung noch vorhandener Hydroxylgruppen und/oder Carboxylgruppen durch Reaktion mit einem Carbonsäureanhydrid von beispielsweise C₂-C₃₀-Carbonsäuren oder C₂-C₃₀-Fettsäuren im Fall freier Hydroxylgruppen oder C₂-C₃₀-Fettalkoholen im Fall freier Carboxylgruppen erfolgen. Dabei kann es sich um lineare oder verzweigte, gesättigte oder ein- oder mehrfach ungesättigte C₂-C₃₀-Carbonsäureanhydride oder C₂-C₃₀-Fettsäuren oder C₂-C₃₀-Fettalkohole handeln. In diesem Zusammenhang können noch vorhandene Hydroxylgruppen insbesondere mit Carbonsäureanhydriden oder Fettsäuren umgesetzt werden und noch vorhandene Carboxylgruppen können insbesondere mit Fettalkoholen umgesetzt werden.

Gemäß einer besonderen Ausführungsform des erfindungsgemäßen Verfahrens können als Reaktionsprodukt (III) ein oder mehrere Carbonsäureester von 3-Hydroxybutanoat, insbesondere ein oder mehrere Carbonsäureester von 4-Oxo-4-(C₁-C₅-alkoxy)-2-butanol oder von 4-Oxo-4-(hydroxy-C₃-C₅-alkoxy)-2-butanol, der allgemeinen Formel (IIIb)

CH₃-CH(OR⁴)-CH₂-C(O)OR¹ (IIIb)

erhalten werden und/oder erhältlich sein, wobei in der allgemeinen Formel (IIIb)
- der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
- der Rest R⁴ abgeleitet ist von einer Carbonsäure, ausgewählt aus der Gruppe von Bernsteinsäure, Weinsäure, Milchsäure, Citronensäure, Äpfelsäure, Adipinsäure, Fumarsäure und Maleinsäure sowie deren Kombinationen oder Mischungen, insbesondere ausgewählt aus der Gruppe von Bernsteinsäure, Weinsäure, Milchsäure, Citronensäure, Äpfelsäure, Adipinsäure und Fumarsäure sowie deren Kombinationen oder Mischungen.

Insbesondere können im Fall der Bernsteinsäure, Weinsäure, Citronensäure, Äpfelsäure, Adipinsäure, Fumarsäure und Maleinsäure vorhandene weitere Carboxylgruppen vollständig oder teilweise mit einem Rest -CH(CH₃)-CH₂-C(O)OR¹ mit R¹ wie zuvor definiert verestert sein.

In diesem Zusammenhang bedeutet "abgeleitet von", dass der Rest R⁴ aus den genannten Carbonsäuren gebildet wird; insbesondere wird durch Veresterung der Wasserstoff des Carboxyls verestert; d. h. also, es liegt jeweils der Carboxylatrest der entsprechenden Säure als Rest R⁴ vor (d. h. also der Rest R⁴ ist ein Succinat-Rest im Fall der Bernsteinsäure, ein Tartrat-Rest im Fall der Weinsäure, ein Lactat-Rest im Fall der Milchsäure, ein Citrat-Rest im Fall der Citronensäure, ein Malat-Rest im Fall der Äpfelsäure, ein Adipat-Rest im Fall der Adipinsäure, ein Fumarat-Rest im Fall der Fumarsäure und ein Maleat-Rest im Fall der Maleinsäure).

Gemäß einer weiteren besonderen Ausführungsform des erfindungsgemäßen Verfahren können als Reaktionsprodukt (III) ein oder mehrere Carbonsäureester von 3-Hydroxybutanoat, insbesondere ein oder mehrere Carbonsäureester von 4-Oxo-4-(C₁-C₅-alkoxy)-2-butanol oder von 4-Oxo-4-(hydroxy-C₃-C₅-alkoxy)-2-butanol, der allgemeinen Formel (IIIb)

CH₃-CH(OR⁴)-CH₂-C(O)OR¹ (IIIb)

erhalten werden und/oder erhältlich sein, wobei in der allgemeinen Formel (IIIb)
- der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
- der Rest R⁴ einen oder mehrere der folgenden Reste darstellt 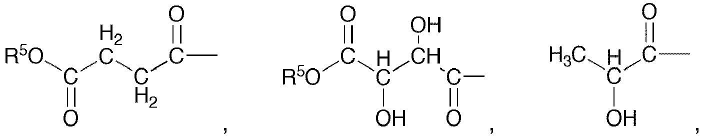 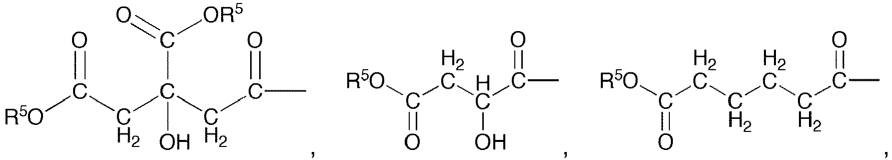 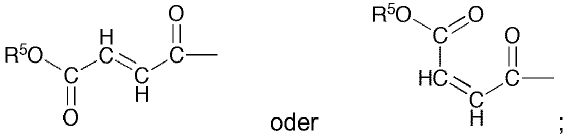 wobei in den vorstehenden Resten der Rest R⁵ Wasserstoff oder einen Rest - CH(CH₃)-CH₂- C(O)OR¹ mit R¹ wie zuvor definiert darstellt.

Gemäß einer wiederum weiteren besonderen Ausführungsform des erfindungsgemäßen Verfahrens kann als Reaktionsprodukt ein Gemisch von mindestens zwei voneinander verschiedenen Carbonsäureestern von 3-Hydroxybutanoat (III), insbesondere Carbonsäureestern von 4-Oxo-4-(C₁-C₅-alkoxy)-2-butanol oder von 4-Oxo-4-(hydroxy-C₃-C₅-alkoxy)-2-butanol, insbesondere wie zuvor definiert, erhalten werden.

Weiterer Gegenstand - gemäß einem **zweiten** Aspekt der vorliegenden Erfindung - ist ein Carbonsäureester von 3-Hydroxybutanoat, wobei der Carbonsäureester von 3-Hydroxybutanoat der allgemeinen Formel (IIIb)

CH₃-CH(OR⁴)-CH₂- C(O)OR¹ (IIIb)

entspricht, wobei in der allgemeinen Formel (IIIb)
- der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
- der Rest R⁴ abgeleitet ist von einer Carbonsäure, ausgewählt aus der Gruppe von Weinsäure, Milchsäure, Citronensäure, Äpfelsäure und Fumarsäure sowie deren Kombinationen oder Mischungen;
insbesondere wobei im Fall der Weinsäure, Citronensäure, Äpfelsäure, und Fumarsäure vorhandene weitere Carboxylgruppen vollständig oder teilweise mit einem Rest - CH(CH₃)-CH₂- C(O)OR¹ mit R¹ wie zuvor definiert verestert sind.

Gemäß dem zuvor beschriebenen Verfahren ist somit ein (chemisches) Produkt oder Produktgemisch, vorzugsweise ein Carbonsäureester von 3-Hydroxybutanoat, insbesondere Carbonsäureester von 4-Oxo-4-(C₁-C₅-alkoxy)-2-butanol oder von 4-Oxo-4-(hydroxy-C₃-C₅-alkoxy)-2-butanol, oder ein Gemisch von mehreren Carbonsäureestern von 3-Hydroxybutanoat, insbesondere ein Gemisch von mehreren Carbonsäureestern von 4-Oxo-4-(C₁-C₅-alkoxy)-2-butanol oder von 4-Oxo-4-(hydroxy-C₃-C₅-alkoxy)-2butanol, erhältlich.

Gemäß einer wiederum weiteren besonderen Ausführungsform kann das Reaktionsprodukt (III) ein oder mehrere Carbonsäureester von 3-Hydroxybutanoat, insbesondere ein oder mehrere Carbonsäureester von 4-Oxo-4-(C₁-C₅-alkoxy)-2-butanol oder von 4-Oxo-4-(hydroxy-C₃-C₅-alkoxy)-2-butanol, der allgemeinen Formel (IIIb)

CH₃-CH(OR⁴)-CH₂-C(O)OR¹ (IIIb)

umfassen, wobei in der allgemeinen Formel (IIIb)
- der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
- der Rest R⁴ abgeleitet ist von einer Carbonsäure, ausgewählt aus der Gruppe von Bernsteinsäure, Weinsäure, Milchsäure, Citronensäure, Äpfelsäure, Adipinsäure, Fumarsäure und Maleinsäure sowie deren Kombinationen oder Mischungen, insbesondere ausgewählt aus der Gruppe von Bernsteinsäure, Weinsäure, Milchsäure, Citronensäure, Äpfelsäure, Adipinsäure und Fumarsäure sowie deren Kombinationen oder Mischungen.

Insbesondere können im Fall der Bernsteinsäure, Weinsäure, Citronensäure, Äpfelsäure, Adipinsäure, Fumarsäure und Maleinsäure vorhandene weitere Carboxylgruppen vollständig oder teilweise mit einem Rest - CH(CH₃)-CH₂-C(O)OR¹ mit R¹ wie zuvor definiert verestert sein.

Wie zuvor ausgeführt, bedeutet in diesem Zusammenhang "abgeleitet von", dass der Rest R⁴ aus den genannten Carbonsäuren gebildet wird, insbesondere wird durch Veresterung der Wasserstoff des Carboxyls verestert; d.h. also es liegt jeweils der Carboxylatrest der entsprechenden Säure als Rest R⁴ vor (d. h. also der Rest R⁴ ist ein Succinat-Rest im Fall der Bernsteinsäure, ein Tartrat-Rest im Fall der Weinsäure, ein Lactat-Rest im Fall der Milchsäure, ein Citrat-Rest im Fall der Citronensäure, ein Malat-Rest im Fall der Äpfelsäure, ein Adipat-Rest im Fall der Adipinsäure, ein Fumarat-Rest im Fall der Fumarsäure und ein Maleat-Rest im Fall der Maleinsäure).

Gemäß einer weiteren besonderen Ausführungsform kann das Reaktionsprodukt (III) ein oder mehrere Carbonsäureester von 3-Hydroxybutanoat, insbesondere ein oder mehrere Carbonsäureester von 4-Oxo-4-(C₁-C₅-alkoxy)-2-butanol oder von 4-Oxo-4-(hydroxy-C₃-C₅-alkoxy)-2-butanol, der allgemeinen Formel (IIIb)

CH₃-CH(OR⁴)-CH₂-C(O)OR¹ (IIIb)

umfassen, wobei in der allgemeinen Formel (IIIb)
- der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
- der Rest R⁴ einen oder mehrere der folgenden Reste darstellt 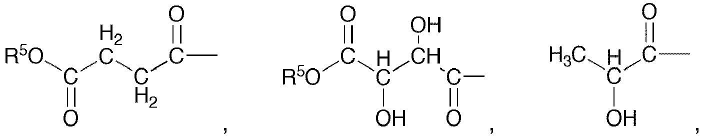 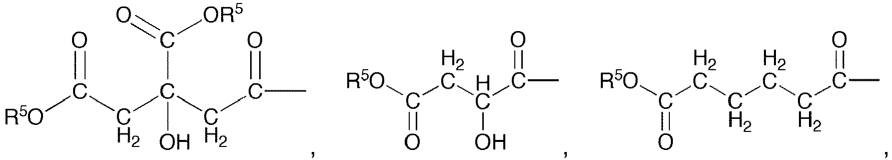 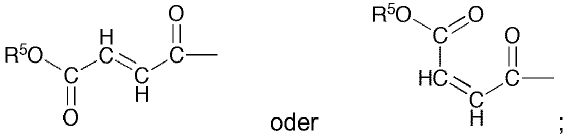 wobei in den vorstehenden Resten der Rest R⁵ Wasserstoff oder einen Rest - CH(CH₃)-CH₂-C(O)OR¹ mit R¹ wie zuvor definiert darstellt.

Gemäß einer besonderen Ausführungsform kann das Reaktionsprodukt ein Gemisch von mindestens zwei voneinander verschiedenen Carbonsäureestern von 3-Hydroxybutanoat (III), insbesondere Carbonsäureestern von 4-Oxo-4-(C₁-C₅-alkoxy)-2-butanol oder von 4-Oxo-4-(hydroxy-C₃-C₅-alkoxy)-2-butanol, insbesondere wie zuvor definiert, umfassen.

Gegenstand der vorliegenden Erfindung ist somit auch ein Carbonsäureester von 3-Hydroxybutanoat, wobei der Carbonsäureester von 3-Hydroxybutanoat der allgemeinen Formel (IIIb)

CH₃-CH(OR⁴)-CH₂-C(O)OR¹ (IIIb)

entspricht, wobei in der allgemeinen Formel (IIIb)
- der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
- der Rest R⁴ abgeleitet ist von einer Carbonsäure, ausgewählt aus der Gruppe von Weinsäure, Milchsäure, Citronensäure, Äpfelsäure und Fumarsäure sowie deren Kombinationen oder Mischungen;
insbesondere wobei im Fall der Weinsäure, Citronensäure, Äpfelsäure, und Fumarsäure vorhandene weitere Carboxylgruppen vollständig oder teilweise mit einem Rest - CH(CH₃)-CH₂-C(O)OR¹ mit R¹ wie zuvor definiert verestert sind.

Wie zuvor ausgeführt bedeutet "abgeleitet von", dass der Rest R⁴ aus den genannten Carbonsäuren gebildet wird, insbesondere wird durch Veresterung der Wasserstoff des Carboxyls verestert; d.h. also es liegt jeweils der Carboxylatrest der entsprechenden Säure als Rest R⁴ vor (d. h. also der Rest R⁴ ist ein Succinat-Rest im Fall der Bernsteinsäure, ein Tartrat-Rest im Fall der Weinsäure, ein Lactat-Rest im Fall der Milchsäure, ein Citrat-Rest im Fall der Citronensäure, ein Malat-Rest im Fall der Äpfelsäure, ein Adipat-Rest im Fall der Adipinsäure, ein Fumarat-Rest im Fall der Fumarsäure und ein Maleat-Rest im Fall der Maleinsäure).

Gemäß einer bevorzugten Ausführungsform der vorliegenden Erfindung kann der Carbonsäureester von 3-Hydroxybutanoat, insbesondere Carbonsäureester von 4-Oxo-4-(C₁-C₅-alkoxy)-2-butanol oder von 4-Oxo-4-(hydroxy-C₃-C₅-alkoxy)-2-butanol, der allgemeinen Formel (IIIb)

CH₃-CH(OR⁴)-CH₂-C(O)OR¹ (IIIb)

entsprechen, wobei in der allgemeinen Formel (IIIb)
- der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
- der Rest R⁴ einen oder mehrere der folgenden Reste darstellt 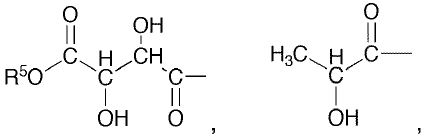 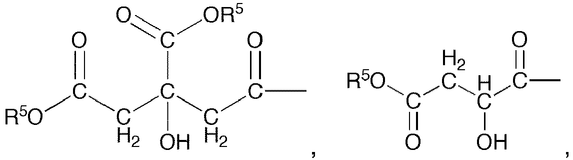 oder 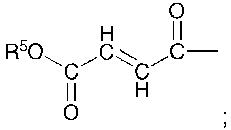 wobei in den vorstehenden Resten der Rest R⁵ Wasserstoff oder einen Rest -CH(CH₃)- CH₂ - C(O)OR¹ mit R¹ wie zuvor definiert darstellt.

Darüber hinaus ist weiterer Gegenstand der vorliegenden Erfindung gemäß einer besonderen Ausführungsform ein Gemisch, umfassend mindestens zwei voneinander verschiedene Carbonsäureester von 3-Hydroxybutanoat (III), insbesondere Carbonsäureester von 4-Oxo-4-(C₁-C₅-alkoxy)-2-butanol oder von 4-Oxo-4-(hydroxy-C₃-C₅-alkoxy)-2-butanol, insbesondere wie zuvor definiert.

Das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Carbonsäureester von 3-Hydroxybutanoat, insbesondere Carbonsäureester von 4-Oxo-4-(C₁-C₅-alkoxy)-2-butanol oder von 4-Oxo-4-(hydroxy-C₃-C₅-alkoxy)-2-butanol, wie zuvor definiert, und/oder das nach dem erfindungsgemäßen Herstellungsverfahren erhältliche bzw. erfindungsgemäße Gemisch, wie zuvor definiert, weist gegenüber dem Stand der Technik eine Vielzahl von Vorteilen und Besonderheiten auf:
Wie die Anmelderin überraschend herausgefunden hat, eignet sich das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Carbonsäureester von 3-Hydroxybutanoat, insbesondere Carbonsäureester von 4-Oxo-4-(C₁-C₅-alkoxy)-2-butanol oder von 4-Oxo-4-(hydroxy-C₃-C₅-alkoxy)-2-butanol, wie zuvor definiert, und/oder das nach dem erfindungsgemäßen Herstellungsverfahren erhältliche bzw. erfindungsgemäße Gemisch, wie zuvor definiert, insbesondere als Präkursor bzw. Metabolit von 3-Hydroxybuttersäure bzw. deren Salzen, da dieser bzw. dieses einerseits physiologisch, insbesondere im Magen/Darm-Trakt, zu 3-Hydroxybuttersäure bzw. deren Salzen umgesetzt wird und andererseits gleichzeitig eine gute physiologische Kompatibilität bzw. Verträglichkeit aufweist, insbesondere im Hinblick auf Nichttoxizität und akzeptable organoleptische Eigenschaften.

Darüber hinaus ist das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Carbonsäureester von 3-Hydroxybutanoat, insbesondere Carbonsäureester von 4-Oxo-4-(C₁-C₅-alkoxy)-2-butanol oder von 4-Oxo-4-(hydroxy-C₃-C₅-alkoxy)-2-butanol, wie zuvor definiert, und/oder das nach dem erfindungsgemäßen Herstellungsverfahren erhältliche bzw. erfindungsgemäße Gemisch, wie zuvor definiert, ohne Weiteres auf synthetischem Wege auch in großtechnischem Maßstab zugänglich bzw. verfügbar, und zwar auch mit der erforderlichen pharmazeutischen bzw. pharmakologischen Qualität.

Zudem kann das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Carbonsäureester von 3-Hydroxybutanoat, insbesondere Carbonsäureester von 4-Oxo-4-(C₁-C₅-alkoxy)-2-butanol oder von 4-Oxo-4-(hydroxy-C₃-C₅-alkoxy)-2-butanol, wie zuvor definiert, und/oder das nach dem erfindungsgemäßen Herstellungsverfahren erhältliche bzw. erfindungsgemäße Gemisch, wie zuvor definiert, erforderlichenfalls in enantiomerenreiner bzw. enantiomerenangereicherter Form bereitgestellt werden.

Das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Carbonsäureester von 3-Hydroxybutanoat, insbesondere Carbonsäureester von 4-Oxo-4-(C₁-C₅-alkoxy)-2-butanol oder von 4-Oxo-4-(hydroxy-C₃-C₅-alkoxy)-2-butanol, wie zuvor definiert, und/oder das nach dem erfindungsgemäßen Herstellungsverfahren erhältliche bzw. erfindungsgemäße Gemisch, wie zuvor definiert, stellt somit ein effizientes pharmakologisches Wirkstoff-Target im Rahmen einer Ketokörper-Therapie des menschlichen oder tierischen Körpers dar.

Nachfolgend werden noch die übrigen Erfindungsaspekte weiterführende erläutert und im Detail beschrieben.

Weiterer Gegenstand der vorliegenden Erfindung - gemäß einem **dritten** Aspekt der vorliegenden Erfindung - ist eine pharmazeutische Zusammensetzung, insbesondere ein Arzneimittel oder Medikament, umfassend einen Carbonsäureester von 3-Hydroxybutanoat (III) oder ein Gemisch von mindestens zwei voneinander verschiedenen Carbonsäureestern von 3-Hydroxybutanoat (III), wobei der Carbonsäureester von 3-Hydroxybutanoat (III) der allgemeinen Formel (IIIb)

CH₃-CH(OR⁴)-CH₂-C(O)OR¹ (IIIb)

entspricht, wobei in der allgemeinen Formel (IIIb)
- der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
- der Rest R⁴ einen oder mehrere der folgenden Reste darstellt 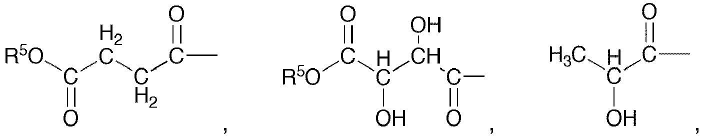 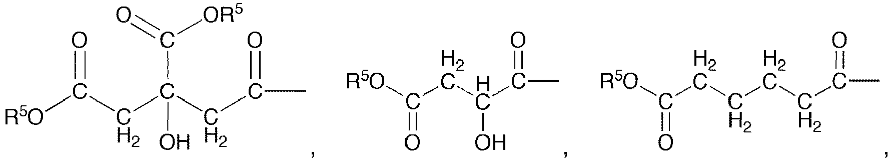 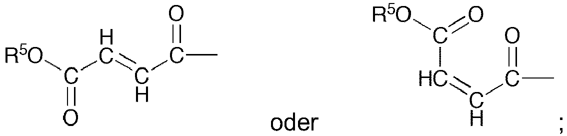 wobei in den vorstehenden Resten der Rest R⁵ Wasserstoff oder einen Rest -CH(CH₃)-CH₂-C(O)OR¹ mit R¹ wie zuvor definiert darstellt.

Insbesondere betrifft die vorliegende Erfindung gemäß diesem Erfindungsaspekt eine pharmazeutische Zusammensetzung zur prophylaktischen und/oder therapeutischen Behandlung bzw. zur Verwendung bei der prophylaktischen und/oder therapeutischen Behandlung von Erkrankungen des menschlichen oder tierischen Körpers. Hierbei kann es sich insbesondere um Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, handeln.

Wiederum weiterer Gegenstand der vorliegenden Erfindung - gemäß einem **vierten** Aspekt der vorliegenden Erfindung - ist ein Carbonsäureester von 3-Hydroxybutanoat (III) oder Gemisch von mindestens zwei voneinander verschiedenen Carbonsäureestern von 3-Hydroxybutanoat (III),
wobei der Carbonsäureester von 3-Hydroxybutanoat (III) der allgemeinen Formel (IIIb)

CH₃-CH(OR⁴)-CH₂-C(O)OR¹ (IIIb)

entspricht, wobei in der allgemeinen Formel (IIIb)
- der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
- der Rest R⁴ einen oder mehrere der folgenden Reste darstellt 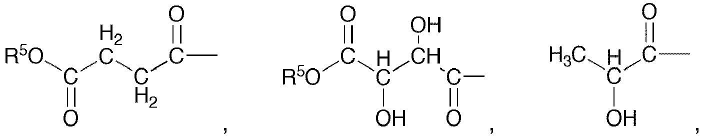 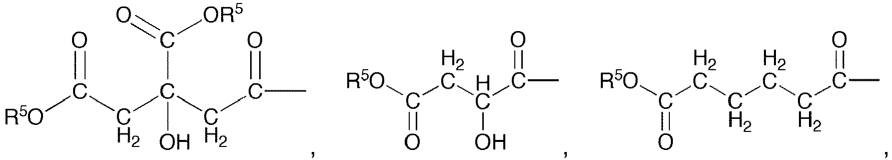 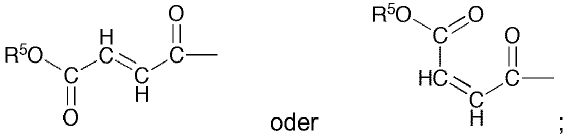 wobei in den vorstehenden Resten der Rest R⁵ Wasserstoff oder einen Rest -CH(CH₃)-CH₂-C(O)OR¹ mit R¹ wie zuvor definiert darstellt, zur prophylaktischen und/oder therapeutischen Behandlung bzw. zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Gleichermaßen weiterer Gegenstand der vorliegenden Erfindung - gemäß einem **fünften** Aspekt der vorliegenden Erfindung - ist die Verwendung eines Carbonsäureesters von 3-Hydroxybutanoat (III) oder Gemischs von mindestens zwei voneinander verschiedenen Carbonsäureestern von 3-Hydroxybutanoat (III), wobei der Carbonsäureester von 3-Hydroxybutanoat (III) der allgemeinen Formel (IIIb)

CH₃-CH(OR⁴)-CH₂-C(O)OR¹ (IIIb)

entspricht, wobei in der allgemeinen Formel (IIIb)
- der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
- der Rest R⁴ einen oder mehrere der folgenden Reste darstellt 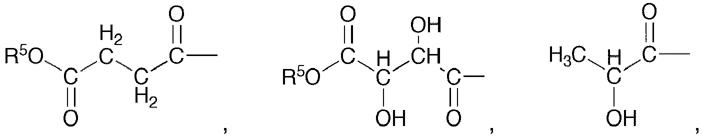 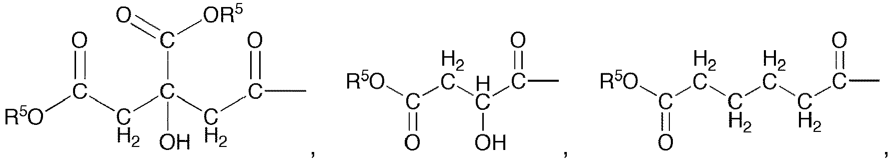 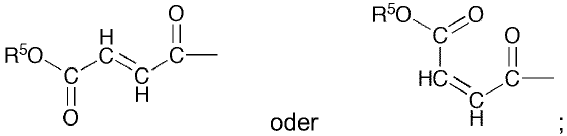 wobei in den vorstehenden Resten der Rest R⁵ Wasserstoff oder einen Rest -CH(CH₃)-CH₂-C(O)OR¹ mit R¹ wie zuvor definiert darstellt, zur prophylaktischen und/oder therapeutischen Behandlung bzw. zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Gleichermaßen weiterer Gegenstand der vorliegenden Erfindung - gemäß einem **sechsten** Aspekt der vorliegenden Erfindung - ist die Verwendung eines Carbonsäureesters von 3-Hydroxybutanoat (III) oder Gemischs von mindestens zwei voneinander verschiedenen Carbonsäureestern von 3-Hydroxybutanoat (III), wobei der Carbonsäureester von 3-Hydroxybutanoat (III) der allgemeinen Formel (IIIb)

CH₃-CH(OR⁴)-CH₂-C(O)OR¹ (IIIb)

entspricht, wobei in der allgemeinen Formel (IIIb)
- der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
- der Rest R⁴ einen oder mehrere der folgenden Reste darstellt 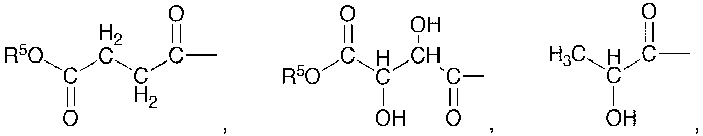 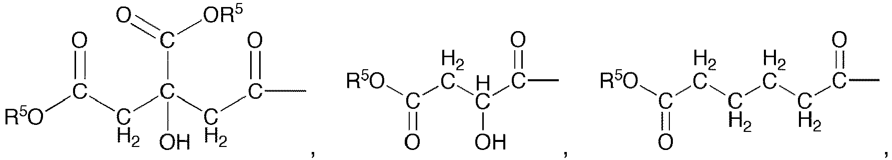 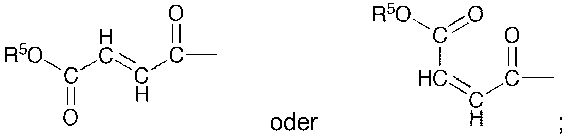 wobei in den vorstehenden Resten der Rest R⁵ Wasserstoff oder einen Rest -CH(CH₃)-CH₂-C(O)OR¹ mit R¹ wie zuvor definiert darstellt, zur prophylaktischen und/oder therapeutischen Behandlung bzw. zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung.

Gleichermaßen weiterer Gegenstand der vorliegenden Erfindung - gemäß einem **siebten** Aspekt der vorliegenden Erfindung - ist ein Nahrungsmittel- und/oder Lebensmittelerzeugnis, welches einen Carbonsäureester von 3-Hydroxybutanoat (III) oder Gemisch von mindestens zwei voneinander verschiedenen Carbonsäureestern von 3-Hydroxybutanoat (III),
wobei der Carbonsäureester von 3-Hydroxybutanoat (III) der allgemeinen Formel (IIIb)

CH₃-CH(OR⁴)-CH₂-C(O)OR¹ (IIIb)

entspricht, wobei in der allgemeinen Formel (IIIb)
- der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
- der Rest R⁴ einen oder mehrere der folgenden Reste darstellt 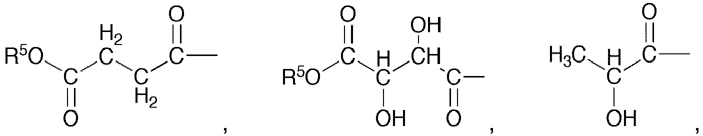 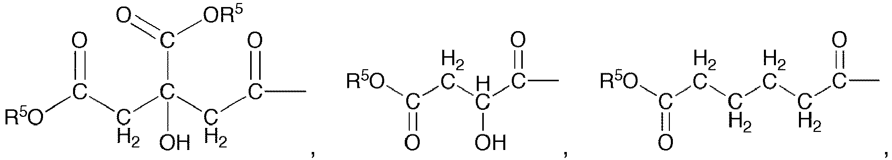 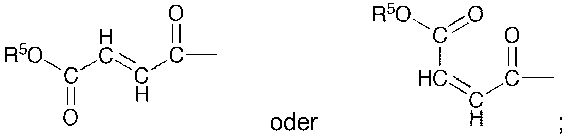 wobei in den vorstehenden Resten der Rest R⁵ Wasserstoff oder einen Rest -CH(CH₃)-CH₂-C(O)OR¹ mit R¹ wie zuvor definiert darstellt, umfasst.

Gemäß einer besonderen Ausführungsform kann das Nahrungsmittel- und/oder Lebensmittelerzeugnis insbesondere ein Nahrungsergänzungsmittel, ein funktionelles Lebensmittel *(Functional Food),* ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Auch kann die Verwendung eines nach dem erfindungsgemäßen Herstellungsverfahren erhältlichen Reaktionsprodukts, wie zuvor definiert, und/oder eines nach dem erfindungsgemäßen Herstellungsverfahren erhältlichen bzw. erfindungsgemäßen Carbonsäureesters von 3-Hydroxybutanoat, insbesondere Carbonsäureesters von 4-Oxo-4-(C₁-C₅-alkoxy)-2-butanol oder von 4-Oxo-4-(hydroxy-C₃-C₅-alkoxy)-2-butanol, wie zuvor definiert, und/oder eines nach dem erfindungsgemäßen Herstellungsverfahren erhältlichen Gemischs, wie zuvor definiert, in einem Nahrungsmittel- und/oder Lebensmittelerzeugnis, vorgesehen sein.

Dabei kann das Nahrungsmittel- und/oder Lebensmittelerzeugnis insbesondere ein Nahrungsergänzungsmittel, ein funktionelles Lebensmittel *(Functional Food),* ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Weitere Ausgestaltungen, Abwandlungen und Variationen der vorliegenden Erfindung sind für den Fachmann beim Lesen der Beschreibung ohne Weiteres erkennbar oder realisierbar, ohne dass er dabei den Rahmen der vorliegenden Erfindung verlässt.

Die vorliegende Erfindung wird anhand der nachfolgenden Ausführungsbeispiele veranschaulicht, welche die vorliegende Erfindung jedoch keinesfalls beschränken sollen, sondern lediglich die beispielhafte und nichtlimitierende Durchführungsweise der Ausgestaltung der vorliegenden Erfindung erläutern sollen. Die Beispiele werden weiterführend anhand der nachfolgenden Beschreibung der Zeichnungen und den Zeichnungen selbst charakterisiert. Dabei bilden alle beschriebenen und/oder bildlich dargestellten Merkmale für sich oder in beliebiger Kombination den Gegenstand der vorliegenden Erfindung, unabhängig von ihrer Zusammenfassung in den Ansprüchen und deren Rückbeziehungen.

Es zeigt:
- Fig. 1: eine Darstellung des Umsatz-Zeit-Verlaufs der Umsetzung von Bernsteinsäureanhydrid mit Ethyl-3-hydroxybutanoat,
- Fig. 2: eine Darstellung des Umsatz-Zeit-Verlaufs der Umsetzung von Citronensäure mit Ethyl-3-hydroxybutanoat,
- Fig. 3: eine Darstellung des Umsatz-Zeit-Verlaufs der Umsetzung von Äpfelsäure mit Ethyl-3-hydroxybutanoat,

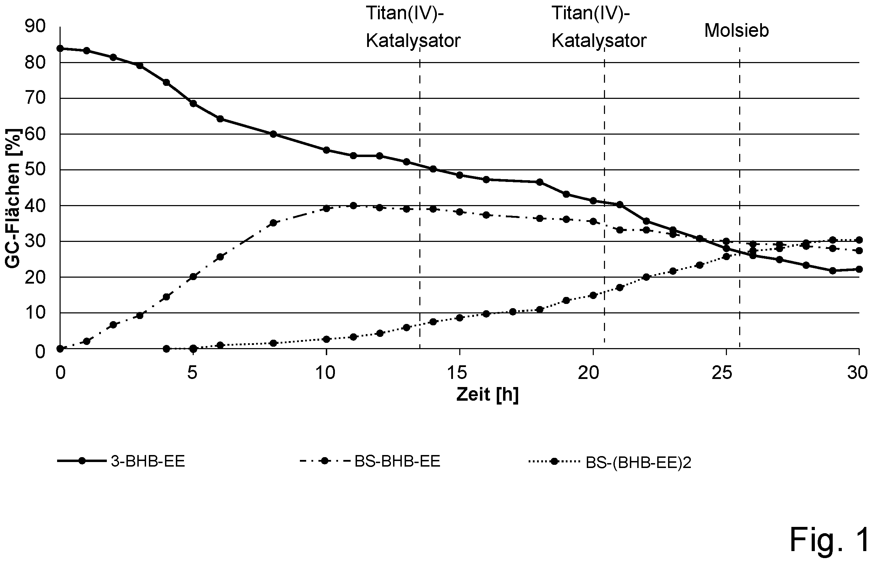

In Fig. 1 ist der Umsatz-Zeit-Verlauf der Umsetzung von Bernsteinsäureanhydid mit Ethyl-3-hydroxybutanoat gezeigt, wobei auf der X-Achse die Umsetzungszeit in Stunden [h] und auf der Y-Achse die GC-Flächenanalyse in Prozent [%] dargestellt ist. Aufgezeigt sind die Verläufe bzw. Mengen der Ausgangsverbindung Ethyl-3-hydroxybutanoat (3-BHB-EE) sowie der Reaktionsprodukte Bernsteinsäuremonoester von Ethyl-3-hydroxybutanoat (BS-BHB-EE) und Bernsteinsäurediester von Ethyl-3-hydroxybutanoat (BS-(BHB-EE)2). Weiterhin sind die Zeitpunkte markiert, zu welchen der Titan(IV)-Katalysator jeweils zugegeben wird und zu welchen das Molekularsieb (Molsieb) zur adsorptiven Entfernung des Wassers zugegeben wird.

Während der gesamten Reaktionszeit wird Ethyl-3-hydroxybutanoat umgesetzt. Nach 10 Stunden Reaktionszeit ist die Umsetzung des Bernsteinsäureanhydrids in der ersten Reaktionsstufe zum Bernsteinsäuremonoester von Ethyl-3-hydroxybutanoat bereits abgeschlossen. Die Zugabe des Katalysators führt zu keiner signifikanten Umsatzsteigerung. Die erneute Zugabe von Katalysator führt zu einer gesteigerten Bildung des Bernsteinsäurediesters von Ethyl-3-hydroxybutanoat, wobei sich die gebildete Menge an Bernsteinsäuremonoester von Ethyl-3-hydroxybutanoat aufgrund der weiteren Umsetzung zum Bernsteinsäurediester von Ethyl-3hydroxybutanoat geringfügig verringert. Die Zugabe des Molekularsiebs führt zu keiner signifikanten Umsatzsteigerung. Nach 30 Stunden Reaktionszeit liegen in etwa gleiche Mengen an Bernsteinsäuremonoester und Bernsteinsäurediester von Ethyl-3-hydroxybutanoat vor.

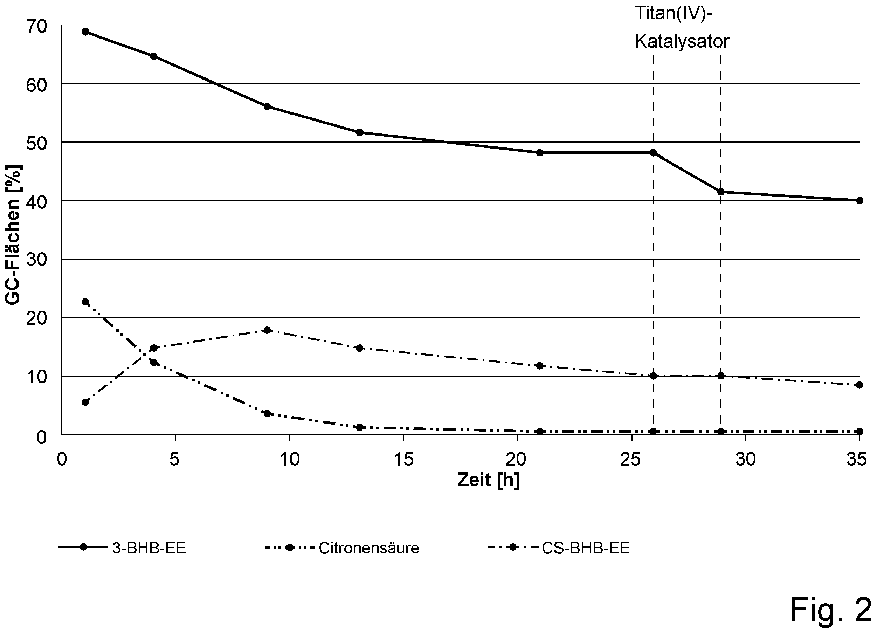

In Fig. 2 ist der Umsatz-Zeit-Verlauf der Umsetzung von Citronensäure mit Ethyl-3-hydroxybutanoat gezeigt, wobei auf der X-Achse die Umsetzungszeit in Stunden [h] und auf der Y-Achse die GC-Flächenanalyse in Prozent [%] dargestellt ist. Aufgezeigt sind die Verläufe bzw. Mengen der Ausgangsverbindung Ethyl-3-hydroxybutanoat (3-BHB-EE) und Citronensäure sowie der Reaktionsprodukte Citronensäuremonoester von Ethyl-3-hydroxybutanoat (CS-BHB-EE), Citronensäurediester von Ethyl-3-hydroxybutanoat (CS-(BHB-EE)2) und Citronensäuretriester von Ethyl-3-hydroxybutanoat (CS-(BHB-EE)3). Weiterhin sind die Zeitpunkte markiert, zu welchen der Titan(IV)-Katalysator jeweils zugegeben wird.

Während der gesamten Reaktionszeit wird das Ethyl-3-hydroxybutanoat umgesetzt. Zu Beginn der Reaktion werden maßgeblich der Citronensäuremonoester und der Citronensäurediester von Ethyl-3-hydroxybutanoat gebildet. Nach einer Reaktionszeit von ungefähr 13 Stunden sinkt die Menge an Citronensäuremonoester von Ethyl-3-hydroxybutanoat wieder, da mehr Citronensäuremonoester von Ethyl-3-hydroxybutanoat zum Citronensäurediester von Ethyl-3-hydroxybutanoat umgesetzt wird als neuer Citronensäuremonoester von Ethyl-3-hydroxybutanoat gebildet wird. Nach einer Reaktionszeit von etwa 21 Stunden ist die Citronensäure vollständig umgesetzt, sodass ab diesem Zeitpunkt kein neuer Citronensäuremonoester von Ethyl-3-hydroxybutanoat mehr gebildet wird. Mit der ersten Zugabe des Titan(IV)-Katalysators wird eine geringe Umsatzsteigerung in Bezug auf den Citronensäuremonoester und den Citronensäurediester von Ethyl-3-hydroxybutanoat erzielt, jedoch führt die zweite Zugabe zu keiner signifikanten weiteren Umsatzsteigerung.

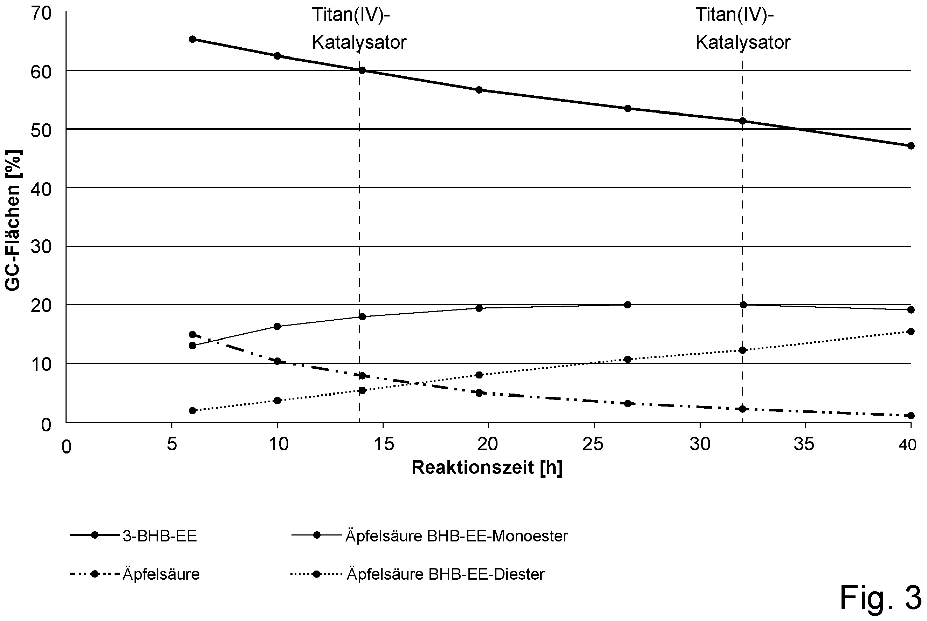

In Fig. 3 ist der Umsatz-Zeit-Verlauf der Umsetzung von Äpfelsäure mit Ethyl-3-hydroxybutanoat gezeigt, wobei auf der X-Achse die Umsetzungszeit in Stunden [h] und auf der Y-Achse die GC-Flächenanalyse in Prozent [%] dargestellt ist. Aufgezeigt sind die Verläufe bzw. Mengen der Ausgangsverbindung Ethyl-3-hydroxybutanoat (3-BHB-EE) und Äpfelsäure sowie der Reaktionsprodukte Äpfelsäuremonoester von Ethyl-3-hydroxybutanoat (Äpfelsäure BHB-EE-Monoester) und Äpfelsäurediester von Ethyl-3-hydroxybutanoat (Äpfelsäure BHB-EE-Diester). Weiterhin sind die Zeitpunkte markiert, zu welchen der Titan(IV)-Katalysator jeweils zugegeben wird.

Während der gesamten Reaktionszeit wird das Ethyl-3-hydroxybutanoat umgesetzt. Nach 25 Stunden Reaktionszeit ist die Umsetzung des Äpfelsäure in der ersten Reaktionsstufe zum Äpfelsäuremonoester von Ethyl-3-hydroxybutanoat bereits abgeschlossen. Die Zugaben des Katalysators führen jeweils zu keinen signifikanten Umsatzsteigerungen.

### AUSFÜHRUNGSBEISPIELE:

### Verwendete Abkürzungen

| | |
|---|---|
| • 3-BHB-EE = BHB-EE: | 3-Hydroxybuttersäureethylester (= Ethyl-3-hydroxybutanoat oder 4-Ethoxy-4-oxobutan-2-ol) |
| • 3-BHB = BHB: | 3-Hydroxybuttersäure |
| • BS: | Bernsteinsäure |
| • CS: | Citronensäure |

### I. Herstellungsbeispiele einer besonderen Ausführungsform des erfindungsgemäßen Verfahrens ohne die Verwendung eines Katalysators

### I. 1. Herstellung von Weinsäureestern von Ethyl-3-hydroxybutanoat

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch) und 38 g Weinsäure vorgelegt. Das Reaktionsgemisch wird unter Rühren bei 120 °C und unter N₂ für 7 h zur Reaktion gebracht und das entstehende Reaktionswasser wird kontinuierlich destillativ entfernt. Anschließend wird der überschüssige 3-Hydroxybuttersäureethylester unter Vakuum abdestilliert. Der erhaltene Rückstand wird für 2 bis 4 h im Hochvakuum dampfbehandelt.

Es wird ein Gemisch aus Weinsäuremonoester von Ethyl-3-hydroxybutanoat [= Mono-(4-ethoxy-4-oxo-butan-2-yl)-tartrat oder Weinsäuremono-(4-ethoxy-4-oxo-butan-2-yl)-ester] und Weinsäurediester von Ethyl-3-hydroxybutanoat [= Bis-(4-ethoxy-4-oxo-butan-2-yl)-tartrat oder Weinsäuredi-(4-ethoxy-4-oxo-butan-2-yl)-ester] erhalten.

Der Weinsäuremonoester von Ethyl-3-hydroxybutanoat kann synonym auch als 4-[(4-Ethoxy-4-oxobutan-2-yl)-oxy]-2,3-dihydroxy-4-oxobutansäure bezeichnet werden, während der Weinsäurediester von Ethyl-3-hydroxybutanoat synonym auch als 1,4-Bis-(4-ethoxy-4-oxobutan-2-yl)-2,3-dihydroxybutandioat) oder auch als Bis-(4-ethoxy-4-oxobutan-2-yl)-2,3-hydroxysuccinat bezeichnet werden kann.

Die Charakterisierung erfolgt mittels Massenspektrometrie (MS), Gelpermeationschromatographie (GPC) und Protonenresonanzspektroskopie (¹H-NMR).

Im Folgenden ist der Reaktionsverlauf schematisch dargestellt:

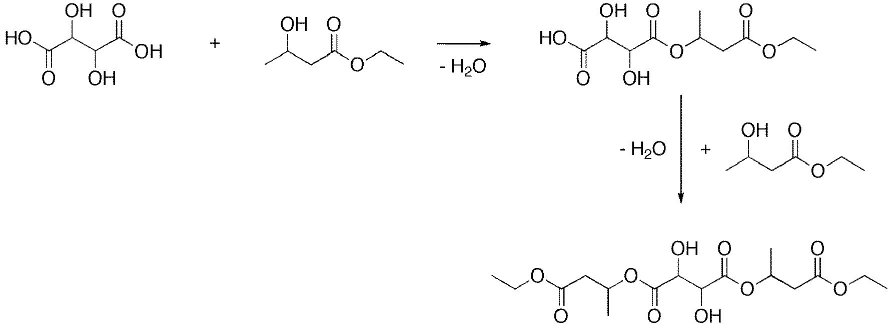

### I. 2. Herstellung von Milchsäureester von Ethyl-3-hydroxybutanoat

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch) und 57 g Milchsäure vorgelegt. Das Reaktionsgemisch wird unter Rühren bei 120 °C und unter N₂ für 7 h zur Reaktion gebracht und das entstehende Reaktionswasser wird kontinuierlich destillativ entfernt. Anschließend wird der überschüssige 3-Hydroxybuttersäureethylester unter Vakuum abdestilliert. Der erhaltene Rückstand wird für 2 bis 4 h im Hochvakuum dampfbehandelt.

Es wird der Milchsäuremonoester von Ethyl-3-hydroxybutanoat [= Mono-(4-ethoxy-4-oxo-butan-2-yl)-lactat oder Milchsäuremono-(4-ethoxy-4-oxo-butan-2-yl)-ester] erhalten. Die Charakterisierung erfolgt mittels MS, GPC und ¹H-NMR.

Im Folgenden ist der Reaktionsverlauf schematisch dargestellt:

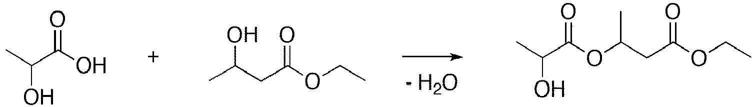

### I. 3. Herstellung von Bernsteinsäureestern von Ethyl-3-hydroxybutanoat

### Variante 1: Verwendung von Bernsteinsäureanhydrid

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch) und 24 g Bernsteinsäureanhydrid vorgelegt. Das Reaktionsgemisch wird unter Rühren bei 120 °C und unter N₂ für 7 h zur Reaktion gebracht und das entstehende Reaktionswasser wird kontinuierlich destillativ entfernt. Anschließend wird der überschüssige 3-Hydroxybuttersäureethylester unter Vakuum abdestilliert. Der erhaltene Rückstand wird für 2 bis 4 h im Hochvakuum dampfbehandelt.

Es wird ein Gemisch aus Bernsteinsäuremonoester von Ethyl-3-hydroxybutanoat [= Mono-(4-ethoxy-4-oxo-butan-2-yl)-succinat oder Bernsteinsäuremono-(4-ethoxy-4-oxo-butan-2-yl)-ester] und Bernsteinsäurediester von Ethyl-3-hydroxybutanoat [= Bis-(4-ethoxy-4-oxo-butan-2-yl)-succinat oder Bernsteinsäuredi-(4-ethoxy-4-oxo-butan-2-yl)-ester] erhalten. Die Charakterisierung erfolgt mittels MS, GPC und ¹H-NMR.

Der Bernsteinsäuremonoester von Ethyl-3-hydroxybutanoat kann synonym auch als 4-[(4-Ethoxy-4-oxobutan-2-yl)-oxy]-4-oxobutansäure bezeichnet werden, während der Bernsteinsäurediester von Ethyl-3-hydroxybutanoat synonym auch als 1,4-Bis-(4-ethoxy-4-oxobutan-2-yl)-butandioat oder auch als Bis-(4-ethoxy-4-oxobutan-2-yl)-succinat bezeichnet werden kann.

Im Folgenden ist der Reaktionsverlauf schematisch dargestellt:

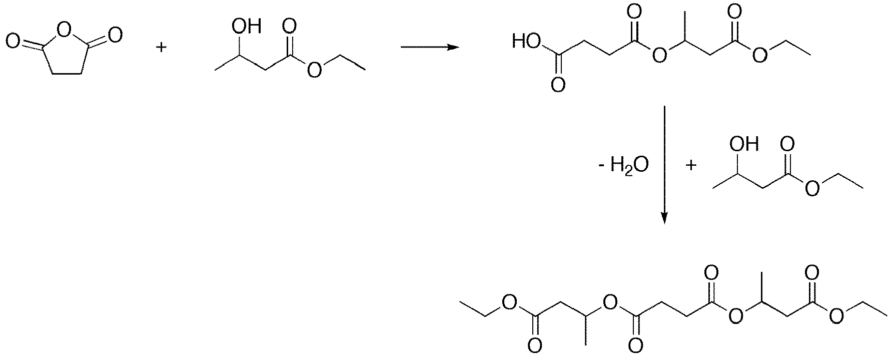

### Variante 2: Verwendung der freien Bernsteinsäure

In einem 500-ml-Mehrhalskolben mit Dephlegmator (Partialkondensator) und Destillationsbrücke werden 132 g (R)/(S)-3- Hydroxybuttersäureethylester (3-BHB-EE = Ethyl-3-hydroxybutanoat oder 4-Ethoxy-4-oxobutan-2-ol) (racemisch) und 33 g Bernsteinsäure vorgelegt. Das Reaktionsgemisch wird unter Rühren bei 120 °C und unter N₂ für 7 h zur Reaktion gebracht und das entstehende Reaktionswasser wird kontinuierlich destillativ entfernt. Anschließend wird der überschüssige 3-Hydroxybuttersäureethylester unter Vakuum abdestilliert. Der erhaltene Rückstand wird für 2 bis 4 h im Hochvakuum dampfbehandelt.

Es wird ein Gemisch aus Bernsteinsäuremonoester von Ethyl-3-hydroxybutanoat [= Mono-(4-ethoxy-4-oxo-butan-2-yl)-succinat oder Bernsteinsäuremono-(4-ethoxy-4-oxo-butan-2-yl)-ester] und Bernsteinsäurediester von Ethyl-3-hydroxybutanoat [= Bis-(4-ethoxy-4-oxo-butan-2-yl)-succinat oder Bernsteinsäuredi-(4-ethoxy-4-oxo-butan-2-yl)-ester] erhalten. Die Charakterisierung erfolgt mittels MS, GPC und ¹H-NMR.

Der Bernsteinsäuremonoester von Ethyl-3-hydroxybutanoat kann synonym auch als 4-[(4-Ethoxy-4-oxobutan-2-yl)-oxy]-4-oxobutansäure bezeichnet werden, während der Bernsteinsäurediester von Ethyl-3-hydroxybutanoat synonym auch als 1,4-Bis-(4-ethoxy-4-oxobutan-2-yl)-butandioat oder auch als Bis-(4-ethoxy-4-oxobutan-2-yl)-succinat bezeichnet werden kann.

Im Folgenden ist der Reaktionsverlauf schematisch dargestellt:

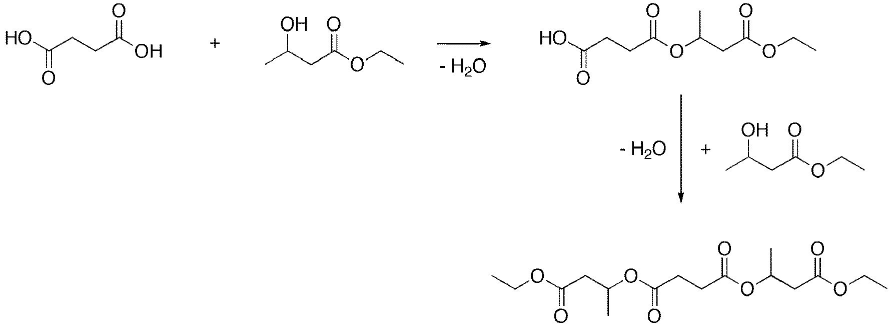

### I. 4. Herstellung von Citronensäureestern von Ethyl-3-hydroxybutanoat

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch) und 32 g Citronensäure vorgelegt. Das Reaktionsgemisch wird unter Rühren bei 120 °C und unter N₂ für 7 h zur Reaktion gebracht und das entstehende Reaktionswasser wird kontinuierlich destillativ entfernt. Anschließend wird der überschüssige 3-Hydroxybuttersäureethylester unter Vakuum abdestilliert. Der erhaltene Rückstand wird für 2 bis 4 h im Hochvakuum dampfbehandelt.

Es wird ein Gemisch aus Citronensäuremonoester von Ethyl-3-hydroxybutanoat [= Mono-(4-ethoxy-4-oxo-butan-2-yl)-citrat oder Citronensäuremono-(4-ethoxy-4-oxo-butan-2-yl)-ester] und Citronensäurediester von Ethyl-3-hydroxybutanoat [= Bis-(4-ethoxy-4-oxo-butan-2-yl)-citrat oder Citronensäuredi-(4-ethoxy-4-oxo-butan-2-yl)-ester] und Citronensäuretriester von Ethyl-3-hydroxybutanoat [= Tris-(4-ethoxy-4-oxo-butan-2-yl)-citrat oder Citronensäuretri-(4-ethoxy-4-oxo-butan-2-yl)-ester] erhalten. Die Charakterisierung erfolgt mittels MS, GPC und ¹H-NMR.

Der Citronensäuremonoester von Ethyl-3-hydroxybutanoat kann synonym auch als 2-{2-[(4-Ethoxy-4-oxobutan-2-yl)-oxy]-2-oxoethyl}-2-hydroxybutansäure oder auch als 2-{2-[(4-Ethoxy-4-oxobutan-2-yl)-oxy]-2-oxoethyl}-2-hydroxysuccinylsäure bezeichnet werden, während der Citronensäurediester von Ethyl-3-hydroxybutanoat synonym auch als 4-[(4-Ethoxy-4-oxobutan-2-yl)-oxy]-2-{2-[(4-ethoxy-4-oxobutan-2-yl)-oxy]-2-oxoethyl}-2-hydroxy-4-oxobutansäure bezeichnet werden kann, und der Citronensäuretriester von Ethyl-3-hydroxybutanoat synonym auch als 1,2,3-Tris-(4-ethoxy-4-oxobutan-2yl)-2-hydroxypropane-1,2,3-tricarboxylat bezeichnet werden kann.

Im Folgenden ist der Reaktionsverlauf schematisch dargestellt:

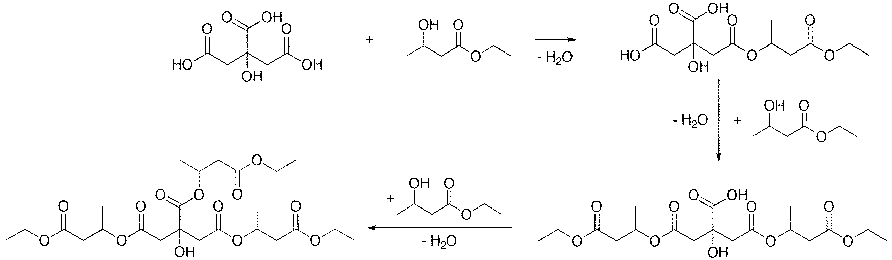

### I. 5. Herstellung von Maleinsäureestern von Ethyl-3-hydroxybutanoat

### Variante 1: Verwendung von Maleinsäureanhydrid

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch) und 29 g Maleinsäureanhydrid vorgelegt. Das Reaktionsgemisch wird unter Rühren bei 120 °C und unter N₂ für 7 h zur Reaktion gebracht und das entstehende Reaktionswasser wird kontinuierlich destillativ entfernt. Anschließend wird der überschüssige 3-Hydroxybuttersäureethylester unter Vakuum abdestilliert. Der erhaltene Rückstand wird für 2 bis 4 h im Hochvakuum dampfbehandelt.

Es wird ein Gemisch aus Maleinsäuremonoester von Ethyl-3-hydroxybutanoat [= Mono-(4-ethoxy-4-oxo-butan-2-yl)-maleat oder Maleinsäuremono-(4-ethoxy-4-oxo-butan-2-yl)-ester] und Maleinsäurediester von Ethyl-3-hydroxybutanoat [= Bis-(4-ethoxy-4-oxo-butan-2-yl)-maleat oder Maleinsäuredi-(4-ethoxy-4-oxo-butan-2-yl)-ester] erhalten. Die Charakterisierung erfolgt mittels MS, GPC und ¹H-NMR.

Im Folgenden ist der Reaktionsverlauf schematisch dargestellt:

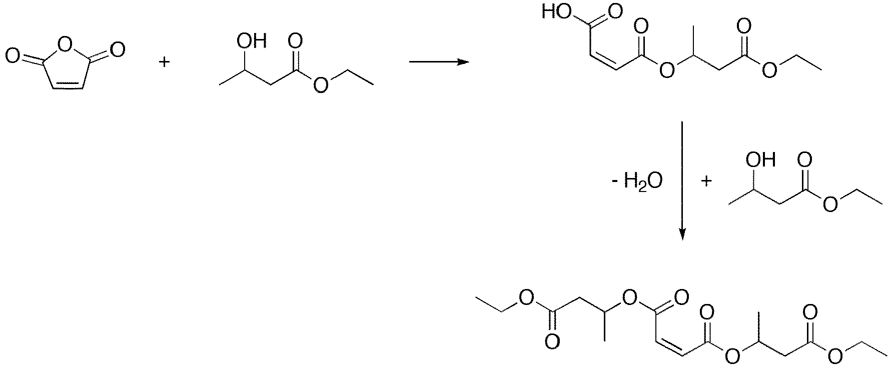

### Variante 2: Verwendung der freien Maleinsäure

In einem 500-ml-Mehrhalskolben mit Dephlegmator (Partialkondensator) und Destillationsbrücke werden 132 g (R)/(S)-3- Hydroxybuttersäureethylester (3-BHB-EE = Ethyl-3-hydroxybutanoat oder 4-Ethoxy-4-oxobutan-2-ol) (racemisch) und 33 g Maleinsäure vorgelegt. Das Reaktionsgemisch wird unter Rühren bei 120 °C und unter N₂ für 7 h zur Reaktion gebracht und das entstehende Reaktionswasser wird kontinuierlich destillativ entfernt. Anschließend wird der überschüssige 3-Hydroxybuttersäureethylester unter Vakuum abdestilliert. Der erhaltene Rückstand wird für 2 bis 4 h im Hochvakuum dampfbehandelt.

Es wird ein Gemisch aus Maleinsäuremonoester von Ethyl-3-hydroxybutanoat [= Mono-(4-ethoxy-4-oxo-butan-2-yl)-maleat oder Maleinsäuremono-(4-ethoxy-4-oxo-butan-2-yl)-ester] und Maleinsäurediester von Ethyl-3-hydroxybutanoat [= Bis-(4-ethoxy-4-oxo-butan-2-yl)-maleat oder Maleinsäuredi-(4-ethoxy-4-oxo-butan-2-yl)-ester] erhalten. Die Charakterisierung erfolgt mittels MS, GPC und ¹H-NMR.

Im Folgenden ist der Reaktionsverlauf schematisch dargestellt:

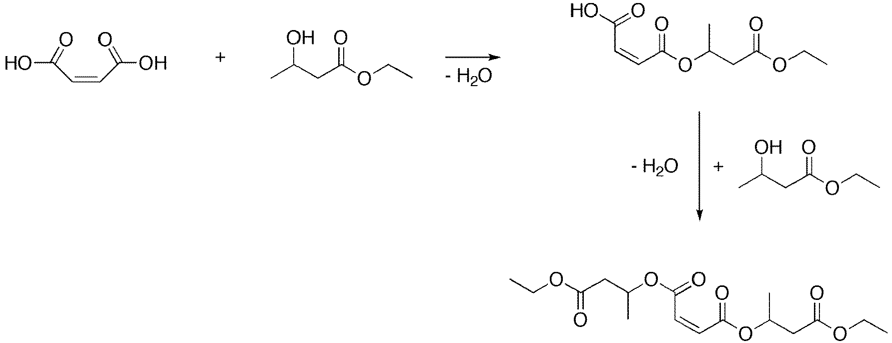

### I. 6. Herstellung von Fumarsäureestern von Ethyl-3-hydroxybutanoat

In einem 500-ml-Mehrhalskolben mit Dephlegmator (Partialkondensator) und Destillationsbrücke werden 132 g (R)/(S)-3- Hydroxybuttersäureethylester (3-BHB-EE = Ethyl-3-hydroxybutanoat oder 4-Ethoxy-4-oxobutan-2-ol) (racemisch) und 33 g Fumarsäure vorgelegt. Das Reaktionsgemisch wird unter Rühren bei 120 °C und unter N₂ für 7 h zur Reaktion gebracht und das entstehende Reaktionswasser wird kontinuierlich destillativ entfernt. Anschließend wird der überschüssige 3-Hydroxybuttersäureethylester unter Vakuum abdestilliert. Der erhaltene Rückstand wird für 2 bis 4 h im Hochvakuum dampfbehandelt.

Es wird ein Gemisch aus Fumarsäuremonoester von Ethyl-3-hydroxybutanoat [= Mono-(4-ethoxy-4-oxo-butan-2-yl)-fumarat oder Fumarsäuremono-(4-ethoxy-4-oxo-butan-2-yl)-ester] und Fumarsäurediester von Ethyl-3-hydroxybutanoat [= Bis-(4-ethoxy-4-oxo-butan-2-yl)-fumarat oder Fumarsäuredi-(4-ethoxy-4-oxo-butan-2-yl)-ester] erhalten. Die Charakterisierung erfolgt mittels MS, GPC und ¹H-NMR.

Im Folgenden ist der Reaktionsverlauf schematisch dargestellt:

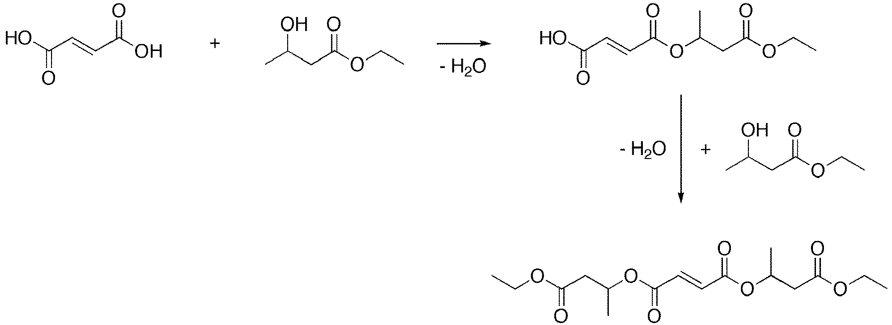

### I. 7. Herstellung von Äpfelsäureestern von Ethyl-3-hydroxybutanoat

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch) und 33 g Äpfelsäure (2-Hydroxybernsteinsäure) vorgelegt. Das Reaktionsgemisch wird unter Rühren bei 120 °C und unter N₂ für 7 h zur Reaktion gebracht und das entstehende Reaktionswasser wird kontinuierlich abgetrennt. Anschließend wird der überschüssige 3-Hydroxybuttersäureethylester unter Vakuum abdestilliert. Der erhaltene Rückstand wird für 2 bis 4 h im Hochvakuum dampfbehandelt.

Es wird ein Gemisch aus Äpfelsäuremonoester von Ethyl-3-hydroxybutanoat [= Mono-(4-ethoxy-4-oxo-butan-2-yl)-malat oder Äpfelsäuremono-(4-ethoxy-4-oxo-butan-2-yl)-ester] und Äpfelsäurediester von Ethyl-3-hydroxybutanoat [= Bis-(4-ethoxy-4-oxo-butan-2-yl)-malat oder Äpfelsäuredi-(4-ethoxy-4-oxo-butan-2-yl)-ester] erhalten. Die Charakterisierung erfolgt mittels MS, GPC und ¹H-NMR.

Der Äpfelsäuremonoester von Ethyl-3-hydroxybutanoat kann synonym auch als 4-[(4-Ethoxy-4-oxobutan-2-yl)-oxy]-2-hydroxy-4-oxobutansäure bezeichnet werden, während der Äpfelsäurediester von Ethyl-3-hydroxybutanoat synonym auch als 1,4-Bis-(4-ethoxy-4-oxobutan-2-yl)-2-hydroxybutanedioat oder auch als Bis-(4-ethoxy-4-oxobutan-2-yl)-2-hydroxysuccinat bezeichnet werden kann.

Im Folgenden ist der Reaktionsverlauf schematisch dargestellt:

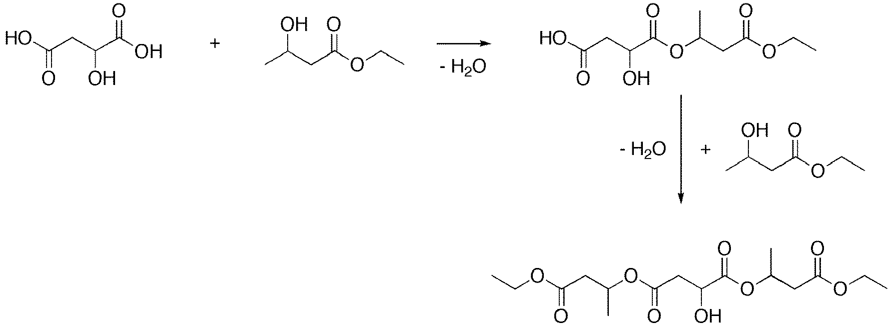

### I. 8. Herstellung von Adipinsäureestern von Ethyl-3-hydroxybutanoat

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch) und 37 g Adipinsäure vorgelegt. Das Reaktionsgemisch wird unter Rühren bei 120 °C und unter N₂ für 7 h zur Reaktion gebracht und das entstehende Reaktionswasser wird kontinuierlich destillativ entfernt. Anschließend wird der überschüssige 3-Hydroxybuttersäureethylester unter Vakuum abdestilliert. Der erhaltene Rückstand wird für 2 bis 4 h im Hochvakuum dampfbehandelt.

Es wird ein Gemisch aus Adipinsäuremonoester von Ethyl-3-hydroxybutanoat [= Mono-(4-ethoxy-4-oxo-butan-2-yl)-adipat oder Adipinsäuremono-(4-ethoxy-4-oxo-butan-2-yl)-ester] und Adipinsäurediester von Ethyl-3-hydroxybutanoat [= Bis-(4-ethoxy-4-oxo-butan-2-yl)-adipat oder Adipinsäuredi-(4-ethoxy-4-oxo-butan-2-yl)-ester] erhalten. Die Charakterisierung erfolgt mittels MS, GPC und ¹H-NMR.

Im Folgenden ist der Reaktionsverlauf schematisch dargestellt:

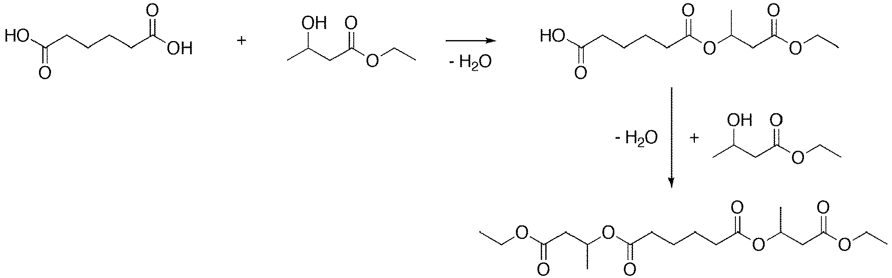

### I. 9. Herstellung weiterer Carbonsäureester von Ethyl-3-hydroxybutanoat

Darüber hinaus werden die vorgenannten Synthesen Nr. I. 1, I. 2, I. 4 und I. 6 bis I.8 jeweils wiederholt, jedoch unter Verwendung der entsprechenden Carbonsäureanhydride anstelle der freien Carbonsäuren (d.h. im Fall von Beispiel I. 1. Weinsäureanhydrid anstelle von Weinsäure, im Fall von Beispiel I. 2 Milchsäureanhydrid anstelle von Milchsäure, im Fall von Beispiel I. 4. Citronensäureanhydrid anstelle von Citronensäure, im Fall von Beispiel I. 6. Fumarsäureanhydrid anstelle von Fumarsäure, im Fall von Beispiel I. 7. Äpfelsäureanhydrid anstelle von Äpfelsäure, im Fall von Beispiel I. 8. Adipinsäureanhydrid anstelle von Adipinsäure).

Es werden vergleichbare Ergebnisse erhalten. In diesem Zusammenhang werden die entsprechenden gebildeten Carbonsäuren während der Reaktion nicht kontinuierlich entfernt, sondern können weiter mit 3-Hydroxybuttersäureethylester reagieren. Nach abgeschlossener Reaktion wird überschüssiger 3-Hydroxybuttersäureethylester bzw. überschüssiges Carbonsäureanhydrid bzw. freie Carbonsäure (in Abhängigkeit von den eingesetzten Mengenverhältnissen an Edukten) destillativ entfernt.

Weiterhin werden die zuvor genannten Herstellungsbeispiele Nr. I. 1 bis I. 8 wiederholt, jedoch jeweils mit anderen 3-Hydroxybutanoaten (nämlich jeweils 3-Hydroxybuttersäure-(hydroxybutyl)-ester bzw. 3-Hydroxybuttersäure-(hydroxypentyl)-ester anstelle von 3-Hydroxybuttersäureethylester). 3-Hydroxybuttersäure-(hydroxybutyl)-ester wird durch Veresterung von 3-Hydroxybuttersäure mit Butandiol (z. B. mit 1,3-Butandiol) erhalten, während 3-Hydroxybuttersäure-(hydroxypentyl)-ester durch Veresterung von 3-Hydroxybuttersäure mit Pentandiol (z. B. mit 1 ,3-Pentandiol) erhalten wird.

Es werden in einer ersten Serie jeweils die freien Carbonsäuren als Edukte eingesetzt und in einer zweiten Serie die entsprechenden Carbonsäureanhydride.

Es werden vergleichbare Ergebnisse erhalten. Aufreinigung und Trennung erfolgen in gleicher Weise.

Auch werden die zuvor genannten Herstellungsbeispiele wiederholt, jedoch mit den Methyl- und Ethylestern der Carbonsäuren. Es werden vergleichbare Ergebnisse erhalten. Aufreinigung und Trennung erfolgen in gleicher Weise.

### II. Herstellungsbeispiele unter Verwendung eines Metallkatalysators

Alle zuvor unter Abschnitt I. beschriebenen chemischen Synthesebeispiele werden erneut durchgeführt, jedoch unter Zugabe von 1,6 g Titantetrabutylat (Titan(IV)-Katalysator). Der Titan(IV)-Katalysator wird zusammen mit den weiteren Reaktanden im Kolben vorgelegt. Im Anschluss entspricht der Reaktionsverlauf den zuvor beschriebenen Beispielen. Es werden vergleichbare Ergebnisse erzielt. Der Katalysator wird nach Reaktionsende abgetrennt und rezykliert.

### III. Herstellungsbeispiele einer weiteren alternativen besonderen Ausführungsform des erfindungsgemäßen Verfahrens unter Verwendung eines Enzymkatalysators

Alle zuvor unter Abschnitt I. beschriebenen chemischen Synthesebeispiele werden erneut durchgeführt, jedoch unter Zugabe eines Enzyms als Katalysators. Es werden vergleichbare Ergebnisse erzielt. Der Katalysator (d. h. das Enzym) wird nach Reaktionsende abgetrennt und rezykliert.

Einige ausgeführte Beispiele sind im Folgenden ausgewählt und beschrieben, wobei alle weiteren zuvor genannten Synthesebeispiele analog durchgeführt werden und vergleichbare Ergebnisse liefern.

### III. 1. Herstellung von Weinsäureestern von Ethyl-3-hydroxybutanoat

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch), 38 g Weinsäure und 1,7 g immobilisiertes Enzym (CALB-Lipase auf Polymerträger, abgeleitet von *Candida antarctica,* z. B. Novozym^{®} 435 von der Fa. Sigma-Aldrich bzw. Merck oder Lipozym^{®} 435 von der Fa. Strem Chemicals, Inc.) vorgelegt. Das Reaktionsgemisch wird unter Rühren bei 70 °C und unter Vakuum (<500 mbar) für 7 h zur Reaktion gebracht. Anschließend wird das Enzym abfiltriert und der überschüssige 3-Hydroxybuttersäureethylester unter Vakuum abdestilliert. Der erhaltene Rückstand wird für 2 bis 4 h im Hochvakuum dampfbehandelt.

Es wird ein Gemisch aus Weinsäuremonoester von Ethyl-3-hydroxybutanoat [= Mono-(4-ethoxy-4-oxo-butan-2-yl)-tartrat oder Weinsäuremono-(4-ethoxy-4-oxo-butan-2-yl)-ester] und Weinsäurediester von Ethyl-3-hydroxybutanoat [= Bis-(4-ethoxy-4-oxo-butan-2-yl)-tartrat oder Weinsäuredi-(4-ethoxy-4-oxo-butan-2-yl)-ester] erhalten. Die Charakterisierung erfolgt mittels MS, GPC und ¹H-NMR.

### III. 2. Herstellung von Milchsäureester von Ethyl-3-hydroxybutanoat

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch), 57 g Milchsäure und 1,8 g immobilisiertes Enzym (CALB-Lipase auf Polymerträger, abgeleitet von *Candida antarctica,* z. B. Novozym^{®} 435 von der Fa. Sigma-Aldrich bzw. Merck oder Lipozym^{®} 435 von der Fa. Strem Chemicals, Inc.) vorgelegt. Das Reaktionsgemisch wird unter Rühren bei 70 °C und unter Vakuum (<500 mbar) für 7 h zur Reaktion gebracht. Anschließend wird das Enzym abfiltriert und der überschüssige 3-Hydroxybuttersäureethylester unter Vakuum abdestilliert. Der erhaltene Rückstand wird für 2 bis 4 h im Hochvakuum dampfbehandelt.

Es wird der Milchsäuremonoester von Ethyl-3-hydroxybutanoat [= Mono-(4-ethoxy-4-oxo-butan-2-yl)-lactat oder Milchsäuremono-(4-ethoxy-4-oxo-butan-2-yl)-ester] erhalten. Die Charakterisierung erfolgt mittels MS, GPC und ¹H-NMR.

### III. 3. Herstellung von Bernsteinsäureestern von Ethyl-3-hydroxybutanoat

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch), 33 g Bernsteinsäure und 1,6 g immobilisiertes Enzym (CALB-Lipase auf Polymerträger, abgeleitet von *Candida antarctica,* z. B. Novozym^{®} 435 von der Fa. Sigma-Aldrich bzw. Merck oder Lipozym^{®} 435 von der Fa. Strem Chemicals, Inc.) vorgelegt. Das Reaktionsgemisch wird unter Rühren bei 70 °C und unter Vakuum (<500 mbar) für 7 h zur Reaktion gebracht. Anschließend wird das Enzym abfiltriert und der überschüssige 3-Hydroxybuttersäureethylester unter Vakuum abdestilliert. Der erhaltene Rückstand wird für 2 bis 4 h im Hochvakuum dampfbehandelt.

Es wird ein Gemisch aus Bernsteinsäuremonoester von Ethyl-3-hydroxybutanoat [= Mono-(4-ethoxy-4-oxo-butan-2-yl)-succinat oder Bernsteinsäuremono-(4-ethoxy-4-oxo-butan-2-yl)-ester] und Bernsteinsäurediester von Ethyl-3-hydroxybutanoat [= Bis-(4-ethoxy-4-oxo-butan-2-yl)-succinat oder Bernsteinsäuredi-(4-ethoxy-4-oxo-butan-2-yl)-ester] erhalten. Die Charakterisierung erfolgt mittels MS, GPC und ¹H-NMR.

### III. 4. Herstellung von Citronensäureestern von Ethyl-3-hydroxybutanoat

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch), 32 g Citronensäure und 1,8 g immobilisiertes Enzym (CALB-Lipase auf Polymerträger, abgeleitet von *Candida antarctica,* z. B. Novozym^{®} 435 von der Fa. Sigma-Aldrich bzw. Merck oder Lipozym^{®} 435 von der Fa. Strem Chemicals, Inc.) vorgelegt. Das Reaktionsgemisch wird unter Rühren bei 70 °C und unter Vakuum (<500 mbar) für 7 h zur Reaktion gebracht. Anschließend wird das Enzym abfiltriert und der überschüssige 3-Hydroxybuttersäureethylester unter Vakuum abdestilliert. Der erhaltene Rückstand wird für 2 bis 4 h im Hochvakuum dampfbehandelt.

Es wird ein Gemisch aus Citronensäuremonoester von Ethyl-3-hydroxybutanoat [= Mono-(4-ethoxy-4-oxo-butan-2-yl)-citrat oder Citronensäuremono-(4-ethoxy-4-oxo-butan-2-yl)-ester] und Citronensäurediester von Ethyl-3-hydroxybutanoat [= Bis-(4-ethoxy-4-oxo-butan-2-yl)-citrat oder Citronensäuredi-(4-ethoxy-4-oxo-butan-2-yl)-ester] und Citronensäuretriester von Ethyl-3-hydroxybutanoat [= Tris-(4-ethoxy-4-oxo-butan-2-yl)-citrat oder Citronensäuretri-(4-ethoxy-4-oxo-butan-2-yl)-ester] von Ethyl-3-hydroxybutanoat erhalten. Die Charakterisierung erfolgt mittels MS, GPC und ¹H-NMR.

### III. 5. Herstellung von Maleinsäureestern von Ethyl-3-hydroxybutanoat

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch), 33 g Maleinsäure und 1,6 g immobilisiertes Enzym (CALB-Lipase auf Polymerträger, abgeleitet von *Candida antarctica,* z. B. Novozym^{®} 435 von der Fa. Sigma-Aldrich bzw. Merck oder Lipozym^{®} 435 von der Fa. Strem Chemicals, Inc.) vorgelegt. Das Reaktionsgemisch wird unter Rühren bei 70 °C und unter Vakuum (<500 mbar) für 7 h zur Reaktion gebracht. Anschließend wird das Enzym abfiltriert und der überschüssige 3-Hydroxybuttersäureethylester unter Vakuum abdestilliert. Der erhaltene Rückstand wird für 2 bis 4 h im Hochvakuum dampfbehandelt.

Es wird ein Gemisch aus Maleinsäuremonoester von Ethyl-3-hydroxybutanoat [= Mono-(4-ethoxy-4-oxo-butan-2-yl)-maleat oder Maleinsäuremono-(4-ethoxy-4-oxo-butan-2-yl)-ester] und Maleinsäurediester von Ethyl-3-hydroxybutanoat [= Bis-(4-ethoxy-4-oxo-butan-2-yl)-maleat oder Maleinsäuredi-(4-ethoxy-4-oxo-butan-2-yl)-ester] erhalten. Die Charakterisierung erfolgt mittels MS, GPC und ¹H-NMR.

### III. 6. Herstellung von Äpfelsäureestern von Ethyl-3-hydroxybutanoat

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch), 33 g Äpfelsäure (2-Hydroxybernsteinsäure) und 1,6 g immobilisiertes Enzym (CALB-Lipase auf Polymerträger, abgeleitet von *Candida antarctica,* z. B. Novozym^{®} 435 von der Fa. Sigma-Aldrich bzw. Merck oder Lipozym^{®} 435 von der Fa. Strem Chemicals, Inc.) vorgelegt. Das Reaktionsgemisch wird unter Rühren bei 70 °C und unter Vakuum (<500 mbar) für 7 h zur Reaktion gebracht. Anschließend wird das Enzym abfiltriert und der überschüssige 3-Hydroxybuttersäureethylester unter Vakuum abdestilliert. Der erhaltene Rückstand wird für 2 bis 4 h im Hochvakuum dampfbehandelt.

Es wird ein Gemisch aus Äpfelsäuremonoester von Ethyl-3-hydroxybutanoat [= Mono-(4-ethoxy-4-oxo-butan-2-yl)-malat oder Äpfelsäuremono-(4-ethoxy-4-oxo-butan-2-yl)-ester] und Äpfelsäurediester von Ethyl-3-hydroxybutanoat [= Bis-(4-ethoxy-4-oxo-butan-2-yl)-malat oder Äpfelsäuredi-(4-ethoxy-4-oxo-butan-2-yl)-ester] erhalten. Die Charakterisierung erfolgt mittels MS, GPC und ¹H-NMR.

### III. 7. Herstellung von Adipinsäureestern von Ethyl-3-hydroxybutanoat

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch) und 37 g Adipinsäure und 1,7 g immobilisiertes Enzym (CALB-Lipase auf Polymerträger, abgeleitet von *Candida antarctica,* z. B. Novozym^{®} 435 von der Fa. Sigma-Aldrich bzw. Merck oder Lipozym^{®} 435 von der Fa. Strem Chemicals, Inc.) vorgelegt. Das Reaktionsgemisch wird unter Rühren bei 70 °C und unter Vakuum (<500 mbar) für 7 h zur Reaktion gebracht. Anschließend wird das Enzym abfiltriert und der überschüssige 3-Hydroxybuttersäureethylester unter Vakuum abdestilliert. Der erhaltene Rückstand wird für 2 bis 4 h im Hochvakuum dampfbehandelt.

Es wird ein Gemisch aus Adipinsäuremonoester von Ethyl-3-hydroxybutanoat [= Mono-(4-ethoxy-4-oxo-butan-2-yl)-adipat oder Adipinsäuremono-(4-ethoxy-4-oxo-butan-2-yl)-ester] und Adipinsäurediester von Ethyl-3-hydroxybutanoat [= Bis-(4-ethoxy-4-oxo-butan-2-yl)-adipat oder Adipinsäuredi-(4-ethoxy-4-oxo-butan-2-yl)-ester] erhalten. Die Charakterisierung erfolgt mittels MS, GPC und ¹H-NMR.

### III. 8. Herstellung weiterer Carbonsäureester von Ethyl-3-hydroxybutanoat

Weiterhin werden die zuvor genannten Herstellungsbeispiele wiederholt, jedoch mit anderen 3-Hydroxybutanoaten (nämlich jeweils 3-Hydroxybuttersäure-(hydroxybutyl)-ester bzw. 3-Hydroxybuttersäure-(hydroxypentyl)-ester anstelle von 3-Hydroxybuttersäureethylester). Es werden vergleichbare Ergebnisse erhalten. Aufreinigung und Trennung erfolgen in gleicher Weise.

Auch werden die zuvor genannten Herstellungsbeispiele wiederholt, jedoch mit den Methyl- und Ethylestern der Carbonsäuren. Es werden vergleichbare Ergebnisse erhalten. Aufreinigung und Trennung erfolgen in gleicher Weise.

### IV. Kinetik und Strukturanalyse

Darüber hinaus wird die Kinetik des Herstellungsverfahrens analysiert. Dazu werden die Umsatz-Zeit-Verläufe der Reaktionen von Bernsteinsäureanhydrid mit Ethyl-3-hydroxybutanoat (3-Hydroxybuttersäureethylester), Citronensäure mit Ethyl-3-hydroxybutanoat (3-Hydroxybuttersäureethylester) und Äpfelsäure mit Ethyl-3-hydroxybutanoat (3-Hydroxybuttersäureethylester) ermittelt.

### IV. 1. Umsetzung von Bernsteinsäureanhydrid mit Ethyl-3-hydroxybutanoat

In einem 500-ml-Mehrhalskolben mit Dephlegmator (Partialkondensator) und Destillationsbrücke werden 525 g (R)/(S)-3-Hydroxybuttersäureethylester (3-BHB-EE = Ethyl-3-hydroxybutanoat oder 4-Ethoxy-4-oxobutan-2-ol) (racemisch) und 133 g Bernsteinsäureanhydrid für 13 h bei 130 °C und 70 mbar zur Reaktion gebracht. Anschließend wird der Ansatz halbiert.

Zu der erste Hälfte werden 100 °C drei Tropfen Titantetrabutylat (Titan(IV)-Katalysator) zur Reaktionsmischung gegeben und die Reaktion für weitere 17 h bei 130 °C gerührt, wobei der Druck schrittweise auf 40 mbar reduziert wird. Nach 7 h der Reaktionszeit wird frisches Titantetrabutylat und nach weiteren 6 h 22 g Molekularsieb (Molsieb) 3 Å zur Entfernung des Reaktionswassers zugegeben. Nach abgeschlossener Reaktionszeit wird das Reaktionsgemisch filtriert.

Während der gesamten Reaktionszeit werden in regelmäßigen Abständen Proben entnommen und die Zusammensetzung analysiert. Der daraus resultierende Umsatz-Zeit-Verlauf ist in Fig. 1 dargestellt.

Aus dem Umsatz-Zeit-Verlauf ergibt sich, dass nach 10 h Reaktionszeit die Umsetzung des Bernsteinsäureanhydrids in der ersten Reaktionsstufe zum BS-E-BHB-Monoester (Bernsteinsäuremonoester von Ethyl-3-hydroxybutanoat) bereits abgeschlossen ist. Die Zugabe des Katalysators führt zu keiner signifikanten Umsatzsteigerung.

Die zweite Hälfte des Ansatzes wird für weiteren Analysen aufgereinigt. Dazu wird zunächst der Überschuss an 3-BHB-EE entfernt und der Rückstand daraufhin säulenchromatographisch aufgereinigt. Zur Struktur-Identifikation der Bernsteinsäureester von Ethyl-3-Hdroxybutanoat werden die einzelnen Ester mittels säulenchromatographischer Aufreinigung jeweils isoliert (d. h. der Monoester und der Diester werden isoliert). Dazu wird der 3-BHB-EE Überschuss entfernt und das Produktgemisch im Anschluss über Kieselgel gesäult. Als Laufmittel wird ein Ethylacetat/Cyclohexan-Gemisch im Verhältnis 2/1 mit 0,5 % Triethylamin verwendet. Anschließend werden die beiden Fraktionen A und B sowie eine nicht fraktionierte Probe mittels GC (Gaschromatographie) analysiert; die GC-Flächenanalysen sind in Tabelle 1 zusammengefasst.

**Tabelle 1: GC-Flächenanalyse [%] der Umsetzung von Bernsteinsäureanhydrid mit Ethyl-3-hydroxybutanoat**

| | **vor Fraktionierung** | **Fraktion A** | **Fraktion B** |
|---|---|---|---|
| **3-BHB-EE** | 0.9 | - | - |
| **3-BHB** | 0.1 | - | - |
| **Bernsteinsäure** | 1.5 | - | - |
| **3-BHB-Derivate *** | 2.1 | 0.11 | - |
| **BS-(BHB-EE)-Monoester** | 77.7 | 97.6 | 0.23 |
| **BS-(BHB-EE)-Diester** | 14.7 | - | 94.39 |
| **Nebenprodukte **** | 1.6 | 1.44 | 4.56 |
| **Unbekannt** | 1.6 | 0.84 | 0.82 |

| | | | |
|---|---|---|---|
| * verschiedene 3-BHB-Derivate (z. B. Oligomere wie Dimere, Trimere und Tetramere etc.) ** verschiedene oligomere und polykondensierte BS-(BHB-EE)-Derivate sowie BS-Derivate (z. B. jeweils Dimere, Trimere, Di- und Polyester etc.) | | | |

Zur weiterführenden Charakterisierung der Fraktionen A und B werden ¹H-NMR-und ¹³C-NMR-Spektren sowie 2D-NMR Spektren (COSY, HSQC, HMBC) gemessen. Die Spektren zeigen (genauso wie die GC-Analyse), dass Fraktion A den Bernsteinsäuremonoester von Ethyl-3-hydroxybutanoat aufweist und Fraktion B den Bernsteinsäurediester von Ethyl-3-hydroxybutanoat aufweist.

Im Folgenden ist die Struktur für den Bernsteinsäuremonoester von Ethyl-3-hydroxybutanoat (aus Fraktion A) dargestellt und die durch die NMR-Spektren eindeutig zuweisbaren chemischen Verschiebungen für die ¹H-NMR-Signale (1), und die ¹³C-NMR-Signale (2) sind gekennzeichnet:

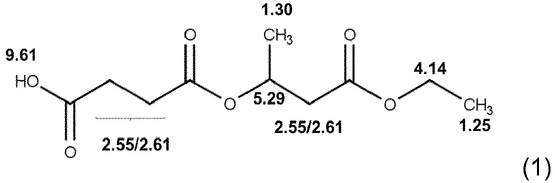

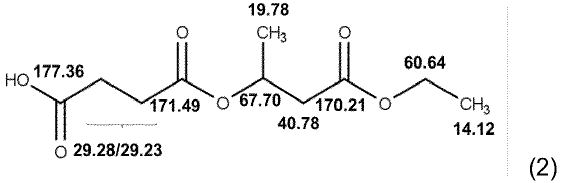

Nachfolgend ist die Struktur für Bernsteinsäurediester von Ethyl-3-hydroxybutanoat (aus Fraktion B) dargestellt und die durch die NMR-Spektren eindeutig zuweisbaren chemischen Verschiebungen für die ¹H-NMR-Signale (3) und die ¹³C-NMR-Signale (4) sind gekennzeichnet:

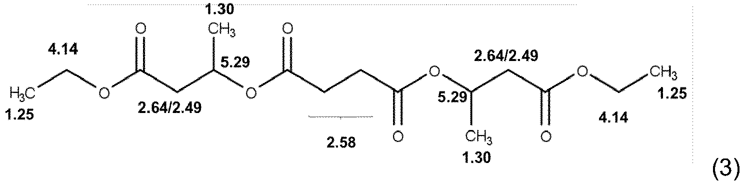

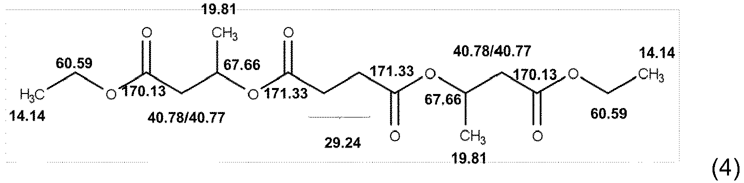

Im Anschluss werden die Fraktionen A und B jeweils weiter aufgereinigt (z. B. chromatographisch). Im Fall der Fraktion A wird reiner Bernsteinsäuremonoester von Ethyl-3-hydroxybutanoat (Reinheit > 99 %) erhalten und im Fall der Fraktion B wird reiner Bernsteinsäurediester von Ethyl-3-hydroxybutanoat (Reinheit > 99 %) erhalten. Aus einem Teil der aufgereinigten Ester wird zudem ein 1 : 1-Gemisch von Monoester/Diester hergestellt.

### IV. 2. Umsetzung von Citronensäure mit Ethyl-3-hydroxybutanoat

In einem 500-ml-Mehrhalskolben mit Dephlegmator (Partialkondensator) und Destillationsbrücke werden 599 g (R)/(S)-3-Hydroxybuttersäureethylester (3-BHB-EE = Ethyl-3-hydroxybutanoat oder 4-Ethoxy-4-oxobutan-2-ol) (racemisch) und 146 g Citronensäure bei 130 °C und 70 mbar zur Reaktion gebracht. Nach 26 h Reaktionszeit werden 67 mg Titantetrabutylat (Titan(IV)-Katalysator) zur Reaktionsmischung gegeben und nach weiteren 3 h Reaktionszeit werden weitere 43 mg Titantetrabutylat (Titan(IV)-Katalysator) zur hinzugegeben. Anschließend wird die Reaktionsmischung für weitere 6 h zur Reaktion gebracht.

Während der gesamten Reaktionszeit werden in regelmäßigen Abständen Proben entnommen und die Zusammensetzung analysiert. Der daraus resultierende Umsatz-Zeit-Verlauf ist in Fig. 2 dargestellt.

Aus dem Umsatz-Zeit-Verlauf ergibt sich, dass Anfangs maßgeblich der Citronensäuremonoester und der Citronensäurediester von Ethyl-3-hydroxybutanoat gebildet werden. Mit der ersten Zugabe des Titan(IV)-Katalysators wird eine geringe Umsatzsteigerung erzielt, jedoch führt die zweite Zugabe zu keiner signifikanten weiteren Umsatzsteigerung.

Das Produktgemisch wird für weitere Analysen aufgereinigt. Dazu wird zunächst der Überschuss an 3-BHB-EE entfernt und der Rückstand anschließend säulenchromatographisch aufgereinigt. Zur Struktur-Identifikation der Citronensäureester von Ethyl 3-Hdroxybutanoat werden die einzelnen Ester jeweils mittels säulenchromatographischer Aufreinigung isoliert. Hierzu wird das Produktgemisch über eine mit Kieselgel gefüllte Chromatographiesäule chromatographiert, als Laufmittel wird ein Ethylacetat/Cyclohexan-Gemisch im Verhältnis 5/3 mit 0,7 % Eisessig verwendet. Anschließend werden die beiden Fraktionen A und B sowie jeweils eine nicht fraktionierte Probe vor und nach der Entfernung des Überschusses an 3-BHB-EE mittels GC (Gaschromatographie) analysiert; die GC-Flächenanalysen sind in Tabelle 2 zusammengefasst.

**Tabelle 2: GC-Flächenanalyse [%] der Umsetzung von Citronensäure mit Ethyl-3-hydroxybutanoat**

| | **vor Fraktionierung; vor 3-BHB-EE Entfernung** | **vor Fraktionierung; nach 3-BHB-EE Entfernung** | **Fraktion A** | **Fraktion B** |
|---|---|---|---|---|
| **3-BHB-EE** | 39.8 | 0.3 | 1 | 0.1 |
| **3-BHB** | 0.1 | 0.1 | - | - |
| **3-BHB-Derivate *** | 14.9 | 8.7 | 1.5 | 5.2 |
| **CS-(BHB-EE)-Monoester** | 8.5 | 15.3 | - | 3.5 |
| **CS-(BHB-EE)-Diester** | 22.2 | 41.8 | 6 | 65.1 |
| **CS-(BHB-EE)-Triester** | 10.7 | 23.1 | 71 | 21.4 |
| **Nebenprodukte **** | 3.3 | 9.5 | 12 | 3.3 |
| **Unbekannt** | 0.6 | 1.3 | 8.4 | 1.4 |

| | | | | |
|---|---|---|---|---|
| * verschiedene 3-BHB-Derivate (z. B. Oligomere wie Dimere, Trimere und Tetramere; deprotonierte Derivate wie Acetoacetate etc.) ** verschiedene oligomere und polykondensierte CS-(BHB-EE)-Derivate sowie CS-Derivate (z. B. jeweils Dimere, Trimere, Di- und Polyester etc.) | | | | |

Die GC-Analyse zeigt, dass vor allem die Di- und Triester der Citronensäure von 3-Hydroxybutanoat gebildet werden; d. h. der Citronensäuremonoester von 3-Hydroxybutanoat fast vollständig weiter zu dem entsprechenden Diester umgesetzt wird. Fraktion A weist maßgeblich den Citronensäuretriester von 3-Hydroxbutanoat auf und Fraktion B weist maßgeblich den Citronensäurediester von 3-Hydroxbutanoat auf.

Zur weiterführenden Charakterisierung der Fraktion A werden ¹H-NMR- und ¹³C-NMR-Spektren sowie 2D-NMR Spektren (COSY, HSQC, HMBC) gemessen. Die Spektren zeigen (genauso wie die GC-Analyse), dass Fraktion A den Citronensäuretriester von Ethyl-3-hydroxybutanoat aufweist. Durch die drei verschiedenen Stereozentren kommt es in zu 4 diastereomeren Verbindungen und entsprechend vielen Signalen innerhalb der einzelnen Gruppen. Das IsomerenGemisch selbst hat eine hohe Reinheit.

Im Folgenden ist die Struktur für den Citronensäuretriester von Ethyl-3-hydroxybutanoat (aus Fraktion A) dargestellt und die durch die NMR-Spektren eindeutig zuweisbaren chemischen Verschiebungen für die ¹H-NMR-Signale (5) und die ¹³C-NMR-Signale (6) sind gekennzeichnet:

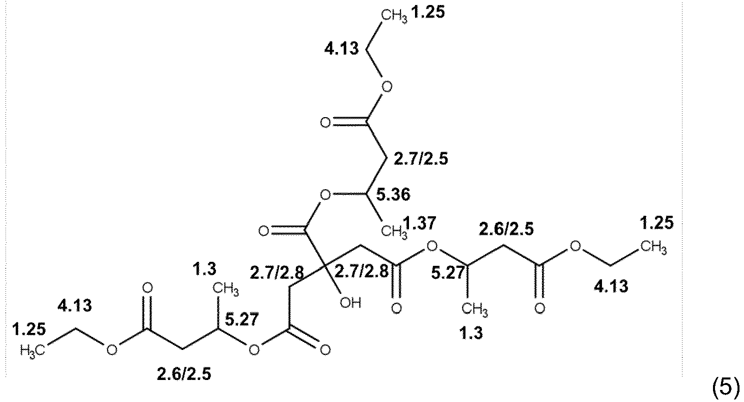

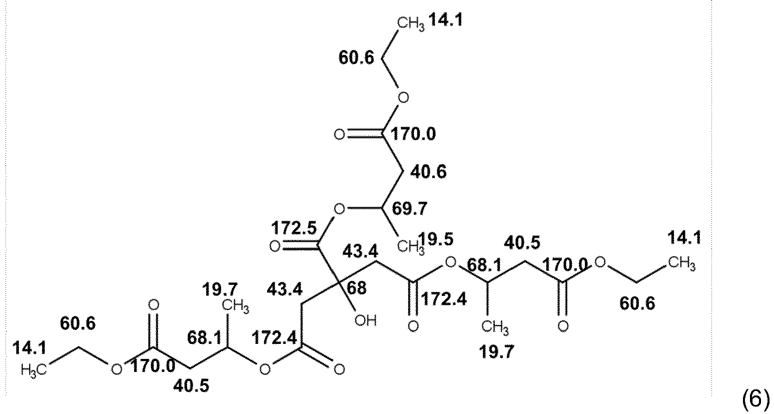

Im Anschluss werden die Fraktionen A und B jeweils weiter aufgereinigt (z. B. chromatographisch). Im Fall der Fraktion A wird reiner Citronensäuretriester von Ethyl-3-hydroxybutanoat (Reinheit > 99 %) erhalten und im Fall der Fraktion B wird eine Mischung aus Citronensäuredi- und Citronensäuremonoester von Ethyl-3-hydroxybutanoat (Reinheit > 99 %) erhalten. Aus einem Teil der aufgereinigten Ester wird zudem ein 1:1:1-Gemisch von Monoester/Diester/Triester hergestellt.

### IV. 3. Umsetzung von Äpfelsäure mit Ethyl-3-hydroxybutanoat

In einem 500-ml-Mehrhalskolben mit Dephlegmator (Partialkondensator) und Destillationsbrücke werden 476 g (R)/(S)-3-Hydroxybuttersäureethylester (3-BHB-EE = Ethyl-3-hydroxybutanoat oder 4-Ethoxy-4-oxobutan-2-ol) (racemisch) und 121 g Äpfelsäure für 10 h bei 120 °C und 70 mbar zur Reaktion gebracht. Anschließend werden 100 g entnommen und der Rest weiter zur Reaktion gebracht. Nach 4 h Reaktionszeit wird die Reaktionsmischung auf 100 °C abgekühlt und es werden 100 mg Titantetrabutylat (Titan(IV)-Katalysator) zur Reaktionsmischung gegeben. Dann wird die Reaktion erneut auf 120 °C aufgeheizt und weitere 24 h bei konstanter Temperatur und 60 mbar gerührt, wobei nach 18 h Reaktionszeit frischer Titantetrabutylat (Titan(IV)-Katalysator) zugegeben wird.

Während der gesamten Reaktionszeit werden in regelmäßigen Abständen Proben entnommen und die Zusammensetzung analysiert. Der daraus resultierende Umsatz-Zeit-Verlauf ist in Fig. 3 dargestellt.

Aus dem Umsatz-Zeit-Verlauf ergibt sich, dass die Zugabe des Titan(IV)-Katalysators zu keiner signifikanten Umsatzsteigerung führt.

Die entnommenen 100 g der Reaktionsmischung werden für weitere Analysen aufgereinigt. Dazu wird zunächst der Überschuss an 3-BHB-EE entfernt und der Rückstand daraufhin säulenchromatographisch aufgereinigt. Dazu wird das Produktgemisch über eine mit Kieselgel gefüllte Chromatographiesäule chromatographiert, als Laufmittel wird ein Ethylacetat/Cyclohexan-Gemisch im Verhältnis 2/1 mit 1 % Eisessig verwendet. Anschließend werden die beiden Fraktionen A und B sowie jeweils eine nicht fraktionierte Probe vor und nach der Entfernung des Überschusses an 3-BHB-EE mittels GC (Gaschromatographie) analysiert; die GC-Flächenanalysen sind in Tabelle 3 zusammengefasst.

**Tabelle 3: GC-Flächenanalyse [%] der Umsetzung von Äpfelsäure mit Ethyl-3-hydroxybutanoat**

| | **vor Fraktionierung** | **Fraktion A** | **Fraktion B** |
|---|---|---|---|
| **3-BHB-EE** | 22.9 | 0.8 | 0.8 |
| **3-BHB** | 0.5 | - | - |
| **Bernsteinsäure** | 0.6 | - | 1.1 |
| **Äpfelsäure** | 13.5 | - | 0.5 |
| **3-BHB-Derivate *** | 5.6 | 3.0 | - |
| **BS-(BHB-EE)-Monoester** | 0.9 | 5.1 | - |
| **Äpfelsäure-(BHB-EE)-Monoester** | 33.3 | 0.2 | 88.9 |
| **Äpfelsäure-Dimer** | 2.4 | 3.4 | - |
| **Äpfelsäure-(BHB-EE)-Diester** | 11.3 | 73.1 | 4.6 |
| **Nebenprodukte **** | 7.2 | 11.9 | 1.2 |
| **Unbekannt** | 1.9 | 2.5 | 1.2 |

| | | | |
|---|---|---|---|
| * verschiedene 3-BHB-Derivate (z. B. Oligomere wie Dimere, Trimere und Tetramere etc.) ** verschiedene oligomere und polykondensierte Äpfelsäure-(BHB-EE)-Derivate sowie Äpfelsäure-Derivate (z. B. jeweils Dimere, Trimere, Di- und Polyester etc.) | | | |

Zur weiterführenden Charakterisierung der Fraktionen A und B werden ¹H-NMR-und ¹³C-NMR-Spektren sowie 2D-NMR Spektren (COSY, HSQC, HMBC) gemessen. Die Spektren zeigen (genauso wie die GC-Analyse), dass Fraktion A den Äpfelsäurediester von Ethyl-3-hydroxybutanoat aufweist und Fraktion B den Äpfelsäuremonoester von Ethyl-3-hydroxybutanoat aufweist.

Im Folgenden ist die Struktur für den Äpfelsäurediester von Ethyl-3-hydroxybutanoat (aus Fraktion A) dargestellt und die durch die NMR-Spektren eindeutig zuweisbaren chemischen Verschiebungen für die ¹H-NMR-Signale (7) und die ¹³C-NMR-Signale (8) sind gekennzeichnet:

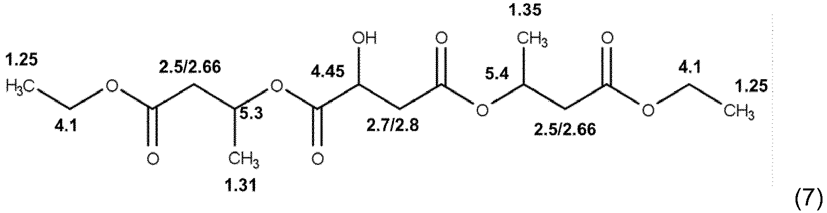

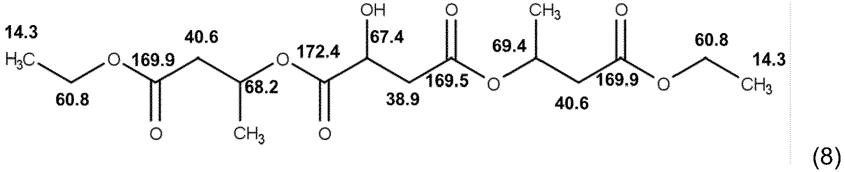

Nachfolgend ist die Struktur für Äpfelsäurediester von Ethyl-3-hydroxybutanoat (aus Fraktion B) dargestellt und die durch die NMR-Spektren eindeutig zuweisbaren chemischen Verschiebungen für die ¹H-NMR-Signale (9) und die ¹³C-NMR-Signale (10) sind gekennzeichnet:

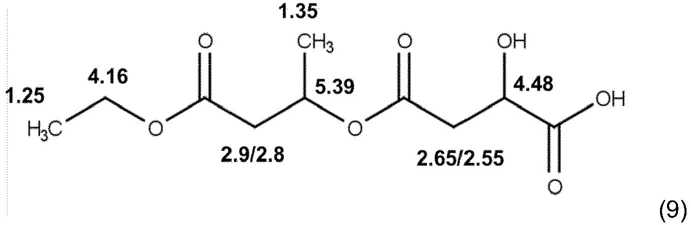

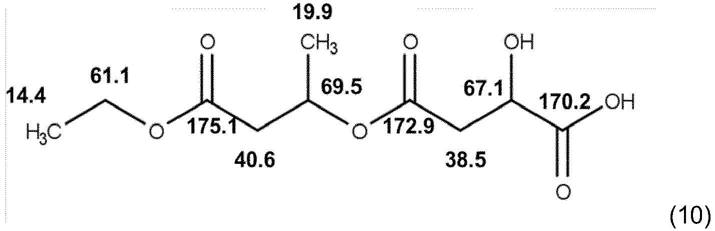

Im Anschluss werden die Fraktionen A und B jeweils weiter aufgereinigt (z. B. chromatographisch). Im Fall der Fraktion A wird reiner Äpfelsäurediester von Ethyl-3-hydroxybutanoat (Reinheit > 99 %) erhalten und im Fall der Fraktion B wird reiner Äpfelsäuremonoester von Ethyl-3-hydroxybutanoat (Reinheit > 99 %) erhalten. Aus einem Teil der aufgereinigten Ester wird zudem ein 1 : 1-Gemisch von Monoester/Diester hergestellt.

### V. Physiologische Anwendunasversuche: in-vitro-Verdauversuche

### V. 1. Verdauversuche (Spalt- bzw. Spaltungsversuche) von erfindungsgemäßen Carbonsäureestern von 3-Hydroxybutanoat

Mittels Spaltungsversuchen wird gezeigt, dass erfindungsgemäß hergestellte Carbonsäureester von 3-Hydroxybutanoat bzw. deren Gemische (vgl. zuvor beschriebene Versuche gemäß I. und III.), einschließlich der Reaktionsnebenprodukte wie Dimeren etc., im menschlichen gastrointestinalen Trakt gespalten werden können.

Als Ausgangsgemisch werden jeweils aufgereinigte, nach dem erfindungsgemäßen Verfahren erhaltene Reaktionsprodukte eingesetzt (d. h. Weinsäuresäureester von 3-Hydroxybutanoat, Milchsäureester von 3-Hydroxybutanoat, Bernsteinsäureester von 3-Hydroxybutanoat, Citronensäureester von 3-Hydroxybutanoat, Maleinsäureester von 3-Hydroxybutanoat, Fumarsäureester von 3-Hydroxybutanoat, Äpfelsäureester von 3-Hydroxybutanoat und Adipinsäureester von 3-Hydroxybutanoat).

Für die Spaltungsversuche unter köpernahen Bedingungen werden zwei Medien untersucht:
- FaSSGF, welches den Magen simuliert
- FaSSIF, welches den Darmtrakt simuliert

Beide Medien stammen von der Firma Biorelevant^{®}, Ltd., Großbritannien. Zusätzlich wird in einigen Experimenten beiden Medien jeweils Schweine-Pankrease zugesetzt (Panzytrat^{®} 40.000, Fa. Allergan).

Die Ergebnisse der Spaltungsversuche in einem FaSSGF- bzw. FaSSIF-Medium mit Panzytrat^{®} und ohne Panzytrat^{®} (jeweils 35 °C, 24 h) zeigen, dass die Proben unter FaSSGF-Bedingungen mit Panzytrat^{®} und ohne Panzytrat^{®} hydrolysieren; dies liegt hauptsächlich am niedrigen pH-Wert (pH = 1,6) des Mediums. Bei FaSSIF-Bedingungen findet eine geringere Umsetzung unter Verwendung von Panzytrat^{®} statt.

Bei den Spaltungsversuchen z. B. der Citronensäuretriester von 3-Hydroxybutanoaten ist zu erkennen, dass die Abspaltung von 3-Hydroxybutanoat in einer Kaskade verläuft (d. h. der Citronensäuretriester wird zum Citronensäurediester, der Citronensäurediester wird zum Citronensäuremonoester und der Citronensäuremonoester wird schließlich zur freien Citronensäure, wobei in jedem Schritt das entsprechende 3-Hydroxybutanoat freigesetzt wird, welches nachfolgend weiterführend zu der freien 3-Hydroxybuttersäure und Ethanol gespalten werden kann).

Entsprechend verlaufen auch die Spaltungsversuche von Dicarbonsäureestern von 3-Hydroxybutanoaten in einer Kaskade. Somit liegt insgesamt ein Retardierungseffekt vor.

### V. 2. Weitere Verdauversuche (Spaltungsversuche) von erfindungsgemäßen Carbonsäuren von 3-Hydroxybutanoat

### Spaltungsversuche mit Pankreatin

2 g eines wie zuvor beschrieben hergestellten Carbonsäureester von 3-Hydroxybutanoat bzw. deren Mischung (z. B. Mischung aus entsprechenden Mono- und Di- bzw. Mono-, Di- und Tricarbonsäureester von 3-Hydroxybutanoat) werden in 50 g Wasser gelöst und mit 0,5 g (1 Gew.-%) Pankreatin versetzt. Das Pankreatin wird in Form des kommerziell verfügbaren Produkts Panzytrat^{®} 40.000 von der Fa. Allergan eingesetzt. Das Ganze wird auf einer Heizplatte bei 50 °C gerührt; der Reaktionsverlauf wird mittels kontinuierlicher Erfassung der Säurezahl über die Zeit ermittelt und verfolgt. Die Säurezahl steigt über den Beobachtungszeitraum an (Spaltung der Carbonsäureester von 3-Hydroxybutanoat zu der freien 3-Hydroxybuttersäure). Der Umsatz/Zeit-Verlauf der wässrigen Spaltung der erfindungsgemäßen Carbonsäureester von 3-Hydroxybuttersäure mittels Pankreatin, einschließlich Zunahme der Säurezahl über die Zeit, belegt die gewünschte Zersetzung des Eduktgemischs zu der freien Carbonsäure und der freien 3-Hydroxybuttersäure. Dies wird durch entsprechende Analytik bestätigt. Der Versuch belegt, dass das erfindungsgemäße Ausgangsgemisch ein geeigneter physiologischer Präkursor für 3-Hydroxybuttersäure bzw. deren Ester (3-Hydroxybutanoate) für die entsprechenden Ketokörpertherapien darstellt.

Der Versuch wird jeweils anhand der einzelnen Ester in Reinform durchgeführt und verifiziert. Es werden jeweils vergleichbare Ergebnisse erhalten, d. h. die Carbonsäureester von 3-Hydroxybutanot werden durch Pankreatin jeweils zu der freien Carbonsäure und 3-Hydroxybutanoat bzw. 3-Hydroxybuttersäure gespalten.

### V. 3. Schlussfolgerung

Die zuvor geschilderten Spaltungsversuche belegen, dass die Carbonsäureester von 3-Hydroxybutanoat effiziente Präkursoren bzw. Metabolite der freien Hydroxybuttersäure bzw. deren Estern (hier: Ethylester, Hydroxybutylester und Hydroxypentylester) darstellen, insbesondere im Hinblick auf ihre beabsichtigte Wirkung, welche in physiologisch verträglicher bzw. physiologisch kompatibler Form vorliegen. Gleichermaßen werden ebenfalls metabolisch verwertbare bzw. umsetzbare, im natürlichen Stoffwechsel (z. B. Citratzyklus) vorkommende Carbonsäuren bzw. deren Derivate, insbesondere Salze, gebildet (z. B. Citronensäure bzw. Citrate, Äpfelsäure bzw. Malate, Weinsäure bzw. Tartrate etc.).

### VI. Weitere Testungen (Oraanoleptik und Toxizität)

Weitere Versuche und Testreihen werden in Bezug auf Organoleptik und Toxizität der erfindungsgemäßen Carbonsäureester von 3-Hydroxybutanoat durchgeführt. Diese zeigen, dass die erfindungsgemäßen Carbonsäureester von 3-Hydroxybutanoat organoleptisch akzeptabel und kompatibel sind, insbesondere im Vergleich zu reiner 3-Hydroxybuttersäure sowie deren Salzen und Estern signifikant verbesserte organoleptische Eigenschaften aufweisen, sowie zudem keine für die Anwendung entgegenstehende Toxizität aufweisen.

## Patentansprüche

1. Verfahren zur Herstellung von Carbonsäureestern von 3-Hydroxybutanoat,
wobei mindestens ein 3-Hydroxybutanoat der allgemeinen Formel (I)
CH₃ - CH(OH) - CH₂ - C(O)OR¹ (I)
wobei in der allgemeinen Formel (I) der Rest R¹ ein C₁-C₅-Alkyl oder ein Hydroxy-Cs-Csalkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
mit mindestens einer Carbonsäure (II), ausgewählt aus der Gruppe von Bernsteinsäure, Weinsäure, Milchsäure, Citronensäure, Äpfelsäure, Adipinsäure, Fumarsäure und Maleinsäure und deren Anhydriden sowie deren Kombinationen oder Mischungen, in einer Veresterungsreaktion und/oder unter Veresterungsbedingungen umgesetzt wird,
wobei die Umsetzung in Abwesenheit von Lösemitteln und/oder ohne jedwedes Lösemittel durchgeführt wird; und
wobei die Umsetzung in Abwesenheit eines Katalysators und/oder ohne jedweden Katalysator durchgeführt wird oder aber die Umsetzung in Gegenwart eines Enzyms als Katalysator durchgeführt wird,
so dass als Reaktionsprodukt (III) ein oder mehrere Carbonsäureester von 3-Hydroxybutanoat erhalten werden.

2. Verfahren nach Anspruch 1,
wobei die Carbonsäure (II) in Form der freien Carbonsäure, in Form eines Salzes der Carbonsäure, in Form eines Esters der Carbonsäure oder in Form des Carbonsäureanhydrids, insbesondere in Form der freien Carbonsäure oder in Form des Carbonsäureanhydrids, eingesetzt wird.

3. Verfahren nach Anspruch 1 oder Anspruch 2,
wobei das 3-Hydroxybutanoat der allgemeinen Formel (I), bezogen auf die Carboxylgruppen der Carbonsäure (II) in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird; und/oder
wobei das 3-Hydroxybutanoat der allgemeinen Formel (I) und die Carbonsäure (II) in einem Molverhältnis von 3-Hydroxybutanoat der allgemeinen Formel (I) / Carbonsäure (II) in einem Bereich von 1 :1 bis 10 : 1, insbesondere in einem Bereich von 2 : 1 bis 8 : 1, vorzugsweise in einem Bereich von 3 : 1 bis 6 : 1, eingesetzt werden.

4. Verfahren nach einem der vorangehenden Ansprüche,
wobei bei der Umsetzung von 3-Hydroxybutanoat der allgemeinen Formel (I) mit einer Carbonsäure (II) in Form der freien Säure gleichzeitig Wasser gebildet wird, insbesondere wobei das Wasser der Umsetzung entzogen wird, insbesondere kontinuierlich entzogen wird, insbesondere mittels vorzugsweise kontinuierlicher, insbesondere destillativer oder adsorptiver Entfernung; und/oder
wobei bei der Umsetzung von 3-Hydroxybutanoat der allgemeinen Formel (I) mit einer Carbonsäure (II) in Form des Anhydrids pro Mol des eingesetzten Anhydrids ein Mol der entsprechenden freien Carbonsäure (II) gebildet wird, insbesondere wobei die entstehende freie Carbonsäure (II) mit 3-Hydroxybutanoat der allgemeinen Formel (I) umgesetzt wird oder nach erfolgter Reaktion entfernt und gegebenenfalls rezykliert wird, insbesondere in Abhängigkeit von den Mengen und/oder Mengenverhältnissen der eingesetzten Ausgangsverbindungen (I) und (II).

5. Verfahren nach einem der vorangehenden Ansprüche,
wobei im Reaktionsprodukt nach erfolgter Umsetzung noch vorhandene Hydroxylgruppen und/oder Carboxylgruppen zumindest teilweise, vorzugsweise vollständig, funktionalisiert, insbesondere verestert werden; und/oder
wobei sich der Umsetzung eine teilweise, insbesondere vollständige Funktionalisierung, insbesondere Veresterung, noch vorhandener Hydroxylgruppen und/oder Carboxylgruppen anschließt.

6. Carbonsäureester von 3-Hydroxybutanoat, wobei der Carbonsäureester von 3-Hydroxybutanoat der allgemeinen Formel (Illb)
CH₃ - CH(OR⁴) - CH₂ - C(O)OR¹ (Illb)
entspricht, wobei in der allgemeinen Formel (Illb)
• der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
• der Rest R⁴ abgeleitet ist von einer Carbonsäure, ausgewählt aus der Gruppe von Weinsäure, Milchsäure, Citronensäure, Äpfelsäure und Fumarsäure sowie deren Kombinationen oder Mischungen ;
insbesondere wobei im Fall der Weinsäure, Citronensäure, Äpfelsäure, und Fumarsäure vorhandene weitere Carboxylgruppen vollständig oder teilweise mit einem Rest - CH(CH₃) - CH₂ - C(O)OR¹ mit R¹ wie zuvor definiert verestert sind.

7. Carbonsäureester von 3-Hydroxybutanoat nach Anspruch 6,
wobei der Carbonsäureester von 3-Hydroxybutanoat der allgemeinen Formel (IIIb)
CH₃ - CH(OR⁴) - CH₂ - C(O)OR¹ (IIIb)
entspricht, wobei in der allgemeinen Formel (IIIb)
• der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
• der Rest R⁴ einen oder mehrere der folgenden Reste darstellt 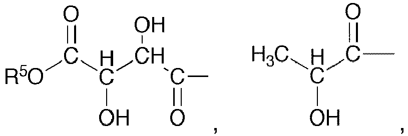 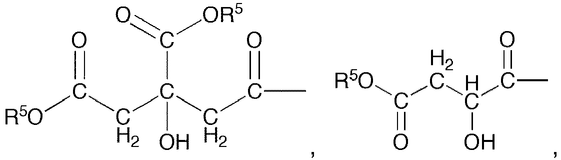 oder 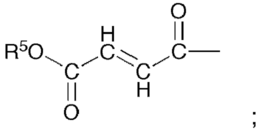
wobei in den vorstehenden Resten der Rest R⁵ Wasserstoff oder einen Rest - CH(CH₃) - CH₂ - C(O)OR¹ mit R¹ wie zuvor definiert darstellt.

8. Gemisch, umfassend mindestens zwei voneinander verschiedene Carbonsäureester von 3-Hydroxybutanoat (III), erhältlich gemäß einem Verfahren nach den Ansprüchen 1 bis 5.

9. Pharmazeutische Zusammensetzung, insbesondere Arzneimittel oder Medikament, umfassend einen Carbonsäureester von 3-Hydroxybutanoat (III) oder ein Gemisch von mindestens zwei voneinander verschiedenen Carbonsäureestern von 3-Hydroxybutanoat (III),
wobei der Carbonsäureester von 3-Hydroxybutanoat (III) der allgemeinen Formel (IIIb)
CH₃ - CH(OR⁴) - CH₂ - C(O)OR¹ (IIIb)
entspricht, wobei in der allgemeinen Formel (IIIb)
• der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
• der Rest R⁴ einen oder mehrere der folgenden Reste darstellt 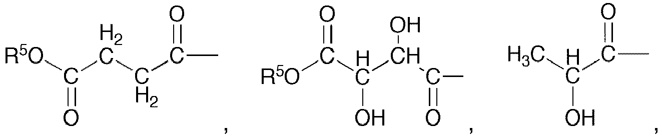 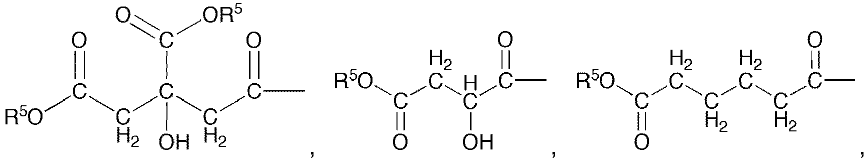 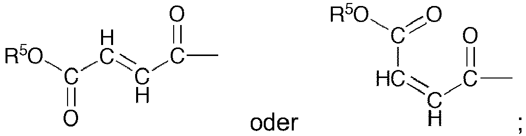
wobei in den vorstehenden Resten der Rest R⁵ Wasserstoff oder einen Rest - CH(CH₃) - CH₂ - C(O)OR¹ mit R¹ wie zuvor definiert darstellt.

10. Pharmazeutische Zusammensetzung nach Anspruch 9 zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

11. Carbonsäureester von 3-Hydroxybutanoat (III) oder Gemisch von mindestens zwei voneinander verschiedenen Carbonsäureestern von 3-Hydroxybutanoat (III),
wobei der Carbonsäureester von 3-Hydroxybutanoat (III) der allgemeinen Formel (IIIb)
CH₃ - CH(OR⁴) - CH₂ - C(O)OR¹ (IIIb)
entspricht, wobei in der allgemeinen Formel (IIIb)
• der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
• der Rest R⁴ einen oder mehrere der folgenden Reste darstellt 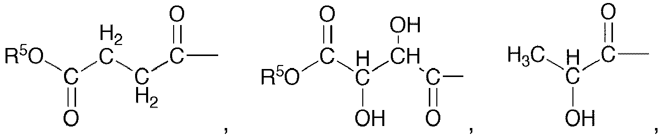 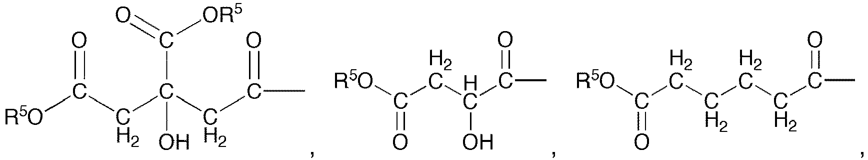 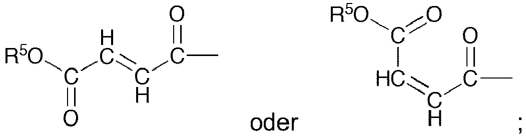
wobei in den vorstehenden Resten der Rest R⁵ Wasserstoff oder einen Rest - CH(CH₃) - CH₂ - C(O)OR¹ mit R¹ wie zuvor definiert darstellt, zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

12. Verwendung eines Carbonsäureesters von 3-Hydroxybutanoat (III) oder eines Gemischs von mindestens zwei voneinander verschiedenen Carbonsäureestern von 3-Hydroxybutanoat (III),
wobei der Carbonsäureester von 3-Hydroxybutanoat (III) der allgemeinen Formel (IIIb)
CH₃ - CH(OR⁴) - CH₂ - C(O)OR¹ (IIIb)
entspricht, wobei in der allgemeinen Formel (IIIb)
• der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
• der Rest R⁴ einen oder mehrere der folgenden Reste darstellt 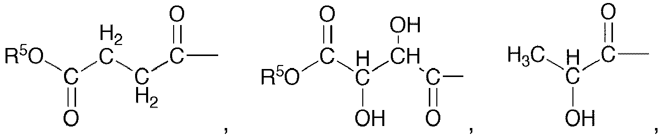 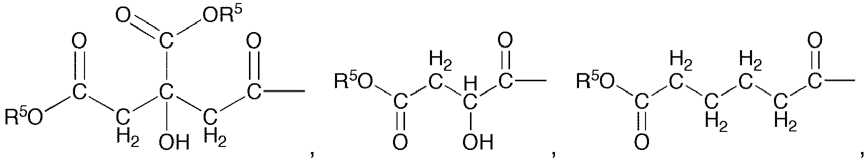 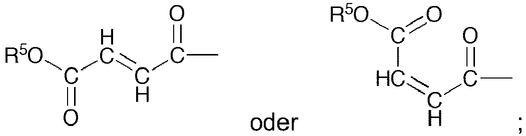
wobei in den vorstehenden Resten der Rest R⁵ Wasserstoff oder einen Rest - CH(CH₃) - CH₂ - C(O)OR¹ mit R¹ wie zuvor definiert darstellt, zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

13. Verwendung eines Carbonsäureesters von 3-Hydroxybutanoat (III) oder eines Gemischs von mindestens zwei voneinander verschiedenen Carbonsäureestern von 3-Hydroxybutanoat (III),
wobei der Carbonsäureester von 3-Hydroxybutanoat (III) der allgemeinen Formel (IIIb)
CH₃ - CH(OR⁴) - CH₂ - C(O)OR¹ (IIIb)
entspricht, wobei in der allgemeinen Formel (IIIb)
• der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
• der Rest R⁴ einen oder mehrere der folgenden Reste darstellt 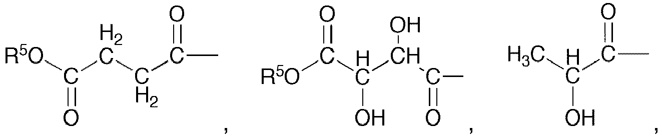 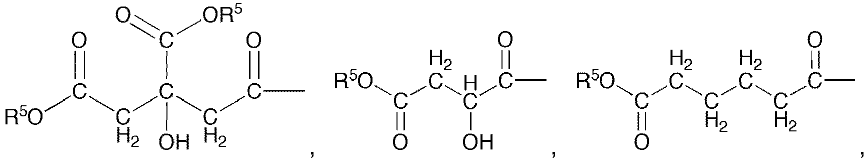 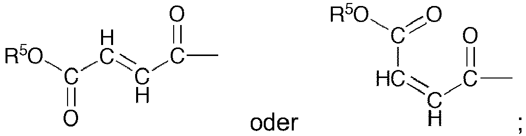
wobei in den vorstehenden Resten der Rest R⁵ Wasserstoff oder einen Rest - CH(CH₃) - CH₂ - C(O)OR¹ mit R¹ wie zuvor definiert darstellt, zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung.

14. Nahrungsmittel- und/oder Lebensmittelerzeugnis, umfassend einen Carbonsäureester von 3-Hydroxybutanoat (III) oder ein Gemisch von mindestens zwei voneinander verschiedenen Carbonsäureestern von 3-Hydroxybutanoat (III),
wobei der Carbonsäureester von 3-Hydroxybutanoat (III) der allgemeinen Formel (IIIb)
CH₃ - CH(OR⁴) - CH₂ - C(O)OR¹ (IIIb)
entspricht, wobei in der allgemeinen Formel (IIIb)
• der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
• der Rest R⁴ einen oder mehrere der folgenden Reste darstellt 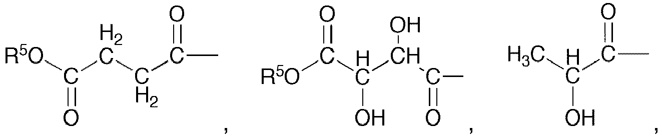 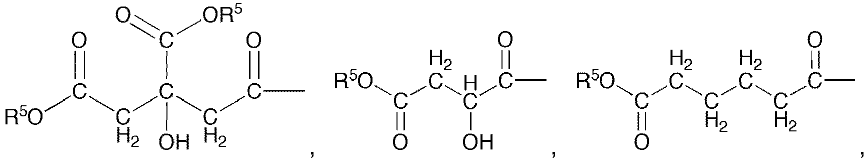 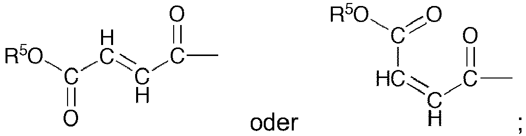
wobei in den vorstehenden Resten der Rest R⁵ Wasserstoff oder einen Rest - CH(CH₃) - CH₂ - C(O)OR¹ mit R¹ wie zuvor definiert darstellt.

15. Nahrungsmittel- und/oder Lebensmittelerzeugnis nach Anspruch 14, wobei das Nahrungsmittel- und/oder Lebensmittelerzeugnis ein Nahrungsergänzungsmittel, ein funktionelles Lebensmittel (*Functional Food*), ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement ist.

## Claims

1. A method for producing carboxylic acid esters of 3-hydroxybutanoate,
wherein at least one 3-hydroxybutanoate of the general formula (I)
CH₃ - CH(OH) - CH₂ - C(O)OR¹ (I)
wherein, in general formula (I), the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl,
is reacted with at least one carboxylic acid (II), selected from the group of succinic acid, tartaric acid, lactic acid, citric acid, malic acid, adipic acid, fumaric acid and maleic acid and anhydrides thereof as well as combinations or mixtures thereof, in an esterification reaction and/or under esterification conditions,
wherein the reaction is carried out in the absence of solvents and/or without any solvent; and
wherein the reaction is carried out in the absence of a catalyst and/or without any catalyst or else the reaction is carried out in the presence of an enzyme as a catalyst,
so that, as a reaction product (III), one or more carboxylic acid esters of 3-hydroxybutanoate are obtained.

2. The method according to claim 1,
wherein the carboxylic acid (II) is used in the form of the free carboxylic acid, in the form of a salt of the carboxylic acid, in the form of an ester of the carboxylic acid or in the form of the carboxylic acid anhydride, especially in the form of the free carboxylic acid or in the form of the carboxylic acid anhydride.

3. The method according to claim 1 or claim 2,
wherein the 3-hydroxybutanoate of the general formula (I), based on the carboxyl groups of the carboxylic acid (II), is used in molar amounts in a range of from equimolar amount up to a molar excess of 200 mol-%, especially in a range of from equimolar amount up to a molar excess of 150 mol-%, preferentially in a range of from equimolar amount to a molar excess of 100 mol-%; and/or
wherein the 3-hydroxybutanoate of the general formula (I) and the carboxylic acid (II) are used in a molar ratio of 3-hydroxybutanoate of the general formula (I)/carboxylic acid (II) in a range of from 1 :1 to 10 : 1, especially in a range of from 2 : 1 to 8 : 1, preferentially in a range of from 3 : 1 to 6 : 1.

4. The method according to any of the preceding claims,
wherein, during the reaction of 3-hydroxybutanoate of the general formula (I) with a carboxylic acid (II) in the form of the free acid, water is formed simultaneously, especially wherein the water is withdrawn from the reaction, especially continuously withdrawn, especially by means of preferentially continuous, especially distillative or adsorptive removal; and/or
wherein, during the reaction of 3-hydroxybutanoate of the general formula (I) with a carboxylic acid (II) in the form of the anhydride, one mol of the corresponding free carboxylic acid (II) is formed per mol of the anhydride used, especially wherein the resulting free carboxylic acid (II) is reacted with 3-hydroxybutanoate of the general formula (I) or, after the reaction has taken place, is removed and optionally recycled, especially depending on the amounts and/or ratios of the starting compounds (I) and (II) used.

5. The method according to any of the preceding claims,
wherein hydroxyl groups and/or carboxyl groups still present in the reaction product after the reaction has been performed are at least partially, preferentially completely, functionalized, especially esterified; and/or
wherein the reaction is followed by a partial, especially complete functionalization, especially esterification, of hydroxyl groups and/or carboxyl groups still present.

6. A carboxylic acid ester of 3-hydroxybutanoate, wherein the carboxylic acid ester of 3-hydroxybutanoate corresponds to the general formula (IIIb)
CH₃ - CH(OR⁴) - CH₂ - C(O)OR¹ (IIIb)
wherein, in the general formula (IIIb),
• the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl, and
• the radical R⁴ is derived from a carboxylic acid selected from the group of tartaric acid, lactic acid, citric acid, malic acid and fumaric acid as well as combinations or mixtures thereof;
especially wherein, in the case of tartaric acid, citric acid, malic acid and fumaric acid, further carboxyl groups present are esterified completely or partially with a radical - CH(CH₃) - CH₂ - C(O)OR¹ with R¹ as defined hereinabove.

7. The carboxylic acid ester of 3-hydroxybutanoate according to claim 6,
wherein the carboxylic acid ester of 3-hydroxybutanoate corresponds to the general formula (IIIb)
CH₃ - CH(OR⁴) - CH₂ - C(O)OR¹ (IIIb)
wherein, in the general formula (IIIb),
• the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl, and
• the radical R⁴ represents one or more of the following radicals 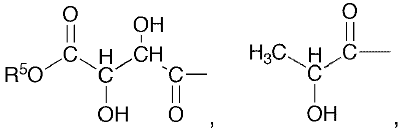 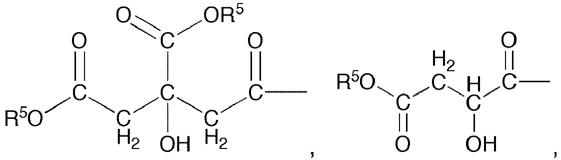 or 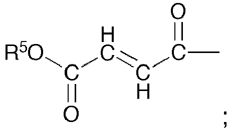
wherein, in the above radicals, the radical R⁵ represents hydrogen or a radical - CH(CH₃) - CH₂ - C(O)OR¹ with R¹ as defined hereinabove.

8. A mixture comprising at least two different carboxylic acid esters of 3-hydroxybutanoate (III), obtainable by a method according to claims 1 to 5.

9. A pharmaceutical composition, especially a drug or medicament, comprising a carboxylic acid ester of 3-hydroxybutanoate (III) or a mixture of at least two different carboxylic acid ester of 3-hydroxybutanoate (III),
wherein the carboxylic acid ester of 3-hydroxybutanoate (III) corresponds to the general formula (lllb)
CH₃ - CH(OR⁴) - CH₂ - C(O)OR¹ (IIIb)
wherein, in the general formula (IIIb),
• the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl, and
• the radical R⁴ represents one or more of the following radicals 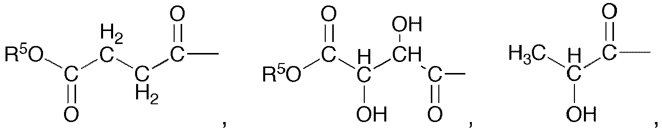 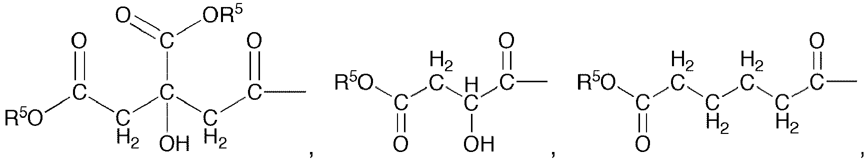 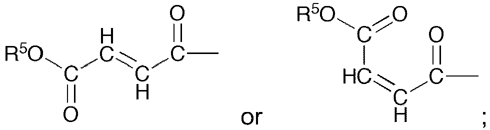
wherein, in the above radicals, the radical R⁵ represents hydrogen or a radical - CH(CH₃) - CH₂ - C(O)OR¹ with R¹ as defined hereinabove.

10. The pharmaceutical composition according to claim 9 for use in the prophylactic and/or therapeutic treatment of diseases of the human or animal body, especially diseases associated with a disorder of the energy metabolism, especially keto-body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus and effects or side-effects of chemotherapy.

11. A carboxylic acid ester of 3-hydroxybutanoate (III) or a mixture of at least two different carboxylic acid ester of 3-hydroxybutanoate (III),
wherein the carboxylic acid ester of 3-hydroxybutanoate (III) corresponds to the general formula (lllb)
CH₃ - CH(OR⁴) - CH₂ - C(O)OR¹ (IIIb)
wherein, in the general formula (IIIb),
• the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl, and
• the radical R⁴ represents one or more of the following radicals 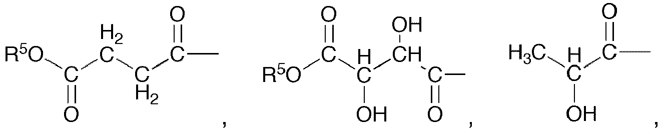 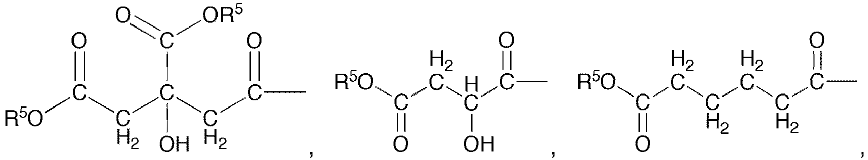 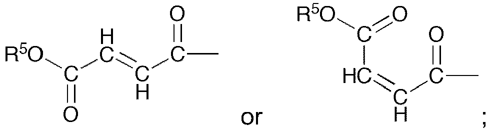
wherein, in the above radicals, the radical R⁵ represents hydrogen or a radical - CH(CH₃) - CH₂ - C(O)OR¹ with R¹ as defined hereinabove, for use in the prophylactic and/or therapeutic treatment of diseases of the human or animal body, especially diseases associated with a disorder of the energy metabolism, especially keto-body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus and effects or side-effects of chemotherapy.

12. Use of a carboxylic acid ester of 3-hydroxybutanoate (III) or a mixture of at least two different carboxylic acid ester of 3-hydroxybutanoate (III),
wherein the carboxylic acid ester of 3-hydroxybutanoate (III) corresponds to the general formula (lllb)
CH₃ - CH(OR⁴) - CH₂ - C(O)OR¹ (IIIb)
wherein, in the general formula (IIIb),
• the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl, and
• the radical R⁴ represents one or more of the following radicals 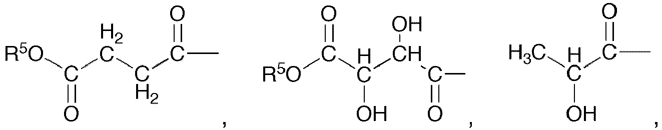 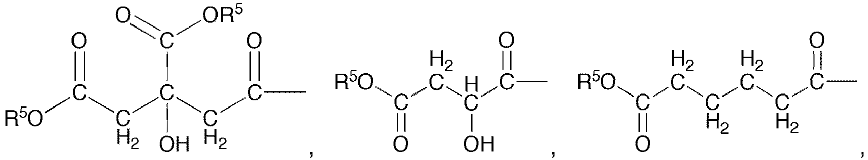 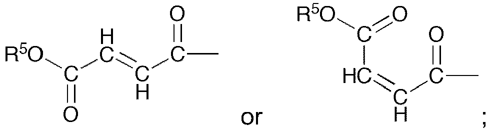
wherein, in the above radicals, the radical R⁵ represents hydrogen or a radical - CH(CH₃) - CH₂ - C(O)OR¹ with R¹ as defined hereinabove, for producing a medicament for the prophylactic and/or therapeutic treatment of diseases of the human or animal body, especially diseases associated with a disorder of the energy metabolism, especially keto-body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus and effects or side-effects of chemotherapy.

13. Use of a carboxylic acid ester of 3-hydroxybutanoate (III) or a mixture of at least two different carboxylic acid ester of 3-hydroxybutanoate (III),
wherein the carboxylic acid ester of 3-hydroxybutanoate (III) corresponds to the general formula (lllb)
CH₃ - CH(OR⁴) - CH₂ - C(O)OR¹ (IIIb)
wherein, in the general formula (IIIb),
• the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl, and
• the radical R⁴ represents one or more of the following radicals 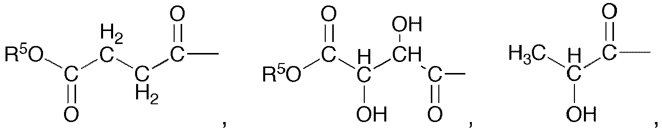 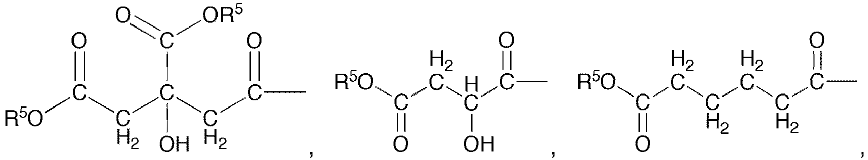 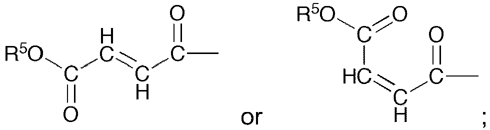
wherein, in the above radicals, the radical R⁵ represents hydrogen or a radical - CH(CH₃) - CH₂ - C(O)OR¹ with R¹ as defined hereinabove, for producing a medicament for the prophylactic and/or therapeutic treatment of or for the application for catabolic metabolic states, such as hunger, diets or low-carbohydrate nutrition.

14. A food and/or a food product comprising a carboxylic acid ester of 3-hydroxybutanoate (III) or a mixture of at least two different carboxylic acid ester of 3-hydroxybutanoate (III),
wherein the carboxylic acid ester of 3-hydroxybutanoate (III) corresponds to the general formula (lllb)
CH₃ - CH(OR⁴) - CH₂ - C(O)OR¹ (IIIb)
wherein, in the general formula (IIIb),
• the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl, and
• the radical R⁴ represents one or more of the following radicals 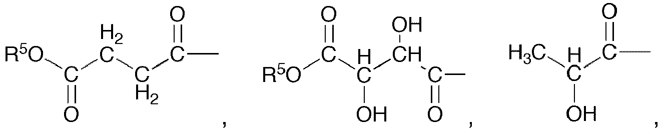 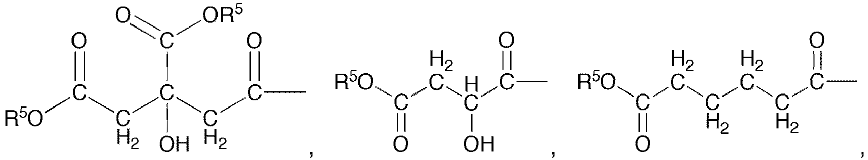 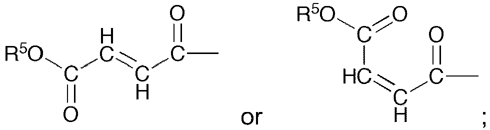
wherein, in the above radicals, the radical R⁵ represents hydrogen or a radical - CH(CH₃) - CH₂ - C(O)OR¹ with R¹ as defined hereinabove.

15. The food and/or food product according to claim 14, wherein the food and/or the food product is a dietary supplement, a functional food, a novel food, a food additive, a food supplement, a dietary food, a power snack, an appetite suppressant or a strength and/or endurance sports supplement.

## Revendications

1. Procédé de préparation d'esters d'acides carboxyliques du 3-hydroxybutanoate,
où l'on fait réagir au moins un 3-hydroxybutanoate de formule générale (I)
CH₃ - CH(OH) - CH₂ - C(O)OR¹ (I)
où, dans la formule générale (I), le radical R¹ représente un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle,
avec au moins un acide carboxylique (II) choisi dans le groupe constitué par l'acide succinique, l'acide tartrique, l'acide lactique, l'acide citrique, l'acide malique, l'acide adipique, l'acide fumarique et l'acide maléique et leurs anhydrides ainsi que leurs combinaisons ou mélanges, dans une réaction d'estérification et/ou dans des conditions d'estérification,
la réaction étant effectuée en l'absence de solvants et/ou sans aucun solvant; et
où la réaction est effectuée en l'absence d'un catalyseur et/ou sans aucun catalyseur, ou bien la réaction est effectuée en présence d'une enzyme comme catalyseur,
de manière à obtenir comme produit de réaction (III) un ou plusieurs esters d'acide carboxylique de 3-hydroxybutanoate.

2. Procédé selon la revendication 1,
l'acide carboxylique (II) étant utilisé sous forme de l'acide carboxylique libre, sous forme d'un sel de l'acide carboxylique, sous forme d'un ester de l'acide carboxylique ou sous forme de l'anhydride de l'acide carboxylique, en particulier sous forme de l'acide carboxylique libre ou sous forme de l'anhydride de l'acide carboxylique.

3. Procédé selon la revendication 1 ou la revendication 2,
le 3-hydroxybutanoate de formule générale (I) étant utilisé, par rapport aux groupes carboxyle de l'acide carboxylique (II), en quantités molaires dans une plage allant d'une quantité équimolaire jusqu'à un excès molaire de 200 % en moles, en particulier dans une plage allant d'une quantité équimolaire jusqu'à un excès molaire de 150 moles %, de préférence dans une plage allant d'une quantité équimolaire jusqu'à un excès molaire de 100 % en moles; et/ou
le 3-hydroxybutanoate de formule générale (I) et l'acide carboxylique (II) étant utilisés dans un rapport molaire 3-hydroxybutanoate de formule générale (I)/acide carboxylique (II) dans une plage de 1:1 à 10:1, en particulier dans une plage de 2:1 à 8:1, de préférence dans une plage de 3:1 à 6:1.

4. Procédé selon l'une quelconque des revendications précédentes,
où, lors de la réaction du 3-hydroxybutanoate de formule générale (I) avec un acide carboxylique (II) sous forme d'acide libre, de l'eau est formée simultanément, en particulier où l'eau est extraite de la réaction, en particulier extraite en continu, notamment au moyen d'une élimination de préférence continue, en particulier par distillation ou adsorption; et/ou
où, lors de la réaction du 3-hydroxybutanoate de formule générale (I) avec un acide carboxylique (II) sous forme d'anhydride, une mole de l'acide carboxylique libre (II) correspondant est formée par mole de l'anhydride utilisé, en particulier où l'acide carboxylique libre (II) formé est mis à réagir avec le 3-hydroxybutanoate de formule générale (I) ou est éliminé et éventuellement recyclé après que la réaction a eu lieu, en particulier en fonction des quantités et/ou des rapports quantitatifs des composés de départ (I) et (II) utilisés.

5. Procédé selon l'une quelconque des revendications précédentes,
les groupes hydroxyle et/ou les groupes carboxyle encore présents dans le produit de réaction après la réaction étant au moins partiellement, de préférence complètement, fonctionnalisés, en particulier estérifiés; et/ou
la réaction étant suivie d'une fonctionnalisation partielle, en particulier complète, en particulier d'une estérification, de groupes hydroxyle et/ou de groupes carboxyle encore présents.

6. L'ester d'acide carboxylique du 3-hydroxybutanoate, l'ester d'acide carboxylique du 3-hydroxybutanoate répondant à la formule générale (IIIb)
CH₃- CH(OR⁴) - CH₂ - C(O)OR¹ (IIIb)
où dans la formule générale (IIIb)
• le radical R¹ représente un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle et
• le radical R⁴ est dérivé d'un acide carboxylique choisi dans le groupe constitué par l'acide tartrique, l'acide lactique, l'acide citrique, l'acide malique et l'acide fumarique, ainsi que leurs combinaisons ou mélanges;
en particulier, dans le cas de l'acide tartrique, de l'acide citrique, de l'acide malique et de l'acide fumarique, les autres groupes carboxyliques présents étant totalement ou partiellement liés à un radical - CH(CH₃) - CH₂ - C(O)OR¹ avec R¹ comme défini précédemment.

7. Ester d'acide carboxylique de 3-hydroxybutanoate selon la revendication 6,
où l'ester d'acide carboxylique du -3-hydroxybutanoate de formule générale (IIIb)
CH₃- CH(OR⁴) - CH₂ - C(O)OR¹ (IIIb)
où dans la formule générale (IIIb)
• le radical R¹ représente un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle et
• le radical R⁴ représente un ou plusieurs des radicaux suivants 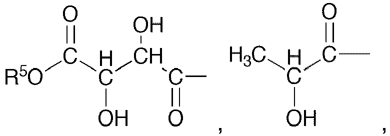 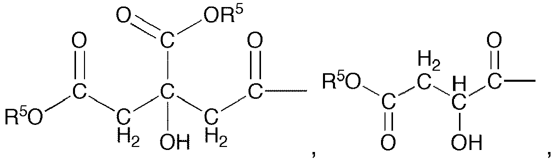 ou 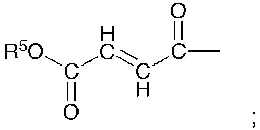
où, dans les radicaux précédents, le radical R⁵ représente un atome d'hydrogène ou un radical - CH(CH₃) - CH₂ - C(O)OR¹ avec R¹ tel que défini précédemment.

8. Mélange comprenant au moins deux esters d'acide carboxylique de 3-hydroxybutanoate (III) différents l'un de l'autre, pouvant être obtenu selon un procédé selon les revendications 1 à 5.

9. Composition pharmaceutique, en particulier médicament ou produit pharmaceutique, comprenant un ester d'acide carboxylique de -3-hydroxybutanoate (III) ou un mélange d'au moins deux esters d'acide carboxylique de 3-hydroxybutanoate (III) différents l'un de l'autre,
où l'ester d'acide carboxylique du 3-hydroxybutanoate (III) de formule générale (IIIb)
CH₃ - CH(OR⁴) - CH₂ - C(O)OR¹ (IIIb)
où dans la formule générale (IIIb)
• le radical R¹ représente un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle et
• le radical R⁴ représente un ou plusieurs des radicaux suivants 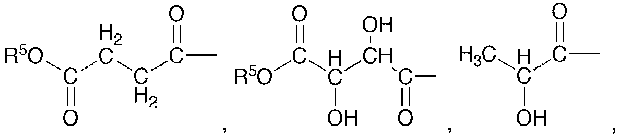 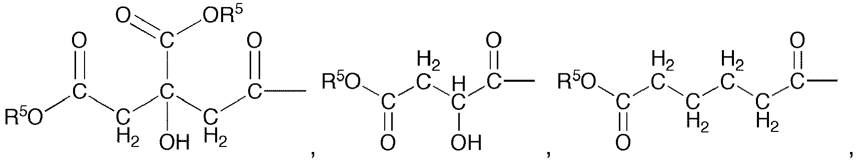 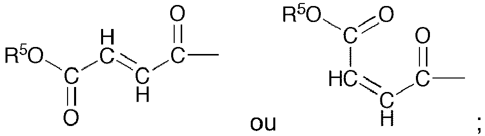
où, dans les radicaux précédents, le radical R⁵ représente un atome d'hydrogène ou un radical - CH(CH₃) - CH₂ - C(O)OR¹ avec R¹ tel que défini précédemment.

10. Composition pharmaceutique selon la revendication 9 pour son utilisation dans le traitement prophylactique et/ou thérapeutique de maladies du corps humain ou animal, en particulier de maladies liées à un trouble du métabolisme énergétique, en particulier du métabolisme des corps cétoniques, comme notamment les traumatismes crâniens, les accidents vasculaires cérébraux, les hypoxies, les maladies cardiovasculaires comme l'infarctus du myocarde, le syndrome de réalimentation, les anorexies, l'épilepsie, les maladies neurodégénératives comme la démence, la maladie d'Alzheimer, la maladie de Parkinson, la sclérose en plaques et la sclérose latérale amyotrophique, les maladies du métabolisme lipidique telles que le déficit en transporteur de glucose (déficit GLUT1), la VL-FAOD et les mitochondriopathies telles que le déficit en thiolase mitochondriale, la chorée de Huntington, les cancers tels que les lymphomes à cellules T, les astrocytomes et les glioblastomes, le VIH, les maladies rhumatismales telles que la polyarthrite rhumatoïde et l'arthrite urique, les maladies du tractus gastro-intestinal telles que les maladies inflammatoires chroniques de l'intestin, notamment la colite ulcéreuse et la maladie de Crohn, les maladies du stockage lyosomique telles que les sphingolipidoses, notamment la maladie de Niemann-Pick, le diabète sucré et les effets ou effets secondaires des chimiothérapies.

11. Ester d'acide carboxylique de 3-hydroxybutanoate (III) ou mélange d'au moins deux esters d'acide carboxylique de 3-hydroxybutanoate (III) différents l'un de l'autre,
où l'ester d'acide carboxylique du 3-hydroxybutanoate (III) de formule générale (IIIb)
CH₃ - CH(OR⁴) - CH₂ - C(O)OR¹ (IIIb)
où dans la formule générale (IIIb)
• le radical R¹ représente un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle et
• le radical R⁴ représente un ou plusieurs des radicaux suivants 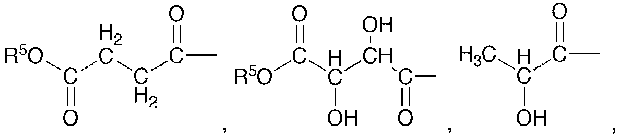 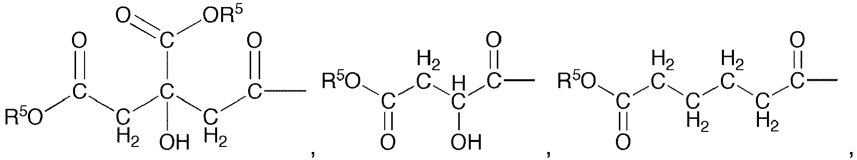 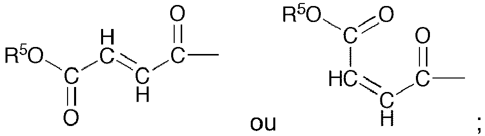
où, dans les radicaux précédents, le radical R⁵ représente un atome d'hydrogène ou un radical - CH(CH₃) - CH₂ - C(O)OR¹ avec R¹ tel que défini précédemment,
pour son utilisation dans le traitement prophylactique et/ou thérapeutique de maladies du corps humain ou animal, notamment de maladies liées à un trouble du métabolisme énergétique, en particulier le métabolisme des corps cétoniques, comme notamment les traumatismes crâniens, les accidents vasculaires cérébraux, les hypoxies, les maladies cardiovasculaires comme l'infarctus du myocarde, le syndrome de réalimentation, les anorexies, l'épilepsie, les maladies neurodégénératives comme la démence, la maladie d'Alzheimer, la maladie de Parkinson, la sclérose en plaques et la sclérose latérale amyotrophique, les maladies du métabolisme lipidique telles que le déficit en transporteur de glucose (déficit GLUT1), la VL-FAOD et les mitochondriopathies telles que le déficit en thiolase mitochondriale, la chorée de Huntington, les cancers tels que les lymphomes à cellules T, les astrocytomes et les glioblastomes, le VIH, les maladies rhumatismales telles que la polyarthrite rhumatoïde et l'arthrite urique, les maladies du tractus gastro-intestinal telles que les maladies inflammatoires chroniques de l'intestin, notamment la colite ulcéreuse et la maladie de Crohn, les maladies du stockage lyosomique telles que les sphingolipidoses, notamment la maladie de Niemann-Pick, le diabète sucré et les effets ou effets secondaires des chimiothérapies.

12. Utilisation d'un ester d'acide carboxylique de 3-hydroxybutanoate (III) ou d'un mélange d'au moins deux esters d'acide carboxylique de 3-hydroxybutanoate (III) différents l'un de l'autre,
où l'ester d'acide carboxylique du 3-hydroxybutanoate (III) de formule générale (IIIb)
CH₃ - CH(OR⁴) - CH₂ - C(O)OR¹ (IIIb)
où dans la formule générale (IIIb)
• le radical R¹ représente un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle et
• le radical R⁴ représente un ou plusieurs des radicaux suivants 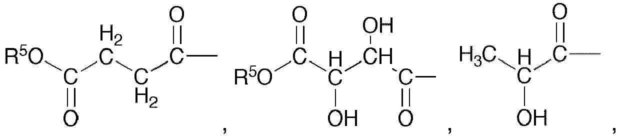 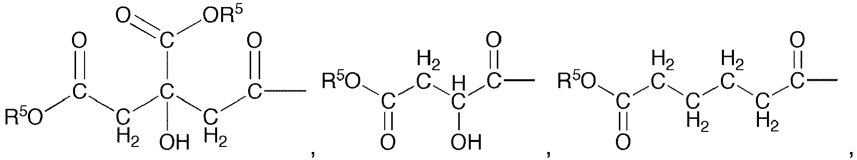 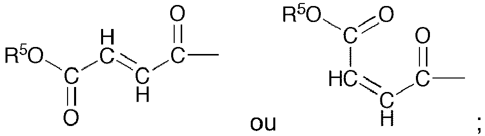
où, dans les radicaux précédents, le radical R⁵ représente un atome d'hydrogène ou un radical - CH(CH₃) - CH₂ - C(O)OR¹ avec R¹ tel que défini précédemment,
pour la fabrication d'un médicament pour le traitement prophylactique et/ou thérapeutique de maladies du corps humain ou animal, en particulier de maladies en relation avec un trouble du métabolisme énergétique, en particulier le métabolisme des corps cétoniques, comme en particulier les traumatismes crâniens, les accidents vasculaires cérébraux, les hypoxies, les maladies cardiovasculaires comme l'infarctus du myocarde, le syndrome de réalimentation, les anorexies, l'épilepsie, les maladies neurodégénératives comme la démence, la maladie d'Alzheimer, la maladie de Parkinson, la sclérose en plaques et la sclérose latérale amyotrophique, les maladies du métabolisme lipidique telles que le déficit en transporteur de glucose (déficit GLUT1), la VL-FAOD et les mitochondriopathies telles que le déficit en thiolase mitochondriale, la chorée de Huntington, les cancers tels que les lymphomes à cellules T, les astrocytomes et les glioblastomes, le VIH, les maladies rhumatismales telles que la polyarthrite rhumatoïde et l'arthrite urique, les maladies du tractus gastro-intestinal telles que les maladies inflammatoires chroniques de l'intestin, notamment la colite ulcéreuse et la maladie de Crohn, les maladies du stockage lyosomique telles que les sphingolipidoses, notamment la maladie de Niemann-Pick, le diabète sucré et les effets ou effets secondaires des chimiothérapies.

13. Utilisation d'un ester d'acide carboxylique de 3-hydroxybutanoate (III) ou d'un mélange d'au moins deux esters d'acide carboxylique de 3-hydroxybutanoate (III) différents l'un de l'autre,
où l'ester d'acide carboxylique du 3-hydroxybutanoate (III) de formule générale (IIIb)
CH₃ - CH(OR⁴) - CH₂ - C(O)OR¹ (IIIb)
où dans la formule générale (IIIb)
• le radical R¹ représente un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle et
• le radical R⁴ représente un ou plusieurs des radicaux suivants 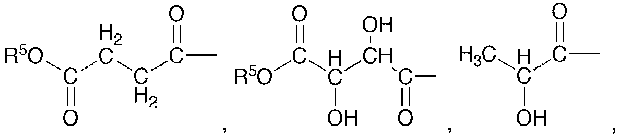 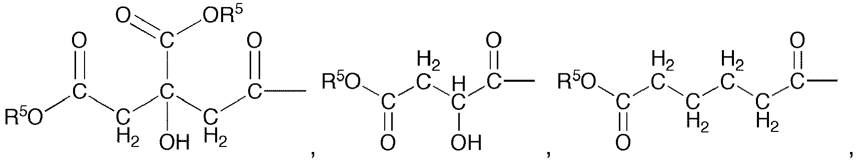 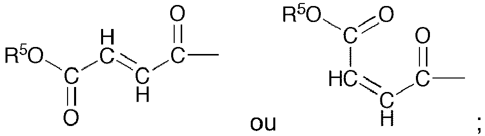
où, dans les radicaux précédents, le radical R⁵ représente un atome d'hydrogène ou un radical - CH(CH₃) - CH₂ - C(O)OR¹ avec R¹ tel que défini précédemment,
pour la préparation d'un médicament destiné au traitement prophylactique et/ou thérapeutique ou à l'application de/aux situations métaboliques cataboliques, telles que la faim, les régimes ou les régimes pauvres en hydrates de carbone.

14. Produit alimentaire et/ou nutritionnel comprenant un ester d'acide carboxylique de 3-hydroxybutanoate (III) ou un mélange d'au moins deux esters d'acide carboxylique de 3-hydroxybutanoate (III) différents l'un de l'autre,
où l'ester d'acide carboxylique du 3-hydroxybutanoate (III) de formule générale (IIIb)
CH₃ - CH(OR⁴) - CH₂ - C(O)OR¹ (IIIb)
où dans la formule générale (IIIb)
• le radical R¹ représente un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle et
• le radical R⁴ représente un ou plusieurs des radicaux suivants 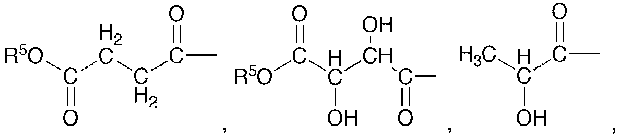 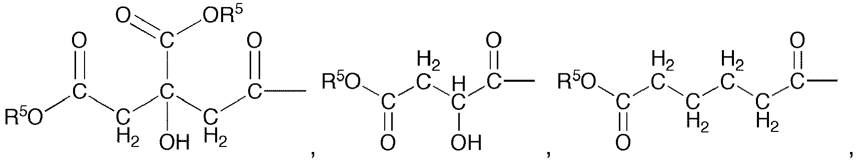 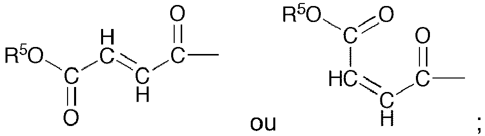
où, dans les radicaux précédents, le radical R⁵ représente un atome d'hydrogène ou un radical - CH(CH₃) - CH₂ - C(O)OR¹ avec R¹ tel que défini précédemment.

15. Produit alimentaire et/ou nutritionnel selon la revendication 14, où le produit alimentaire et/ou nutritionnel est un complément alimentaire, un aliment fonctionnel (*functional food*), un *nouvel aliment,* un additif alimentaire, un complément nutritionnel, un aliment diététique, un power-snack, un coupe-faim ou un supplément de musculation et/ou de sport d'endurance.
